(19) Europäisches Patentamt
European Patent Office
Office européen des brevets

(11) EP 4 458 807 A1

(12) **EUROPEAN PATENT APPLICATION**
published in accordance with Art. 153(4) EPC

(43) Date of publication:
**06.11.2024 Bulletin 2024/45**

(21) Application number: **22916030.4**

(22) Date of filing: **26.12.2022**

(51) International Patent Classification (IPC):
***C07C 235/82*** (2006.01) ***A01N 37/22*** (2006.01)
***A01N 37/24*** (2006.01) ***A01N 43/10*** (2006.01)
***A01N 43/30*** (2006.01) ***A01N 43/40*** (2006.01)
***A01N 43/50*** (2006.01) ***A01N 43/78*** (2006.01)
***A01N 47/02*** (2006.01) ***A01P 5/00*** (2006.01)
***A01P 7/04*** (2006.01) ***A61K 31/167*** (2006.01)
***A61K 31/36*** (2006.01) ***A61K 31/381*** (2006.01)
***A61K 31/426*** (2006.01) ***A61K 31/44*** (2006.01)
***A61K 31/4402*** (2006.01) ***A61K 31/505*** (2006.01)
***A61P 33/14*** (2006.01) ***C07C 317/40*** (2006.01)

(52) Cooperative Patent Classification (CPC):
**A01N 37/22; A01N 37/24; A01N 43/10; A01N 43/30; A01N 43/40; A01N 43/50; A01N 43/78; A01N 47/02; A01P 5/00; A01P 7/04; A61K 31/167; A61K 31/36; A61K 31/381; A61K 31/426; A61K 31/44;** (Cont.)

(86) International application number:
**PCT/JP2022/047949**

(87) International publication number:
**WO 2023/127807 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2021 JP 2021214984**

(71) Applicants:
- **ADEKA CORPORATION**
  **Arakawa-ku**
  **Tokyo**
  **116-8554 (JP)**
- **Nihon Nohyaku Co., Ltd.**
  **Tokyo 104-8386 (JP)**

(72) Inventors:
- **HARA, Kenji**
  **Tokyo 116-8554 (JP)**
- **SUGIHARA, Kousuke**
  **Tokyo 116-8554 (JP)**
- **MASUDA, Tsuyoshi**
  **Tokyo 116-8554 (JP)**
- **YORIFUJI, Nobuyuki**
  **Kawachinagano-shi, Osaka 586-0094 (JP)**
- **TANAKA, Koji**
  **Kawachinagano-shi, Osaka 586-0094 (JP)**
- **FUJII, Kazuki**
  **Kawachinagano-shi, Osaka 586-0094 (JP)**
- **FUKATSU, Kosuke**
  **Kawachinagano-shi, Osaka 586-0094 (JP)**
- **TANAKA, Ryosuke**
  **Kawachinagano-shi, Osaka 586-0094 (JP)**

(74) Representative: **J A Kemp LLP**
**80 Turnmill Street**
**London EC1M 5QU (GB)**

# (54) ARYL CYCLOHEXANEDIONE DERIVATIVE OR SALT THEREOF, PEST CONTROL AGENT CONTAINING SAME, AND METHOD FOR USE THEREOF

(57) In the production of crops in agriculture, horticulture and the like, the damage caused by insect pests and the like is still large, and the development of a novel pest control agent is desired in view of the factors such

as the development of insect pests that are resistant to existing drugs, and the like.

The present invention provides compounds represented by the formula (1)

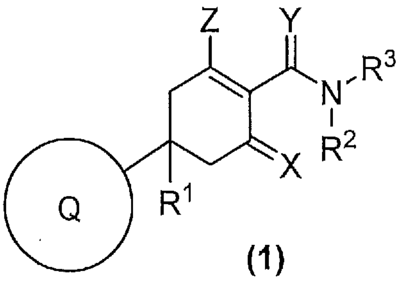

(1)

wherein X and Y are oxygen atoms or sulfur atoms, Z is a hydroxyl group or the like, $R^1$ and $R^2$ are hydrogen atoms and the like, $R^3$ is a substituted phenyl group or the like, and Q is a substituted phenyl group or the like, or salts thereof, pest control agents containing the compounds as active ingredients, and methods for use thereof.

(52) Cooperative Patent Classification (CPC): (Cont.)
**A61K 31/4402; A61K 31/505; A61P 33/14; C07C 235/82; C07C 317/40; C07C 323/40; C07D 213/81; C07D 239/26; C07D 277/44; C07D 317/66; C07D 333/36**

## Description

[Technical Field]

The present invention relates to arylcyclohexanedione derivatives or salts thereof, pest control agents containing the compounds as active ingredients, and methods for use thereof.

[Background Art]

Patent Literature 1 reports that certain kinds of cyclohexanedione carboxamide compounds are useful as insecticides and miticides. However, this Literature only discloses an unsubstituted phenyl group and a phenyl group substituted by a halogen atom as the 5-position substituents of 1,3-cyclohexanedione, and does not describe or suggest the cyclohexanedione derivative having a phenyl group having a substituent and an optionally substituted heterocyclic group of the present invention. In addition, a control effect relating to endoparasites in animals is not described at all.

Patent Literature 2 only reports that certain kinds of cyclohexanedione carboxamide compounds are useful as pharmaceutical compounds. Thus, the control activity of this compound against insect pests in the agricultural and horticultural field and animal parasites is not known at all.

Non Patent Literature 1 describes effects of various compounds on Plasmodium and Eimeria. However, the effect of cyclohexanedione carboxamide compounds is described to be none or low.

[Citation List]

[Patent Literature]

[Patent Literature 1]
US Patent No. 3976785
[Patent Literature 2]
EP-A-0017195

[Non Patent Literature]

[Non Patent Literature 1]
Arzneimittel-Forsching, (Germany) 1987, Vol. 37, p. 647-661

[Summary of Invention]

[Technical Problem]

Parasite control is an important problem when raising animals such as farm animal, companion animal (pet) and the like. In addition to the parasite infection itself, the viruses, bacteria and the like transmitted by the parasites cause considerable suffering to animals, resulting in huge economic losses for farm animals. Therefore, the development of a novel ectoparasite control agent for animals and endoparasite control agent for animals has been desired. In particular, in Dirofilaria immitis infection caused by endoparasites, since the development of resistance to macrocyclic lactone compounds currently used as therapeutic drugs has been observed, the development of a novel therapeutic drug capable of effectively controlling Dirofilaria immitis infection is strongly demanded. In addition, in the production of useful crops in agriculture, horticulture and the like, the damage caused by insect pests is enormous, and many effective agrohorticultural insect pest control agents are on the market. However, due to the demand for a drug that is highly effective against insect pests that are resistant to existing drugs, a drug with physical properties suitable for various laborsaving application methods, a drug superior in human and animal safety with reduced acute toxicity and the like, and a drug superior in environmental safety, such as appropriate persistence in soil and the like, the development of a novel agrohorticultural insect pest control agent is still one of the important problems in the field of agriculture and horticulture.

[Solution to Problem]

The present inventors have conducted intensive studies in an attempt to develop a novel pest control agent,

particularly an agrohorticultural insect pest control agent, an ectoparasite control agent for animals, and an endoparasite control agent for animals, and found that a compound having an arylcyclohexanedione derivative as the carboxylic acid moiety of the carboxamide represented by the formula (1) of the present invention or salts thereof are useful as pest control agents, particularly endoparasite control agents for animals, which resulted in the completion of the present invention.

Accordingly, the present invention relates to

[1] a compound represented by the formula (1)

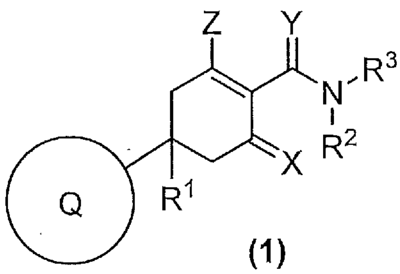

**(1)**

wherein

$R^1$ is

(a1) a hydrogen atom; (a2) a $(C_1-C_6)$alkyl group; (a3) a halo$(C_1-C_6)$alkyl group; (a4) a phenyl group; or (a5) a substituted phenyl group having, on a ring, 1 to 5 substituents each independently selected from the group consisting of a halogen atom, a $(C_1-C_6)$alkyl group, a halo $(C_1-C_6)$ alkyl group, and a $(C_1-C_6)$alkoxy group, $R^2$ is

(b1) a hydrogen atom; (b2) a $(C_1-C_6)$alkyl group; (b3) a $(C_3-C_6)$cycloalkyl group; (b4) a $(C_1-C_6)$alkoxy group; or (b5) a $(C_1-C_6)$alkylcarbonyl group,

$R^3$ is

(c1) a $(C_1-C_6)$alkyl group; (c2) a $(C_2-C_6)$alkenyl group; (c3) a $(C_2-C_6)$alkynyl group; (c4) a $(C_3-C_6)$cycloalkyl group; (c5) a halo$(C_1-C_6)$ alkyl group; (c6) a halo $(C_2-C_6)$ alkenyl group; (c7) a halo$(C_2-C_6)$alkynyl group; (c8) a substituted $(C_1-C_6)$alkyl group having 1 to 3 substituents each independently selected from the group consisting of a cyano group, a $(C_3-C_6)$ cycloalkyl group, a $(C_1-C_6)$ alkoxy group, a $(C_1-C_6)$ alkylthio group, a halo $(C_3-C_6)$ cycloalkyl group, a halo$(C_1-C_6)$alkoxy group, a halo $(C_1-C_6)$ alkylthio group, a $(C_1-C_6)$alkoxycarbonyl group, a $(C_2-C_6)$alkenylcarbonyloxy group, a carboxamido group, a phenylcarbonyl group and a di$(C_1-C_6)$alkylamino group; (c9) a substituted $(C_3-C_6)$cycloalkyl group having, on a ring, 1 to 3 substituents each independently selected from the group consisting of a cyano group, a $(C_3-C_6)$cycloalkyl group, a $(C_1-C_6)$ alkoxy group, a $(C_1-C_6)$ alkylthio group, a $(C_1-C_6)$ alkyl group, a halo $(C_3-C_6)$ cycloalkyl group, a halo $(C_1-C_6)$ alkoxy group, a halo$(C_1-C_6)$alkylthio group and a carboxamido group; (c10) a $(C_1-C_6)$alkylsulfonyl group; (c11) an N-$(C_1-C_6)$alkylaminosulfonyl group; (c12) a phenylcarbonylamino group; (c13) a substituted phenylcarbonylamino group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c14) a piperidinyl group; (c15) a substituted piperidinyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group, a halo $(C_1-C_6)$ alkoxy group, a $(C_1-C_6)$alkoxycarbonyl group and a phenyl$(C_1-C_6)$alkoxycarbonyl group; (c16) a morpholinyl group; (c17) a substituted morpholinyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo $(C_1-C_6)$alkoxy group; (c18) a substituted phenyl group having, on a ring, 1 to 5 substituents each independently selected from the group consisting of (a) a halogen atom, (b) a cyano group, (c) a nitro group, (d) a hydroxyl group, (e) a carboxyl group, (f) a $(C_1-C_6)$ alkyl group, (g) a $(C_1-C_6)$ alkoxy group, (h) a $(C_3-C_6)$ cycloalkyl group, (i) a $(C_1-C_6)$ alkylthio group, (j) a $(C_1-C_6)$alkylsulfinyl group, (k) a $(C_1-C_6)$alkylsulfonyl group, (l) a halo$(C_1-C_6)$alkyl group, (m) a halo $(C_1-C_6)$ alkoxy group, (n) a halo $(C_3-C_6)$ cycloalkyl group, (o) a

halo ($C_1$-$C_6$) alkylthio group, (p) a halo($C_1$-$C_6$)alkylsulfinyl group, (q) a halo($C_1$-$C_6$)alkylsulfonyl group, (r) a ($C_1$-$C_6$)alkylcarbonyl group, (s) a ($C_1$-$C_6$)alkoxycarbonyl group, (t) a ($C_1$-$C_6$) alkylcarbonylamino group, (u) a halo($C_1$-$C_6$)alkylcarbonylamino group, (v) a ($C_1$-$C_6$)alkylsulfonylamino group, (w) a halo ($C_1$-$C_6$) alkylsulfonylamino group, (x) an N,N-di($C_1$-$C_6$)alkylcarboxamido group, (y) an N-($C_1$-$C_6$)alkylcarboxamido group, (z) an N-halo($C_1$-$C_6$)alkylcarboxamido group, (aa) an N-($C_1$-$C_6$)alkylaminosulfonyl group, (ab) a ($C_1$-$C_6$)alkoxy($C_1$-$C_6$)alkyl group, (ac) an $SF_5$ group, (ad) an $R^{10}$-($R^{11}$-N=)O=S group wherein $R^{10}$ is a ($C_1$-$C_6$) alkyl group, a ($C_3$-$C_6$) cycloalkyl group, a halo ($C_1$-$C_6$) alkyl group, or a ($C_1$-$C_6$) alkoxy ($C_1$-$C_6$) alkyl group, and $R^{11}$ is a hydrogen atom, a cyano group, a ($C_1$-$C_6$)alkyl group, a ($C_3$-$C_6$)cycloalkyl group, a halo ($C_1$-$C_6$) alkyl group, a ($C_1$-$C_6$)alkylcarbonyl group, or a halo ($C_1$-$C_6$) alkylcarbonyl group, (ae) an $R^{10}$- ($R^{11}$-N=) S group wherein $R^{10}$ and $R^{11}$ are the same as above, (af) a pyrazolyl group, (ag) a phenoxy group, (ah) a phenyl group, and (ai) a substituted phenyl group having, on a ring, 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$)alkoxy group, or (aj) a methylenedioxy group formed by two adjacent substituents together and optionally substituted by 1 or 2 substituents selected from a halogen atom, a phenyl group and a ($C_1$-$C_6$)alkyl group, provided that an atom adjacent to a bonding atom in the pyrazolyl group is not substituted by the ($C_1$-$C_6$)alkylsulfonyl group, the halo($C_1$-$C_6$)alkylsulfonyl group, the N-($C_1$-$C_6$)alkylaminosulfonyl group, or the $R^{10}$-($R^{11}$-N=)O=S group wherein $R^{10}$ and $R^{11}$ are the same as above; (c19) a pyridyl group; (c20) a substituted pyridyl group having, on a ring, 1 to 4 substituents each independently selected from the group consisting of a halogen atom, a cyano group, a nitro group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group, a halo ($C_1$-$C_6$)alkoxy group, a halo ($C_1$-$C_6$)alkylthio group, a halo ($C_1$-$C_6$)alkylsulfinyl group and a halo($C_1$-$C_6$)alkylsulfonyl group; (c21) an N-oxo-pyridyl group; (c22) a substituted N-oxo-pyridyl group having, on a ring, 1 to 4 substituents each independently selected from the group consisting of a halogen atom, a cyano group, a nitro group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo ($C_1$-$C_6$)alkyl group, a halo ($C_1$-$C_6$)alkoxy group, a halo ($C_1$-$C_6$)alkylthio group, a halo($C_1$-$C_6$)alkylsulfinyl group and a halo($C_1$-$C_6$)alkylsulfonyl group; (c23) a pyridazinyl group; (c24) a substituted pyridazinyl group having, on a ring, 1 to 3 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo ($C_1$-$C_6$)alkyl group and a halo($C_1$-$C_6$)alkoxy group; (c25) a pyrimidinyl group; (c26) a substituted pyrimidinyl group having, on a ring, 1 to 3 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$) alkoxy group; (c27) a pyrazinyl group; (c28) a substituted pyrazinyl group having, on a ring, 1 to 3 substituents each independently selected from the group consisting of a halogen atom, a cyano group, a ($C_1$-$C_6$)alkyl group, a ($C_1$-$C_6$) alkoxy group, a ($C_3$-$C_6$) cycloalkyl group, a ($C_1$-$C_6$) alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$)alkoxy group; (c29) a triazinyl group; (c30) a substituted triazinyl group having, on a ring, 1 or 2 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo ($C_1$-$C_6$)alkyl group and a halo($C_1$-$C_6$)alkoxy group; (c31) a tetrahydrofuranyl group; (c32) a substituted tetrahydrofuranyl group having, on a ring, 1 to 3 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$)alkoxy group; (c33) an oxazolinyl group; (c34) a substituted oxazolinyl group having, on a ring, 1 to 3 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo ($C_1$-$C_6$)alkyl group and a halo($C_1$-$C_6$)alkoxy group; (c35) a thiazolinyl group; (c36) a substituted thiazolinyl group having, on a ring, 1 to 3 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$)alkoxy group; (c37) an oxazolyl group; (c38) a substituted oxazolyl group having, on a ring, 1 or 2 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo($C_1$-$C_6$)alkoxy group; (c39) a thiazolyl group; (c40) a substituted thiazolyl group having, on a ring, 1 or 2 substituents each independently selected from the group consisting of

a halogen atom, a cyano group, a $(C_1-C_6)$alkyl group, a $(C_1-C_6)$ alkoxy group, a $(C_3-C_6)$ cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group and a halo$(C_1-C_6)$alkoxy group; (c41) an isoxazolyl group; (c42) a substituted isoxazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c43) an isothiazolyl group; (c44) a substituted isothiazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c45) an imidazolyl group; (c46) a substituted imidazolyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group and a halo $(C_1-C_6)$alkoxy group; (c47) a pyrazolyl group; (c48) a substituted pyrazolyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c49) a triazolyl group; (c50) a substituted triazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c51) a tetrazolyl group; (c52) a substituted tetrazolyl group having, on a ring, one substituent selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c53) a thiadiazolyl group; (c54) a substituted thiadiazolyl group having, on a ring, one substituent selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c55) a quinolinyl group; (c56) a substituted quinolinyl group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c57) a benzoxazolyl group; (c58) a substituted benzoxazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group and a halo$(C_1-C_6)$alkoxy group; (c59) a benzothiazolyl group; (c60) a substituted benzothiazolyl group having, on a ring, 1 to 4 substituents each independently selected from the group consisting of a halogen atom, a cyano group, a $(C_1-C_6)$ alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c61) a benzimidazolyl group; (c62) a substituted benzimidazolyl group having, on a ring, 1 to 4 substituents each independently selected from the group consisting of a halogen atom, a cyano group, a $(C_1-C_6)$ alkyl group, a $(C_1-C_6)$ alkoxy group, a $(C_3-C_6)$ cycloalkyl group, a $(C_1-C_6)$alkylthio group, a $(C_1-C_6)$alkylsulfinyl group, a $(C_1-C_6)$ alkylsulfonyl group, a halo $(C_1-C_6)$ alkyl group and a halo$(C_1-C_6)$alkoxy group; (c63) a benzothiadiazolyl group; (c64) a substituted benzothiadiazolyl group having, on a ring, 1 to 4 substituents each independently selected from the group consisting of a halogen atom, a cyano group, a $(C_1-C_6)$alkyl group, a $(C_1-C_6)$ alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo $(C_1-C_6)$alkoxy group; (c65) an indazolyl group; (c66) a substituted indazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c67) an indanyl group; (c68) a substituted indanyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$ alkoxy group; (c69) a tetrahydronaphthyl group; (c70) a substituted tetrahydronaphthyl group having, on a ring, 1 to 4 substituents each independently selected from the group consisting of a halogen atom, a cyano group, a $(C_1-C_6)$ alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c71) a tetrahydrofuranyl$(C_1-C_6)$alkyl group; (c72) a sub-

stituted tetrahydrofuranyl($C_1$-$C_6$)alkyl group having, on a ring, 1 to 3 substituents each independently selected from the group consisting of a halogen atom, a cyano group, a ($C_1$-$C_6$) alkyl group, a ($C_1$-$C_6$) alkoxy group, a ($C_3$-$C_6$) cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$)alkoxy group; (c73) a phenyl($C_1$-$C_6$)alkyl group; (c74) a substituted phenyl ($C_1$-$C_6$)alkyl group having, on a ring, 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$)alkoxy group; (c75) a ($C_1$-$C_6$)alkoxycarbonyl group; (c76) a phenylcarbonyl group; (c77) a thienyl group; (c78) a substituted thienyl group having, on a ring, 1 to 3 substituents each independently selected from the group consisting of a halogen atom, a cyano group, a nitro group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo ($C_1$-$C_6$)alkyl group, a halo($C_1$-$C_6$)alkoxy group, a halo ($C_1$-$C_6$)alkylthio group, a halo($C_1$-$C_6$)alkylsulfinyl group and a halo($C_1$-$C_6$)alkylsulfonyl group; (c79) a naphthyl group; (c80) a substituted naphthyl group having, on a ring, 1 to 7 substituents each independently selected from the group consisting of a halogen atom, a cyano group, a nitro group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo ($C_1$-$C_6$) alkyl group, a halo($C_1$-$C_6$)alkoxy group, a halo($C_1$-$C_6$)alkylthio group, a halo($C_1$-$C_6$)alkylsulfinyl group and a halo($C_1$-$C_6$)alkylsulfonyl group; (c81) a 1,1-dioxidetetrahydro-2H-thiopyranyl group; (c82) an imidazopyridyl group; or (c83) a substituted imidazopyridyl group having, on a ring, 1 to 5 substituents each independently selected from the group consisting of a halogen atom, a cyano group, a nitro group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group, a halo ($C_1$-$C_6$) alkoxy group, a halo ($C_1$-$C_6$)alkylthio group, a halo ($C_1$-$C_6$)alkylsulfinyl group, a halo ($C_1$-$C_6$)alkylsulfonyl group and a phenyl group,

Q is

(d1) a substituted phenyl group having, on a ring, 1 to 5 substituents each independently selected from substituent group A; (d2) a naphthyl group; (d3) a substituted naphthyl group having, on a ring, 1 to 7 substituents each independently selected from substituent group B; (d4) a pyridyl group; (d5) a substituted pyridyl group having, on a ring, 1 to 4 substituents each independently selected from substituent group B; (d6) a pyridazinyl group; (d7) a substituted pyridazinyl group having, on a ring, 1 to 3 substituents each independently selected from substituent group B; (d8) a pyrimidinyl group; (d9) a substituted pyrimidinyl group having, on a ring, 1 to 3 substituents each independently selected from substituent group B; (d10) a pyrazinyl group; (d11) a substituted pyrazinyl group having, on a ring, 1 to 3 substituents each independently selected from substituent group B; (d12) a triazinyl group; (d13) a substituted triazinyl group having, on a ring, 1 or 2 substituents each independently selected from substituent group B; (d14) a 2-oxopyridyl group; (d15) a substituted 2-oxopyridyl group having, on a ring, 1 to 4 substituents each independently selected from substituent group B; (d16) an isoxazolyl group; (d17) a substituted isoxazolyl group having, on a ring, 1 or 2 substituents each independently selected from substituent group B; (d18) an oxazolyl group; (d19) a substituted oxazolyl group having, on a ring, 1 or 2 substituents each independently selected from substituent group B; (d20) a thienyl group; (d21) a substituted thienyl group having, on a ring, 1 to 3 substituents each independently selected from substituent group B; (d22) an isothiazolyl group; (d23) a substituted isothiazolyl group having, on a ring, 1 or 2 substituents each independently selected from substituent group B; (d24) a thiazolyl group; (d25) a substituted thiazolyl group having, on a ring, 1 or 2 substituents each independently selected from substituent group B; (d26) an oxadiazolyl group; (d27) a substituted oxadiazolyl group having, on a ring, one substituent each independently selected from substituent group B; (d28) a thiadiazolyl group; (d29) a substituted thiadiazolyl group having, on a ring, one substituent each independently selected from substituent group B; (d30) a pyrrolyl group; (d31) a substituted pyrrolyl group having, on a ring, 1 to 4 substituents each independently selected from substituent group B; (d32) an imidazolyl group; (d33) a substituted imidazolyl group having, on a ring, 1 to 3 substituents each independently selected from substituent group B; (d34) a triazolyl group; (d35) a substituted triazolyl group having, on a ring, 1 or 2 substituents each independently selected from substituent group B; (d36) a tetrazolyl group; (d37) a substituted tetrazolyl group having, on a ring, one substituent each independently selected from substituent group B; (d38) a benzofuranyl group; (d39) a substituted benzofuranyl group having, on a ring, 1 to 5 substituents each independently selected from substituent group B; (d40) a benzoxazolyl group; (d41) a substituted benzoxazolyl group having, on a ring, 1 to 4 substituents each independently selected from substituent group B; (d42) a benzisoxazolyl group; (d43) a substituted benzisoxazolyl group having, on a ring, 1 to 4 substituents each independently selected from substituent group B; (d44) a benzothienyl group; (d45) a substituted benzothienyl group having, on a ring, 1 to 5 substituents each independently selected from substituent group B; (d46) a benzothiazolyl group; (d47) a substituted benzothiazolyl group having, on a ring, 1 to 4 substituents each independently selected from substituent group B; (d48) a benzisothiazolyl group; (d49) a substituted benzisothiazolyl group having, on a ring, 1 to 4 substituents each independently selected from substituent group B; (d50) an indolyl group; (d51) a substituted indolyl group having, on a ring, 1 to 6 substituents each independently selected from substituent group B; (d52) an isoindolyl group; (d53) a substituted isoindolyl group having, on a ring, 1 to 6 substituents each independently selected from substituent group B; (d54) an indazolyl group; (d55) a substituted indazolyl group having, on a ring, 1 to 5 substituents each independently selected from substituent group B; (d56) a benzimidazolyl group; (d57) a substituted benzimidazolyl group having, on a ring, 1 to 5 substituents each independently selected from substituent group B; (d58) a benzotriazolyl group; (d59) a substituted benzotriazolyl group having, on a ring, 1 to 4 substituents each independently selected from substituent group B; (d60) a furopyridyl group; (d61) a substituted furopyridyl group having, on a ring, 1 to 4 substituents each independently selected from substituent group B; (d62) a thienopyridyl group; (d63) a substituted thienopyridyl group having, on a ring, 1 to 4 substituents each independently selected from substituent group B; (d64) an indolizinyl group; (d65) a substituted indolizinyl group having, on a ring, 1 to 6 substituents each independently selected from substituent group B; (d66) a pyrrolopyridyl group; (d67) a substituted pyrrolopyridyl group having, on a ring, 1 to 5 substituents each independently selected from substituent group B; (d68) a pyrrolopyrimidinyl group; (d69) a substituted pyrrolopyrimidinyl group having, on a ring, 1 to 5 substituents each independently selected from substituent group B; (d70) an oxazolopyridyl group; (d71) a substituted oxazolopyridyl group having, on a ring, 1 to 3 substituents each independently selected from substituent group B; (d72) an isoxazolopyridyl group; (d73) a substituted isoxazolopyridyl group having, on a ring, 1 to 3 substituents each independently selected from substituent group B; (d74) a thiazolopyridyl group; (d75) a substituted thiazolopyridyl group having, on a ring, 1 to 3 substituents each independently selected from substituent group B; (d76) an isothiazolopyridyl group; (d77) a substituted isothiazolopyridyl group having, on a ring, 1 to 3 substituents each independently selected from substituent group B; (d78) an imidazopyridyl group; (d79) a substituted imidazopyridyl group having, on a ring, 1 to 5 substituents each independently selected from substituent group B; (d80) an imidazopyrimidinyl group; (d81) a substituted imidazopyrimidinyl group having, on a ring, 1 to 5 substituents each independently selected from substituent group B; (d82) a pyrazolopyridyl group; (d83) a substituted pyrazolopyridyl group having, on a ring, 1 to 5 substituents each independently selected from substituent group B; (d84) a pyrazolopyrimidinyl group; (d85) a substituted pyrazolopyrimidinyl group having, on a ring, 1 to 5 substituents each independently selected from substituent group B; (d86) a triazolopyridyl group; (d87) a substituted triazolopyridyl group having, on a ring, 1 to 4 substituents each independently selected from substituent group B; (d88) a triazolopyrimidinyl group; (d89) a substituted triazolopyrimidinyl group having, on a ring, 1 to 4 substituents each independently selected from substituent group B; (d90) a quinoxalinyl group; (d91) a substituted quinoxalinyl group having, on a ring, 1 to 5 substituents each independently selected from substituent group B; (d92) a quinolinyl group; (d93) a substituted quinolinyl group having, on a ring, 1 to 6 substituents each independently selected from substituent group B; (d94) an isoquinolinyl group; (d95) a substituted isoquinolinyl group having, on a ring, 1 to 6 substituents each independently selected from substituent group B; (d96) a cinnolinyl group; (d97) a substituted cinnolinyl group having, on a ring, 1 to 5 substituents each independently selected from substituent group B; (d98) a phthalazinyl group; (d99) a substituted phthalazinyl group having, on a ring, 1 to 5 substituents each independently selected from substituent group B; (d100) a quinazolinyl group; (d101) a substituted quinazolinyl group having, on a ring, 1 to 5 substituents each independently selected from substituent group B; (d102) a naphthyridinyl group; (d103) a substituted naphthyridinyl group having, on a ring, 1 to 5 substituents each independently selected from substituent group B; or (d104) a ferrocenyl group, X and Y are each independently an oxygen atom or a sulfur atom, Z is an $OR^4$ group wherein $R^4$ is a hydrogen atom, a $(C_1-C_6)$alkyl group, a $(C_1-C_6)$ alkylcarbonyl group or a $(C_1-C_6)$ alkoxycarbonyl group, an $S(O)nR^5$ group wherein n is 0, 1 or 2, and $R^5$ is a hydrogen atom, a $(C_1-C_6)$ alkyl group or a phenyl $(C_1-C_6)$ alkyl group, or an $N(R^6)R^7$ group wherein $R^6$ and $R^7$ are hydrogen atoms, hydroxyl groups, $(C_1-C_6)$ alkyl groups, $(C_1-C_6)$alkoxy groups, $(C_1-C_6)$ alkylcarbonyl groups or $(C_1-C_6)$alkoxycarbonyl groups, and $R^6$ and $R^7$ are the same or different, substituent group A consists of

(e1) a cyano group; (e2) a nitro group; (e3) a hydroxyl group;
(e4) a carboxyl group; (e5) a $(C_1-C_6)$alkyl group; (e6) a $(C_2-C_6)$ alkenyl group; (e7) a $(C_2-C_6)$ alkynyl group; (e8) a $(C_3-C_6)$ cycloalkyl group; (e9) a substituted $(C_3-C_6)$ cycloalkyl group having, on a ring, 1 to 3 substituents each independently selected from the group consisting of a halogen atom, a cyano group, a $(C_1-C_6)$ alkyl group, a $(C_1-C_6)$ alkoxy group and a $(C_1-C_6)$ alkylcarbonyl group; (e10) a $(C_1-C_6)$alkoxy$(C_1-C_6)$alkyl group; (e11) a $(C_1-C_6)$alkylthio group; (e12) a $(C_1-C_6)$alkylsulfinyl group; (e13) a $(C_1-C_6)$alkylsulfonyl group; (e14) a halo $(C_1-C_6)$ alkyl group; (e15) a halo $(C_2-C_6)$alkenyl group; (e16) a halo $(C_2-C_6)$alkynyl group; (e17) a halo $(C_1-C_6)$alkoxy group; (e18) a halo $(C_3-C_6)$cycloalkyl group; (e19) a halo $(C_1-C_6)$ alkylthio group; (e20) a halo $(C_1-C_6)$alkylsulfinyl group; (e21) a halo $(C_1-C_6)$ alkylsulfonyl group; (e22) an $SF_5$ group; (e23) a $(C_1-C_6)$alkylcarbonylamino group; (e24) a halo$(C_1-C_6)$alkylcarbonylamino group; (e25) a $(C_1-C_6)$alkylsul-

fonylamino group; (e26) a halo ($C_1$-$C_6$) alkylsulfonylamino group; (e27) an N,N-di($C_1$-$C_6$)alkylcarboxamido group; (e28) an N-halo($C_1$-$C_6$)alkylcarboxamido group; (e29) a ($C_1$-$C_6$)alkoxycarbonyl group; (e30) an N-($C_1$-$C_6$)alkylaminosulfonyl group; (e31) an N,N-di($C_1$-$C_6$)alkylaminosulfonyl group; (e32) a pyrazolyl group; (e33) a substituted pyrazolyl group having, on a ring, 1 to 3 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$) alkylthio group, a ($C_1$-$C_6$)alkylsulfinyl group, a ($C_1$-$C_6$)alkylsulfonyl group, a halo($C_1$-$C_6$)alkyl group and a halo($C_1$-$C_6$)alkoxy group; (e34) an oxadiazolyl group; (e35) a substituted oxadiazolyl group having, on a ring, one substituent each independently selected from the group consisting of a halogen atom, a cyano group, a ($C_1$-$C_6$)alkyl group, a ($C_1$-$C_6$)alkoxy group, a ($C_3$-$C_6$) cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$)alkoxy group; (e36) a phenyl group; (e37) a substituted phenyl group having, on a ring, 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo ($C_1$-$C_6$) alkyl group and a halo ($C_1$-$C_6$)alkoxy group; (e38) a phenoxy group; (e39) a substituted phenoxy group having, on a ring, 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$) alkylsulfinyl group, a ($C_1$-$C_6$)alkylsulfonyl group, a halo($C_1$-$C_6$)alkyl group and a halo ($C_1$-$C_6$)alkoxy group; (e40) a methylenedioxy group formed by two adjacent substituents together and optionally substituted by 1 or 2 substituents selected from the group consisting of a halogen atom, a phenyl group and a ($C_1$-$C_6$)alkyl group; (e41) an $R^8$-($R^9$-N=)O=S group wherein $R^8$ is a ($C_1$-$C_6$)alkyl group, a ($C_3$-$C_6$)cycloalkyl group, a halo ($C_1$-$C_6$) alkyl group, or a ($C_1$-$C_6$)alkoxy ($C_1$-$C_6$)alkyl group, and $R^9$ is a hydrogen atom, a cyano group, a ($C_1$-$C_6$)alkyl group, a ($C_3$-$C_6$)cycloalkyl group, a halo($C_1$-$C_6$)alkyl group, a ($C_1$-$C_6$)alkylcarbonyl group, or a halo ($C_1$-$C_6$)alkylcarbonyl group; (e42) a carboxamido group; (e43) a morpholinyl group; (e44) an N,N-di($C_1$-$C_6$)alkylamino group; (e45) an $R^8$-($R^9$-N=)S group wherein $R^8$ and $R^9$ are the same as above; (e46) an N-($C_1$-$C_6$)alkylcarboxamido group; (e47) a phenyl ($C_1$-$C_6$)alkylthio group; and (e48) a substituted phenyl($C_1$-$C_6$)alkylthio group having, on a ring, 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$) alkylsulfinyl group, a ($C_1$-$C_6$)alkylsulfonyl group, a halo ($C_1$-$C_6$)alkyl group and a halo($C_1$-$C_6$)alkoxy group, and

substituent group B consists of
(f1) a halogen atom; (f2) a cyano group; (f3) a nitro group; (f4) a hydroxyl group; (f5) a carboxyl group; (f6) a ($C_1$-$C_6$)alkyl group; (f7) a ($C_2$-$C_6$)alkenyl group; (f8) a ($C_2$-$C_6$)alkynyl group; (f9) a ($C_1$-$C_6$)alkoxy group; (f10) a ($C_3$-$C_6$)cycloalkyl group; (f11) a substituted ($C_3$-$C_6$)cycloalkyl group having, on a ring, 1 to 3 substituents each independently selected from the group consisting of a halogen atom, a cyano group, a ($C_1$-$C_6$)alkyl group, a ($C_1$-$C_6$)alkoxy group and a ($C_1$-$C_6$)alkylcarbonyl group; (f12) a ($C_1$-$C_6$)alkoxy($C_1$-$C_6$)alkyl group; (f13) a ($C_1$-$C_6$)alkylthio group; (f14) a ($C_1$-$C_6$)alkylsulfinyl group; (f15) a ($C_1$-$C_6$)alkylsulfonyl group; (f16) a halo ($C_1$-$C_6$)alkyl group; (f17) a halo ($C_2$-$C_6$)alkenyl group; (f18) a halo ($C_2$-$C_6$) alkynyl group; (f19) a halo ($C_1$-$C_6$)alkoxy group; (f20) a halo ($C_3$-$C_6$) cycloalkyl group; (f21) a halo ($C_1$-$C_6$)alkylthio group; (f22) a halo ($C_1$-$C_6$) alkylsulfinyl group; (f23) a halo($C_1$-$C_6$)alkylsulfonyl group; (f24) an $SF_5$ group; (f25) a ($C_1$-$C_6$)alkylcarbonylamino group; (f26) a halo($C_1$-$C_6$)alkylcarbonylamino group; (f27) a ($C_1$-$C_6$)alkylsulfonylamino group; (f28) a halo($C_1$-$C_6$)alkylsulfonylamino group; (f29) an N,N-di($C_1$-$C_6$)alkylcarboxamido group; (f30) an N-halo ($C_1$-$C_6$)alkylcarboxamido group; (f31) a ($C_1$-$C_6$)alkoxycarbonyl group; (f32) an N-($C_1$-$C_6$)alkylaminosulfonyl group; (f33) an N,N-di($C_1$-$C_6$)alkylaminosulfonyl group; (f34) a pyrazolyl group; (f35) a substituted pyrazolyl group having, on a ring, 1 to 3 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo ($C_1$-$C_6$)alkyl group and a halo($C_1$-$C_6$)alkoxy group; (f36) an oxadiazolyl group; (f37) a substituted oxadiazolyl group having, on a ring, one substituent each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo($C_1$-$C_6$)alkoxy group; (f38) a phenyl group; (f39) a substituted phenyl group having, on a ring, 1 to 5 substituents each independently selected from the group consisting of a halogen atom, a cyano group, a ($C_1$-$C_6$)alkyl group, a ($C_1$-$C_6$) alkoxy group, a ($C_3$-$C_6$) cycloalkyl group, a ($C_1$-$C_6$) alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$)alkoxy group; (f40) a phenoxy group; (f41) a substituted phenoxy group having, on a ring, 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio

group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (f42) a methylenedioxy group formed by two adjacent substituents together and optionally substituted by 1 or 2 substituents selected from the group consisting of a halogen atom, a phenyl group and a $(C_1-C_6)$alkyl group; (f43) an $R^8$-($R^9$-N=)O=S group wherein $R^8$ is a $(C_1-C_6)$alkyl group, a $(C_3-C_6)$ cycloalkyl group, a halo $(C_1-C_6)$ alkyl group, or a $(C_1-C_6)$alkoxy$(C_1-C_6)$alkyl group, and $R^9$ is a hydrogen atom, a cyano group, a $(C_1-C_6)$alkyl group, a $(C_3-C_6)$cycloalkyl group, a halo $(C_1-C_6)$ alkyl group, a $(C_1-C_6)$alkylcarbonyl group, or a halo$(C_1-C_6)$alkylcarbonyl group; (f44) a carboxamido group; (f45) a morpholinyl group; and (f46) an N,N-di$(C_1-C_6)$alkylamino group, provided that a compound represented by the following structural formula is excluded:

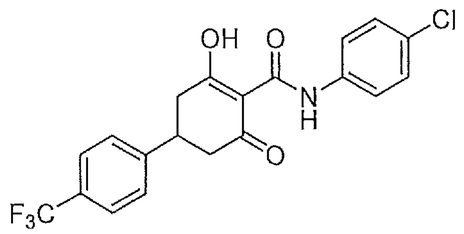

,

and salts thereof,

[2] the compound and salts thereof of [1], wherein

$R^1$ is

(a1) a hydrogen atom; (a2) a $(C_1-C_6)$alkyl group; (a3) a halo$(C_1-C_6)$alkyl group; (a4) a phenyl group; or (a5) a substituted phenyl group having, on a ring, 1 to 5 substituents each independently selected from the group consisting of a halogen atom, a $(C_1-C_6)$ alkyl group, a halo $(C_1-C_6)$ alkyl group, and a $(C_1-C_6)$ alkoxy group, $R^2$ is
(b1) a hydrogen atom; or (b2) a $(C_1-C_6)$ alkyl group,

$R^3$ is
(c1) a $(C_1-C_6)$ alkyl group; (c2) a $(C_2-C_6)$ alkenyl group; (c3) a $(C_2-C_6)$ alkynyl group; (c4) a $(C_3-C_6)$ cycloalkyl group; (c5) a halo$(C_1-C_6)$alkyl group; (c8) a substituted $(C_1-C_6)$alkyl group having 1 to 3 substituents each independently selected from the group consisting of a cyano group, a $(C_3-C_6)$ cycloalkyl group, a $(C_1-C_6)$ alkoxy group, a $(C_1-C_6)$ alkylthio group, a halo $(C_3-C_6)$ cycloalkyl group, a halo $(C_1-C_6)$alkoxy group, a halo$(C_1-C_6)$alkylthio group, a $(C_1-C_6)$alkoxycarbonyl group, a $(C_2-C_6)$alkenylcarbonyloxy group, a carboxamido group, a phenylcarbonyl group and a di$(C_1-C_6)$alkylamino group; (c9) a substituted $(C_3-C_6)$cycloalkyl group having, on a ring, 1 to 3 substituents each independently selected from the group consisting of a cyano group, a $(C_3-C_6)$cycloalkyl group, a $(C_1-C_6)$ alkoxy group, a $(C_1-C_6)$ alkylthio group, a $(C_1-C_6)$ alkyl group, a halo $(C_3-C_6)$ cycloalkyl group, a halo $(C_1-C_6)$ alkoxy group, a halo$(C_1-C_6)$alkylthio group and a carboxamido group; (c18) a substituted phenyl group having, on a ring, 1 to 5 substituents each independently selected from the group consisting of (a) a halogen atom, (b) a cyano group, (c) a nitro group, (d) a hydroxyl group, (e) a carboxyl group, (f) a $(C_1-C_6)$ alkyl group, (g) a $(C_1-C_6)$ alkoxy group, (h) a $(C_3-C_6)$ cycloalkyl group, (i) a $(C_1-C_6)$ alkylthio group, (j) a $(C_1-C_6)$alkylsulfinyl group, (k) a $(C_1-C_6)$alkylsulfonyl group, (l) a halo $(C_1-C_6)$ alkyl group, (m) a halo $(C_1-C_6)$ alkoxy group, (n) a halo $(C_3-C_6)$ cycloalkyl group, (o) a halo $(C_1-C_6)$ alkylthio group, (p) a halo$(C_1-C_6)$alkylsulfinyl group, (q) a halo $(C_1-C_6)$alkylsulfonyl group, (r) a $(C_1-C_6)$alkylcarbonyl group, (s) a $(C_1-C_6)$alkoxycarbonyl group, (t) a $(C_1-C_6)$alkylcarbonylamino group, (u) a halo$(C_1-C_6)$alkylcarbonylamino group, (v) a $(C_1-C_6)$alkylsulfonylamino group, (w) a halo $(C_1-C_6)$alkylsulfonylamino group, (x) an N,N-di$(C_1-C_6)$alkylcarboxamido group, (y) an N-$(C_1-C_6)$alkylcarboxamido group, (z) an N-halo $(C_1-C_6)$ alkylcarboxamido group, (aa) an N-$(C_1-C_6)$alkylaminosulfonyl group, (ab) a $(C_1-C_6)$alkoxy$(C_1-C_6)$alkyl group, (ac) an $SF_5$ group, (ad) an $R^{10}$-($R^{11}$-N=)O=S group wherein $R^{10}$ is a $(C_1-C_6)$alkyl group, a $(C_3-C_6)$cycloalkyl group, a halo $(C_1-C_6)$alkyl group, or a $(C_1-C_6)$alkoxy$(C_1-C_6)$alkyl group, and $R^{11}$ is a hydrogen atom, a cyano group, a $(C_1-C_6)$alkyl group, a $(C_3-C_6)$cycloalkyl group, a halo $(C_1-C_6)$ alkyl group, a $(C_1-C_6)$alkylcarbonyl group, or a halo $(C_1-C_6)$alkylcarbonyl group, (ae) an $R^{10}$- ($R^{11}$-N=)S group wherein $R^{10}$ and $R^{11}$ are the same as above, (af) a pyrazolyl group, (ag) a phenoxy group, (ah) a phenyl group, and (ai) a substituted phenyl group having, on a ring, 1 to 5 substituents each independently selected from the group consisting of a halogen atom, a cyano group, a $(C_1-C_6)$ alkyl group, a

$(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group, or (aj) a methylenedioxy group formed by two adjacent substituents together and optionally substituted by 1 or 2 substituents selected from a halogen atom, a phenyl group and a $(C_1-C_6)$alkyl group, provided that an atom adjacent to a bonding atom in the pyrazolyl group is not substituted by the $(C_1-C_6)$alkylsulfonyl group, the halo$(C_1-C_6)$alkylsulfonyl group, the N-$(C_1-C_6)$alkylaminosulfonyl group, or the $R^{10}$- ($R^{11}$-N=)O=S group wherein $R^{10}$ and $R^{11}$ are the same as above; (c19) a pyridyl group; (c20) a substituted pyridyl group having, on a ring, 1 to 4 substituents each independently selected from the group consisting of a halogen atom, a cyano group, a nitro group, a $(C_1-C_6)$alkyl group, a $(C_1-C_6)$ alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group, a halo $(C_1-C_6)$alkoxy group, a halo $(C_1-C_6)$alkylthio group, a halo $(C_1-C_6)$ alkylsulfinyl group and a halo$(C_1-C_6)$alkylsulfonyl group; (c23) a pyridazinyl group; (c24) a substituted pyridazinyl group having, on a ring, 1 to 3 substituents each independently selected from the group consisting of a halogen atom, a cyano group, a $(C_1-C_6)$alkyl group, a $(C_1-C_6)$ alkoxy group, a $(C_3-C_6)$ cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group and a halo $(C_1-C_6)$ alkoxy group; (c25) a pyrimidinyl group; (c26) a substituted pyrimidinyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo$(C_1-C_6)$alkoxy group; (c27) a pyrazinyl group; (c28) a substituted pyrazinyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c29) a triazinyl group; (c30) a substituted triazinyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group and a halo $(C_1-C_6)$alkoxy group; (c37) an oxazolyl group; (c38) a substituted oxazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$ alkylsulfinyl group, a $(C_1-C_6)$alkylsulfonyl group, a halo $(C_1-C_6)$alkyl group and a halo$(C_1-C_6)$alkoxy group; (c39) a thiazolyl group; (c40) a substituted thiazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$ cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c41) an isoxazolyl group; (c42) a substituted isoxazolyl group having, on a ring, 1 or 2 substituents each independently selected from the group consisting of a halogen atom, a cyano group, a $(C_1-C_6)$alkyl group, a $(C_1-C_6)$ alkoxy group, a $(C_3-C_6)$ cycloalkyl group, a $(C_1-C_6)$ alkylthio group, a $(C_1-C_6)$alkylsulfinyl group, a $(C_1-C_6)$alkylsulfonyl group, a halo $(C_1-C_6)$alkyl group and a halo $(C_1-C_6)$ alkoxy group; (c43) an isothiazolyl group; (c44) a substituted isothiazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c45) an imidazolyl group; (c46) a substituted imidazolyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c47) a pyrazolyl group; (c48) a substituted pyrazolyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo $(C_1-C_6)$alkoxy group; (c49) a triazolyl group; (c50) a substituted triazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group and a halo$(C_1-C_6)$alkoxy group; (c51) a tetrazolyl group; (c52) a substituted tetrazolyl group having, on a ring, one substituent selected from the group consisting of a halogen atom, a cyano group, a $(C_1-C_6)$ alkyl group, a $(C_1-C_6)$ alkoxy group, a $(C_3-C_6)$ cycloalkyl group, a $(C_1-C_6)$ alkylthio group, a $(C_1-C_6)$alkylsulfinyl group, a $(C_1-C_6)$alkylsulfonyl group, a halo $(C_1-C_6)$ alkyl group and a halo $(C_1-C_6)$alkoxy group; (c53) a thiadiazolyl group; (c54) a substituted thiadiazolyl group having, on a ring, one substituent selected from the group consisting of a halogen atom, a cyano group, a $(C_1-C_6)$ alkyl group, a $(C_1-C_6)$alkoxy group, a $(C_3-C_6)$ cycloalkyl group, a $(C_1-C_6)$alkylthio group, a $(C_1-C_6)$alkylsulfinyl group, a $(C_1-C_6)$ alkylsulfonyl group, a halo $(C_1-C_6)$alkyl group and a halo

$(C_1-C_6)$alkoxy group; (c55) a quinolinyl group; (c56) a substituted quinolinyl group having, on a ring, 1 to 5 substituents each independently selected from the group consisting of a halogen atom, a cyano group, a $(C_1-C_6)$ alkyl group, a $(C_1-C_6)$alkoxy group, a $(C_3-C_6)$ cycloalkyl group, a $(C_1-C_6)$ alkylthio group, a $(C_1-C_6)$alkylsulfinyl group, a $(C_1-C_6)$alkylsulfonyl group, a halo $(C_1-C_6)$alkyl group and a halo$(C_1-C_6)$alkoxy group; (c57) a benzoxazolyl group; (c58) a substituted benzoxazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c59) a benzothiazolyl group; (c60) a substituted benzothiazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group and a halo$(C_1-C_6)$alkoxy group; (c67) an indanyl group; (c68) a substituted indanyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c69) a tetrahydronaphthyl group; (c70) a substituted tetrahydronaphthyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c73) a phenyl $(C_1-C_6)$alkyl group; (c74) a substituted phenyl$(C_1-C_6)$alkyl group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c75) a $(C_1-C_6)$alkoxycarbonyl group; (c76) a phenylcarbonyl group; (c77) a thienyl group; (c78) a substituted thienyl group having, on a ring, 1 to 3 substituents each independently selected from the group consisting of a halogen atom, a cyano group, a nitro group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group, a halo$(C_1-C_6)$alkoxy group, a halo$(C_1-C_6)$alkylthio group, a halo$(C_1-C_6)$alkylsulfinyl group and a halo$(C_1-C_6)$alkylsulfonyl group; (c79) a naphthyl group; (c80) a substituted naphthyl group having, on a ring, 1 to 7 substituents each independently selected from the group consisting of a halogen atom, a cyano group, a nitro group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group, a halo $(C_1-C_6)$alkoxy group, a halo$(C_1-C_6)$alkylthio group, a halo$(C_1-C_6)$alkylsulfinyl group and a halo$(C_1-C_6)$alkylsulfonyl group; (c81) a 1,1-dioxidetetrahydro-2H-thiopyranyl group; (c82) an imidazopyridyl group; or (c83) a substituted imidazopyridyl group having, on a ring, 1 to 5 substituents each independently selected from the group consisting of a halogen atom, a cyano group, a nitro group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group, a halo$(C_1-C_6)$alkoxy group, a halo$(C_1-C_6)$alkylthio group, a halo$(C_1-C_6)$alkylsulfinyl group, a halo $(C_1-C_6)$alkylsulfonyl group and a phenyl group,

Q is

(d1) a substituted phenyl group having, on a ring, 1 to 5 substituents each independently selected from substituent group A; (d2) a naphthyl group; (d3) a substituted naphthyl group having, on a ring, 1 to 7 substituents each independently selected from substituent group B; (d4) a pyridyl group; (d5) a substituted pyridyl group having, on a ring, 1 to 4 substituents each independently selected from substituent group B; (d6) a pyridazinyl group; (d7) a substituted pyridazinyl group having, on a ring, 1 to 3 substituents each independently selected from substituent group B; (d8) a pyrimidinyl group; (d9) a substituted pyrimidinyl group having, on a ring, 1 to 3 substituents each independently selected from substituent group B; (d10) a pyrazinyl group; (d11) a substituted pyrazinyl group having, on a ring, 1 to 3 substituents each independently selected from substituent group B; (d12) a triazinyl group; (d13) a substituted triazinyl group having, on a ring, 1 or 2 substituents each independently selected from substituent group B; (d14) a 2-oxopyridyl group; (d15) a substituted 2-oxopyridyl group having, on a ring, 1 to 4 substituents each independently selected from substituent group B; (d16) an isoxazolyl group; (d17) a substituted isoxazolyl group having, on a ring, 1 or 2 substituents each independently selected from substituent group B; (d18) an oxazolyl group; (d19) a substituted oxazolyl group having, on a ring, 1 or 2 substituents each independently selected from substituent group B; (d20) a thienyl group; (d21) a substituted thienyl group having, on a ring, 1 to 3 substituents each independently selected from substituent group B; (d22) an isothiazolyl group; (d23) a substituted isothiazolyl group having, on a ring, 1 or 2 substituents each independently selected from substituent group B; (d24) a thiazolyl group; (d25) a substituted thiazolyl group having, on a ring, 1 or 2 substituents each independently selected from substituent group B; (d44) a benzothienyl group; (d45) a substituted benzothienyl group having, on a ring, 1 to 5 substituents each independently selected from

substituent group B; (d46) a benzothiazolyl group; (d47) a substituted benzothiazolyl group having, on a ring, 1 to 4 substituents each independently selected from substituent group B; (d48) a benzisothiazolyl group; (d49) a substituted benzisothiazolyl group having, on a ring, 1 to 4 substituents each independently selected from substituent group B; (d90) a quinoxalinyl group; (d91) a substituted quinoxalinyl group having, on a ring, 1 to 5 substituents each independently selected from substituent group B; (d92) a quinolinyl group; (d93) a substituted quinolinyl group having, on a ring, 1 to 6 substituents each independently selected from substituent group B; (d94) an isoquinolinyl group; (d95) a substituted isoquinolinyl group having, on a ring, 1 to 6 substituents each independently selected from substituent group B; (d100) a quinazolinyl group; (d101) a substituted quinazolinyl group having, on a ring, 1 to 5 substituents each independently selected from substituent group B; or (d104) a ferrocenyl group,
X and Y are each independently an oxygen atom or a sulfur atom,
Z is an $OR^4$ group wherein $R^4$ is a hydrogen atom, a $(C_1-C_6)$alkyl group, a $(C_1-C_6)$alkylcarbonyl group or a $(C_1-C_6)$alkoxycarbonyl group, an $S(O)nR^5$ group wherein n is 0, 1 or 2, and $R^5$ is a hydrogen atom, a $(C_1-C_6)$alkyl group or a phenyl$(C_1-C_6)$alkyl group, or an $N(R^6)R^7$ group wherein $R^6$ and $R^7$ are hydrogen atoms, hydroxyl groups, $(C_1-C_6)$alkyl groups, $(C_1-C_6)$alkoxy groups, $(C_1-C_6)$alkylcarbonyl groups or $(C_1-C_6)$alkoxycarbonyl groups, and $R^6$ and $R^7$ are the same or different,
substituent group A consists of

(e1) a cyano group; (e2) a nitro group; (e4) a carboxyl group;
(e5) a $(C_1-C_6)$alkyl group; (e6) a $(C_2-C_6)$alkenyl group; (e7) a $(C_2-C_6)$alkynyl group; (e8) a $(C_3-C_6)$cycloalkyl group; (e9) a substituted $(C_3-C_6)$cycloalkyl group having, on a ring, 1 to 3 substituents each independently selected from the group consisting of a halogen atom, a cyano group, a $(C_1-C_6)$alkyl group, a $(C_1-C_6)$alkoxy group and a $(C_1-C_6)$alkylcarbonyl group; (e10) a $(C_1-C_6)$alkoxy$(C_1-C_6)$alkyl group; (e11) a $(C_1-C_6)$alkylthio group; (e12) a $(C_1-C_6)$alkylsulfinyl group; (e13) a $(C_1-C_6)$alkylsulfonyl group; (e14) a halo$(C_1-C_6)$alkyl group; (e17) a halo$(C_1-C_6)$alkoxy group; (e18) a halo$(C_3-C_6)$cycloalkyl group; (e19) a halo $(C_1-C_6)$alkylthio group; (e20) a halo$(C_1-C_6)$alkylsulfinyl group; (e21) a halo$(C_1-C_6)$alkylsulfonyl group; (e22) an $SF_5$ group; (e23) a $(C_1-C_6)$alkylcarbonylamino group; (e24) a halo$(C_1-C_6)$alkylcarbonylamino group; (e25) a $(C_1-C_6)$alkylsulfonylamino group; (e26) a halo$(C_1-C_6)$alkylsulfonylamino group; (e27) an N,N-di$(C_1-C_6)$alkylcarboxamido group; (e28) an N-halo$(C_1-C_6)$alkylcarboxamido group; (e29) a $(C_1-C_6)$alkoxycarbonyl group; (e30) an N-$(C_1-C_6)$alkylaminosulfonyl group; (e32) a pyrazolyl group; (e33) a substituted pyrazolyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (e36) a phenyl group; (e37) a substituted phenyl group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; . (e38) a phenoxy group; (e39) a substituted phenoxy group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (e40) a methylenedioxy group formed by two adjacent substituents together and optionally substituted by 1 or 2 substituents selected from the group consisting of a halogen atom, a phenyl group and a $(C_1-C_6)$alkyl group; (e41) an $R^B$-($R^9$-N=)O=S group wherein $R^8$ is a $(C_1-C_6)$alkyl group, a $(C_3-C_6)$cycloalkyl group, a halo$(C_1-C_6)$alkyl group, or a $(C_1-C_6)$alkoxy$(C_1-C_6)$alkyl group, and $R^9$ is a hydrogen atom, a cyano group, a $(C_1-C_6)$alkyl group, a $(C_3-C_6)$cycloalkyl group, a halo$(C_1-C_6)$alkyl group, a $(C_1-C_6)$alkylcarbonyl group, or a halo $(C_1-C_6)$alkylcarbonyl group; (e42) a carboxamido group; (e43) a morpholinyl group; (e44) an N,N-di$(C_1-C_6)$alkylamino group; (e45) an $R^8$-($R^9$-N=)S group wherein $R^8$ and $R^9$ are the same as above; (e46) an N-$(C_1-C_6)$alkylcarboxamido group; (e47) a phenyl$(C_1-C_6)$alkylthio group; and (e48) a substituted phenyl$(C_1-C_6)$alkylthio group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group, and

substituent group B consists of
(f1) a halogen atom; (f2) a cyano group; (f3) a nitro group; (f6) a $(C_1-C_6)$ alkyl group; (f7) a $(C_2-C_6)$alkenyl group; (f8) a $(C_2-C_6)$alkynyl group; (f9) a $(C_1-C_6)$alkoxy group; (f10) a $(C_3-C_6)$cycloalkyl group; (f11) a substituted $(C_3-C_6)$cycloalkyl group having, on a ring, 1 to 3 substituents each independently selected from the group

consisting of a halogen atom, a cyano group, a ($C_1$-$C_6$)alkyl group, a ($C_1$-$C_6$)alkoxy group and a ($C_1$-$C_6$)alkylcarbonyl group; (f12) a ($C_1$-$C_6$)alkoxy($C_1$-$C_6$)alkyl group; (f13) a ($C_1$-$C_6$)alkylthio group; (f14) a ($C_1$-$C_6$)alkylsulfinyl group; (f15) a ($C_1$-$C_6$)alkylsulfonyl group; (f16) a halo($C_1$-$C_6$)alkyl group; (f19) a halo($C_1$-$C_6$)alkoxy group; (f20) a halo($C_3$-$C_6$)cycloalkyl group; (f21) a halo($C_1$-$C_6$)alkylthio group; (f22) a halo($C_1$-$C_6$)alkylsulfinyl group; (f23) a halo ($C_1$-$C_6$)alkylsulfonyl group; (f25) a ($C_1$-$C_6$)alkylcarbonylamino group; (f26) a halo($C_1$-$C_6$)alkylcarbonylamino group; (f27) a ($C_1$-$C_6$)alkylsulfonylamino group; (f28) a halo($C_1$-$C_6$)alkylsulfonylamino group; (f29) an N,N-di($C_1$-$C_6$)alkylcarboxamido group; (f30) an N-halo($C_1$-$C_6$)alkylcarboxamido group; (f31) a ($C_1$-$C_6$)alkoxycarbonyl group; (f34) a pyrazolyl group; (f35) a substituted pyrazolyl group having, on a ring, 1 to 3 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo($C_1$-$C_6$)alkoxy group; (f38) a phenyl group; (f39) a substituted phenyl group having, on a ring, 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$)alkoxy group; (f40) a phenoxy group; (f41) a substituted phenoxy group having, on a ring, 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo($C_1$-$C_6$)alkoxy group; (f42) a methylenedioxy group formed by two adjacent substituents together and optionally substituted by 1 or 2 substituents selected from the group consisting of a halogen atom, a phenyl group and a ($C_1$-$C_6$)alkyl group; (f44) a carboxamido group; (f45) a morpholinyl group; and (f46) an N,N-di($C_1$-$C_6$)alkylamino group,

[3] the compound and salts thereof of [1] or [2], wherein

$R^1$ is
(a1) a hydrogen atom; (a2) a ($C_1$-$C_6$)alkyl group; or (a4) a phenyl group,
$R^2$ is (b1) a hydrogen atom,
$R^3$ is
(c1) a ($C_1$-$C_6$)alkyl group; (c2) a ($C_2$-$C_6$)alkenyl group; (c4) a ($C_3$-$C_6$)cycloalkyl group; (c8') a substituted ($C_1$-$C_6$)alkyl group having, on a ring, 1 to 3 substituents each independently selected from the group consisting of a ($C_1$-$C_6$)alkoxy group, a ($C_1$-$C_6$)alkoxycarbonyl group and a ($C_2$-$C_6$)alkenylcarbonyloxy group; (c18') a substituted phenyl group having, on a ring, 1 to 5 substituents each independently selected from the group consisting of (a) a halogen atom, (b) a cyano group, (c) a nitro group, (e) a carboxyl group, (f) a ($C_1$-$C_6$) alkyl group, (g) a ($C_1$-$C_6$)alkoxy group, (h) a ($C_3$-$C_6$)cycloalkyl group, (i) a ($C_1$-$C_6$)alkylthio group, (j) a ($C_1$-$C_6$)alkylsulfinyl group, (k) a ($C_1$-$C_6$)alkylsulfonyl group, (l) a halo($C_1$-$C_6$)alkyl group, (m) a halo ($C_1$-$C_6$)alkoxy group, (o) a halo($C_1$-$C_6$)alkylthio group, (p) a halo($C_1$-$C_6$)alkylsulfinyl group, (q) a halo($C_1$-$C_6$)alkylsulfonyl group, (r) a ($C_1$-$C_6$)alkylcarbonyl group, (s) a ($C_1$-$C_6$)alkoxycarbonyl group, (t) a ($C_1$-$C_6$)alkylcarbonylamino group, (v) a ($C_1$-$C_6$)alkylsulfonylamino group, (z) an N-halo($C_1$-$C_6$)alkylcarboxamido group, (aa) an N-($C_1$-$C_6$)alkylaminosulfonyl group, (ab) a ($C_1$-$C_6$)alkoxy($C_1$-$C_6$)alkyl group, (ac) an $SF_5$ group, (ae') an $R^{10}$-($R^{11}$-N=)S group wherein $R^{10}$ is ($C_1$-$C_6$)alkyl, and $R^{11}$ is a cyano group, (ag) a phenoxy group and (ah) (aj') a phenyl group, or a methylenedioxy group formed by two adjacent substituents together; (c19) a pyridyl group; (c20') a substituted pyridyl group having, on a ring, 1 to 4 substituents each independently selected from the group consisting of a halogen atom and a halo($C_1$-$C_6$)alkyl group; (c24') a substituted pyridazinyl group having, on a ring, 1 to 3 substituents each independently selected from halogen atoms; (c26') a substituted pyrimidinyl group having, on a ring, 1 to 3 substituents each independently selected from halogen atoms; (c27) a pyrazinyl group; (c37) an oxazolyl group; (c39) a thiazolyl group; (c42') a substituted isoxazolyl group having, on a ring, 1 or 2 substituents each independently selected from ($C_1$-$C_6$)alkyl groups; (c44') a substituted isothiazolyl group having, on a ring, 1 or 2 substituents each independently selected from ($C_1$-$C_6$)alkyl groups; (c45) an imidazolyl group; (c48') a substituted pyrazolyl group having, on a ring, 1 to 3 substituents each independently selected from ($C_1$-$C_6$)alkyl groups; (c49) a triazolyl group; (c52') a substituted tetrazolyl group having, on a ring, one substituent selected from a ($C_1$-$C_6$)alkyl group; (c54') a substituted thiadiazolyl group having, on a ring, one substituent selected from a halo($C_1$-$C_6$)alkyl group; (c55) a quinolinyl group; (c58') a substituted benzoxazolyl group having, on a ring, 1 to 4 substituents each independently selected from halogen atoms; (c59) a benzothiazolyl group; (c73) a phenyl($C_1$-$C_6$)alkyl group; (c74') a substituted phenyl($C_1$-$C_6$)alkyl group having, on a ring, 1 to 5 substituents each independently selected from halogen atoms; (c75) a ($C_1$-$C_6$)alkoxycarbonyl group; (c76) a phenylcarbonyl group; (c77) a thienyl group; (c79) a naphthyl group; (c81) a 1,1-dioxidetetrahydro-2H-thiopyranyl group; or (c83') a substituted imidazopyridyl group having, on a ring, 1 to 5 phenyl groups,
Q is

(d1) a substituted phenyl group having, on a ring, 1 to 5 substituents each independently selected from substituent group A; (d2) a naphthyl group; (d5) a substituted pyridyl group having, on a ring, 1 to 4 substituents each independently selected from substituent group B; (d8) a pyrimidinyl group; (d17) a substituted isoxazolyl group having, on a ring, 1 or 2 substituents each independently selected from substituent group B; (d18) an oxazolyl group; (d20) a thienyl group; (d21) a substituted thienyl group having, on a ring, 1 to 3 substituents each independently selected from substituent group B; (d22) an isothiazolyl group; (d24) a thiazolyl group; (d92) a quinolinyl group; or (d104) a ferrocenyl group,

X and Y are each independently an oxygen atom or a sulfur atom,

Z is an $OR^4$ group wherein $R^4$ is a hydrogen atom, an $S(O)nR^5$ group wherein n is 0, and $R^5$ is a phenyl($C_1$-$C_6$)alkyl group, or an $N(R^6)R^7$ group wherein $R^6$ and $R^7$ are hydrogen atoms, hydroxyl groups, or ($C_1$-$C_6$)alkoxy groups, and $R^6$ and $R^7$ are the same or different, substituent group A consists of

(e1) a cyano group; (e2) a nitro group; (e4) a carboxyl group; (e5) a ($C_1$-$C_6$)alkyl group; (e11) a ($C_1$-$C_6$)alkylthio group; (e12) a ($C_1$-$C_6$)alkylsulfinyl group; (e13) a ($C_1$-$C_6$)alkylsulfonyl group; (e14) a halo($C_1$-$C_6$)alkyl group; (e17) a halo($C_1$-$C_6$)alkoxy group; (e19) a halo ($C_1$-$C_6$)alkylthio group; (e20) a halo($C_1$-$C_6$)alkylsulfinyl group; (e21) a halo($C_1$-$C_6$)alkylsulfonyl group; (e29) a ($C_1$-$C_6$)alkoxycarbonyl group; (e38) a phenoxy group; (e40') a methylenedioxy group formed by two adjacent substituents together; (e42) a carboxamido group; (e43) a morpholinyl group; (e44) an N,N-di($C_1$-$C_6$)alkylamino group; (e45') an $R^8$-($R^9$-N=)S group wherein $R^8$ is a ($C_1$-$C_6$)alkyl group, and $R^9$ is a cyano group; (e46) an N-($C_1$-$C_6$)alkylcarboxamido group; and (e48') a substituted phenyl($C_1$-$C_6$)alkylthio group having, on a ring, 1 to 5 substituents each independently selected from ($C_1$-$C_6$)alkyl groups, and substituent group B consists of

(f1) a halogen atom; (f6) a ($C_1$-$C_6$)alkyl group; (f16) a halo($C_1$-$C_6$)alkyl group; and (f38) a phenyl group,

[4] a pest control agent comprising the compound or salts thereof of any one of [1] to [3] as an active ingredient,

[5] an agrohorticultural insect pest control agent comprising the compound or salts thereof of any one of [1] to [3] as an active ingredient,

[6] an ectoparasite control agent for animals, comprising the compound or salts thereof of any one of [1] to [3] as an active ingredient,

[7] an endoparasite control agent for animals, comprising the compound or salts thereof of any one of [1] to [3] as an active ingredient,

[8] a method for controlling an agrohorticultural insect pest, comprising treating a plant or soil with an effective amount of the agrohorticultural insect pest control agent of [5],

[9] a method for controlling an ectoparasite in an animal, comprising orally or parenterally administering an effective amount of the ectoparasite control agent for animals of [6] to a target animal,

[10] a method for controlling an endoparasite in an animal, comprising orally or parenterally administering an effective amount of the endoparasite control agent for animals of [7] to a target animal,

[11] use of the compound or salts thereof of any one of [1] to [3] as a pest control agent.

[Advantageous Effects of Invention]

The compounds or salts thereof of the present invention provide arylcyclohexanedione derivatives represented by the formula (1) and having superior effects as pest control agents.

[Description of Embodiments]

In the definition of the formula (I) of the compound of the present invention, the "halo" means a "halogen atom", and is a chlorine atom, a bromine atom, an iodine atom or a fluorine atom.

The "($C_1$-$C_6$)alkyl group" is a straight chain or branched chain alkyl group having 1 to 6 carbon atoms such as methyl group, ethyl group, normal propyl group, isopropyl group, normal butyl group, isobutyl group, secondary butyl group, tertiary butyl group, normal pentyl group, isopentyl group, tertiary pentyl group, neopentyl group, 2,3-dimethylpropyl group, 1-ethylpropyl group, 1-methylbutyl group, 2-methylbutyl group, normal hexyl group, isohexyl group, 2-hexyl group, 3-hexyl group, 2-methylpentyl group, 3-methylpentyl group, 1,1,2-trimethylpropyl group, 3,3-dimethylbutyl group, and the like.

The "($C_2$-$C_6$)alkenyl group" is a straight chain or branched chain alkenyl group having 2 to 6 carbon atoms such as vinyl group, allyl group, isopropenyl group, 1-butenyl group, 2-butenyl group, 2-methyl-2-propenyl group, 1-methyl-2-propenyl group, 2-methyl-1-propenyl group, pentenyl group, 1-hexenyl group, 3,3-dimethyl-1-butenyl group, and the like, and the "($C_2$-$C_6$)alkynyl group" is a straight chain or branched chain alkynyl group having 2 to 6 carbon atoms such as ethynyl group, 1-propynyl group, 2-propynyl group, 1-butynyl group, 2-butynyl group, 3-butynyl group,

3-methyl-1-propynyl group, 2-methyl-3-propynyl group, pentynyl group, 1-hexynyl group, 3-methyl-1-butynyl group, 3,3-dimethyl-1-butynyl group, and the like.

The "($C_3$-$C_6$)cycloalkyl group" is a cyclic alkyl group having 3 to 6 carbon atoms such as cyclopropyl group, cyclobutyl group, cyclopentyl group, cyclohexyl group, and the like, and the "($C_1$-$C_6$)alkoxy group" is a straight chain or branched chain alkoxy group having 1 to 6 carbon atoms such as methoxy group, ethoxy group, normal propoxy group, isopropoxy group, normal butoxy group, secondary butoxy group, tertiary butoxy group, normal pentyloxy group, isopentyloxy group, tertiary pentyloxy group, neopentyloxy group, 2,3-dimethylpropyloxy group, 1-ethylpropyloxy group, 1-methylbutyloxy group, normal hexyloxy group, isohexyloxy group, 1,1,2-trimethylpropyloxy group, and the like.

The "($C_1$-$C_6$)alkylthio group" is a straight chain or branched chain alkylthio group having 1 to 6 carbon atoms such as methylthio group, ethylthio group, normal propylthio group, isopropylthio group, normal butylthio group, secondary butylthio group, tertiary butylthio group, normal pentylthio group, isopentylthio group, tertiary pentylthio group, neopentylthio group, 2,3-dimethylpropylthio group, 1-ethylpropylthio group, 1-methylbutylthio group, normal hexylthio group, isohexylthio group, 1,1,2-trimethylpropylthio group, and the like.

The "($C_1$-$C_6$)alkylsulfinyl group" is a straight chain or branched chain alkylsulfinyl group having 1 to 6 carbon atoms such as methylsulfinyl group, ethylsulfinyl group, normal propylsulfinyl group, isopropylsulfinyl group, normal butylsulfinyl group, secondary butylsulfinyl group, tertiary butylsulfinyl group, normal pentylsulfinyl group, isopentylsulfinyl group, tertiary pentylsulfinyl group, neopentylsulfinyl group, 2,3-dimethylpropylsulfinyl group, 1-ethylpropylsulfinyl group, 1-methylbutylsulfinyl group, normal hexylsulfinyl group, isohexyl sulfinyl group, 1,1,2-trimethylpropylsulfinyl group, and the like.

The "($C_1$-$C_6$)alkylsulfonyl group" is a straight chain or branched chain alkylsulfonyl group having 1 to 6 carbon atoms such as methylsulfonyl group, ethylsulfonyl group, normal propylsulfonyl group, isopropylsulfonyl group, normal butylsulfonyl group, secondary butylsulfonyl group, tertiary butylsulfonyl group, normal pentylsulfonyl group, isopentylsulfonyl group, tertiary pentylsulfonyl group, neopentylsulfonyl group, 2,3-dimethylpropylsulfonyl group, 1-ethylpropylsulfonyl group, 1-methylbutylsulfonyl group, normal hexylsulfonyl group, isohexylsulfonyl group, 1,1,2-trimethylpropylsulfonyl group, and the like.

The "($C_1$-$C_6$)alkylcarbonyl group" is an alkylcarbonyl group having 2 to 7 carbon atoms, for example, an alkylcarbonyl group having the aforementioned ($C_1$-$C_6$)alkyl group, such as acetyl group, propanoyl group, butanoyl group, 2-methylpropanoyl group, pentanoyl group, 2-methylbutanoyl group, 3-methylbutanoyl group, pivaloyl group, hexanoyl group, and the like.

The "($C_1$-$C_6$)alkylcarbonylamino group" is an alkylcarbonylamino group having 2 to 7 carbon atoms, for example, an alkylcarbonylamino group having the aforementioned ($C_1$-$C_6$)alkyl group, such as acetylamino group, propanoylamino group, butanoylamino group, 2-methylpropanoylamino group, pentanoylamino group, 2-methylbutanoylamino group, 3-methylbutanoylamino group, pivaloylamino group, hexanoylamino group, and the like.

The "($C_1$-$C_6$)alkylsulfonylamino group is a straight chain or branched chain alkylsulfonylamino group having 1 to 6 carbon atoms, such as methylsulfonylamino group, ethylsulfonylamino group, normal propylsulfonylamino group, isopropylsulfonylamino group, normal butylsulfonylamino group, secondary butylsulfonylamino group, tertiary butylsulfonylamino group, normal pentylsulfonylamino group, isopentylsulfonylamino group, tertiary pentylsulfonylamino group, neopentylsulfonylamino group, 2,3-dimethylpropylsulfonylamino group, 1-ethylpropylsulfonylamino group, 1-methylbutylsulfonylamino group, normal hexylsulfonylamino group, isohexylsulfonylamino group, 1,1,2-trimethylpropylsulfonylamino group, and the like.

The "N-($C_1$-$C_6$)alkylcarboxamido group" is an alkylcarboxamido group having 2 to 7 carbon atoms and a straight chain or branched chain alkyl group having 1 to 6 carbon atoms, such as N-methylcarboxamido group, N-ethylcarboxamido group, N-normal-propylcarboxamido group, N-isopropylcarboxamido group, N-normal-butylcarboxamido group, N-isobutylcarboxamido group, N-secondary butylcarboxamido group, N-tertiary butylcarboxamido group, N-normal-pentylcarboxamido group, N-isopentylcarboxamido group, N-tertiary pentylcarboxamido group, N-neopentycarboxamido group, N-normal-hexylcarboxamido group, N-isohexylcarboxamido group, and the like.

The "N,N-di($C_1$-$C_6$)alkylcarboxamido group" is a straight chain or branched chain dialkylcarboxamido group having 1 to 6 carbon atoms, such as N,N-dimethylcarboxamido group, N,N-diethylcarboxamido group, N,N-di-normal-propylcarboxamido group, N,N-diisopropylcarboxamido group, N,N-di-normal-butylcarboxamido group, N,N-di-secondary-butylcarboxamido group, N,N-di-tertiary-butylcarboxamido group, N-methyl-N-ethylcarboxamido group, N-methyl-N-normal-propylcarboxamido group, N-methyl-N-isopropylcarboxamido group, N-methyl-N-normal-butylcarboxamido group, N-methyl-N-secondary butylcarboxamido group, N-methyl-N-tertiary butylcarboxamido group, N-methyl-N-normal-pentylcarboxamido group, N-methyl-N-isopentylcarboxamido group, N-methyl-N-tertiary pentylcarboxamido group, N-methyl-N-neopentylcarboxamido group, N-methyl-N-(2,3-dimethylpropyl)carboxamido group, N-methyl-N-(1-ethylpropyl)carboxamido group, N-methyl-N-(1-methylbutyl)carboxamido group, N-methyl-N-normal-hexylcarboxamido group, N-methyl-N-isohexylcarboxamido group, N-methyl-N-(1,1,2-trimethylpropyl)carboxamido group, and the like.

The "($C_1$-$C_6$)alkoxycarbonyl group" is an alkoxycarbonyl group having 2 to 7 carbon atoms, for example, an alkoxycarbonyl group having the aforementioned ($C_1$-$C_6$)alkoxy group, such as methoxycarbonyl group, ethoxycarbonyl

group, normal propoxycarbonyl group, isopropoxycarbonyl group, normal butoxycarbonyl group, isobutoxycarbonyl group, secondary butoxycarbonyl group, tertiary butoxycarbonyl group, pentyloxycarbonyl group, and the like.

The "N-($C_1$-$C_6$)alkylaminosulfonyl group" is a straight chain or branched chain alkylaminosulfonyl group having 1 to 6 carbon atoms, such as N-methylaminosulfonyl group, N-ethylaminosulfonyl group, N-normal-propylaminosulfonyl group, N-isopropylaminosulfonyl group, N-normal-butylaminosulfonyl group, N-secondary butylaminosulfonyl group, N-tertiary butylaminosulfonyl group, N-normal-pentylaminosulfonyl group, N-isopentylaminosulfonyl group, N-tertiary pentylaminosulfonyl group, N-neopentylaminosulfonyl group, N-(2,3-dimethylpropyl)aminosulfonyl group, N-(1-ethylpropyl)aminosulfonyl group, N-(1-methylbutyl)aminosulfonyl group, N-normal-hexylaminosulfonyl group, N-isohexylaminosulfonyl group, N-(1,1,2-trimethylpropyl)aminosulfonyl group, and the like.

The "N,N-di($C_1$-$C_6$)alkylaminosulfonyl group" is a straight chain or branched chain dialkylaminosulfonyl group having 1 to 6 carbon atoms, such as N,N-dimethylaminosulfonyl group, N,N-diethylaminosulfonyl group, N,N-di-normal-propylaminosulfonyl group, N,N-diisopropylaminosulfonyl group, N,N-di-normal-butylaminosulfonyl group, N,N-di-secondary-butylaminosulfonyl group, N,N-di-tertiary-butylaminosulfonyl group, N-methyl-N-ethylaminosulfonyl group, N-methyl-N-normal-propylaminosulfonyl group, N-methyl-N-isopropylaminosulfonyl group, N-methyl-N-normal-butylaminosulfonyl group, N-methyl-N-secondary butylaminosulfonyl group, N-methyl-N-tertiary butylaminosulfonyl group, N-methyl-N-normal-pentylaminosulfonyl group, N-methyl-N-isopentylaminosulfonyl group, N-methyl-N-tertiary pentylaminosulfonyl group, N-methyl-N-neopentylaminosulfonyl group, N-methyl-N-(2,3-dimethylpropyl)aminosulfonyl group, N-methyl-N-(1-ethylpropyl)aminosulfonyl group, N-methyl-N-(1-methylbutyl)aminosulfonyl group, N-methyl-N-normal-hexylaminosulfonyl group, N-methyl-N-isohexylaminosulfonyl group, N-methyl-N-(1,1,2-trimethylpropyl)aminosulfonyl group, and the like.

The "N,N-di($C_1$-$C_6$)alkylamino group" is a straight chain or branched chain dialkylamino group having 1 to 6 carbon atoms such as N,N-dimethylamino group, N,N-diethylamino group, N,N-di-normal-propylamino group, N,N-diisopropylamino group, N,N-di-normal-butylamino group, N,N-di-secondary-butylamino group, N,N-di-tertiary-butylamino group, N-methyl-N-ethylamino group, N-methyl-N-normal-propylamino group, N-methyl-N-isopropylamino group, N-methyl-N-normal-butylamino group, N-methyl-N-secondary butylamino group, N-methyl-N-tertiary butylamino group, N-methyl-N-normal-pentylamino group, N-methyl-N-isopentylamino group, N-methyl-N-tertiary pentylamino group, N-methyl-N-neopentylamino group, N-methyl-N-(2,3-dimethylpropyl)amino group, N-methyl-N-(1-ethylpropyl)amino group, N-methyl-N-(1-methylbutyl)amino group, N-methyl-N-normal-hexylamino group, N-methyl-N-isohexylamino group, N-methyl-N-(1,1,2-trimethylpropyl)amino group, and the like.

The above-mentioned "($C_1$-$C_6$)alkyl group", "($C_2$-$C_6$)alkenyl group", "($C_2$-$C_6$) alkynyl group", "($C_1$-$C_6$)alkoxy group", "($C_1$-$C_6$)alkylthio group", "($C_1$-$C_6$)alkylsulfinyl group", "($C_1$-$C_6$)alkylsulfonyl group", "($C_3$-$C_6$)cycloalkyl group", "($C_1$-$C_6$)alkylcarbonylamino group", " ($C_1$-$C_6$)alkylsulfonylamino group", "N-($C_1$-$C_6$)alkylcarboxamido group", "N, N-di($C_1$-$C_6$)alkylamino group", "N,N-di($C_1$-$C_6$)alkylcarboxamido group", and the like may be substituted by one or more halogen atoms at substitutable position(s) and, when substituted by two or more halogen atoms, the halogen atoms may be the same or different.

They are respectively denoted as "halo($C_1$-$C_6$)alkyl group", "halo($C_2$-$C_6$)alkenyl group", "halo($C_2$-$C_6$)alkynyl group", "halo ($C_1$-$C_6$)alkoxy group", "halo($C_1$-$C_6$)alkylthio group", "halo ($C_1$-$C_6$)alkylsulfinyl group", "halo($C_1$-$C_6$)alkylsulfonyl group", "halo($C_3$-$C_6$)cycloalkyl group", "halo($C_1$-$C_6$)alkylcarbonylamino group", "halo($C_1$-$C_6$)alkylsulfonylamino group", "N-halo ($C_1$-$C_6$)alkylcarboxamido group", "N,N-dihalo($C_1$-$C_6$)alkylamino group", "N,N-dihalo($C_1$-$C_6$)alkylcarboxamido group", and the like.

The expressions of "($C_1$-$C_6$)", "($C_2$-$C_6$)", "($C_3$-$C_6$)", and the like show ranges of the carbon atom numbers of various substituents. Furthermore, the above-mentioned definition applies to the groups to which the above-mentioned substituents are bonded. For example, "($C_1$-$C_6$)alkoxy($C_1$-$C_6$)alkyl group" means that a straight chain or branched chain alkoxy group having 1 to 6 carbon atoms is bonded to a straight chain or branched chain alkyl group having 1 to 6 carbon atoms.

Examples of the salts of the compound represented by the formula (1) of the present invention include inorganic acid salts such as hydrochloride, sulfate, nitrate, phosphate, and the like, organic acid salts such as acetate, fumarate, maleate, oxalate, methanesulfonate, benzenesulfonate, paratoluenesulfonate, and the like, and salts with inorganic or organic bases such as sodium ion, potassium ion, calcium ion, trimethylammonium, and the like.

The compound represented by the formula (1) and salts thereof of the present invention may contain one or plural number of asymmetric centers in the structural formula, and in some cases, two or more optical isomers and diastereomers may be present. The present invention encompasses any of such optical isomers and mixtures containing them at any ratio. In addition, the compound represented by the formula (1) and salts thereof of the present invention may have two types of geometric isomers derived from a C-C double bond in the structural formula. The present invention encompasses all of geometric isomers and the mixtures containing them at any ratio. Furthermore, the compound represented by the formula (1) and salts thereof of the present invention may have a plurality of tautomers. The present invention encompasses all of tautomers and the mixtures containing them at any ratio.

Preferred embodiments of the compound represented by the formula (1) of the present invention are shown

below.

In the formula (1), $R^1$ is preferably a group of the above-mentioned (a1), (a2), (a3), (a4), or (a5), more preferably a group of the above-mentioned (a1), (a2), or (a4).

In the formula (1), $R^2$ is preferably a group of the above-mentioned (b1), or (b2), more preferably a group of the above-mentioned (b1).

In the formula (1), $R^3$ is preferably a group of the above-mentioned (c1), (c2), (c3), (c4), (c5), (c8), (c9), (c18), (c19), (c20), (c23), (c24), (c25), (c26), (c27), (c28), (c29), (c30), (c37), (c38), (c39), (c40), (c41), (c42), (c43), (c44), (c45), (c46), (c47), (c48), (c49), (c50), (c51), (c52), (c53), (c54), (c55), (c56), (c57), (c58), (c59), (c60), (c67), (c68), (c69), (c70), (c73), (c74), (c75), (c76), (c77), (c78), (c79), (c80), (c81), (c82), or (c83),

more preferably a group of the above-mentioned (c1), (c2), (c4), (c8'), (c18'), (c19), (c20'), (c24'), (c26'), (c27), (c37), (c39), (c42'), (c44'), (c45), (c48'), (c49), (c52'), (c54'), (c55), (c58'), (c59), (c73), (c74'), (c75), (c76), (c77), (c79), (c81), or (c83').

In the formula (1), Q is preferably a group of the above-mentioned (d1), (d2), (d3), (d4), (d5), (d6), (d7), (d8), (d9), (d10), (d11), (d12), (d13), (d14), (d15), (d16), (d17), (d18), (d19), (d20), (d21), (d22), (d23), (d24), (d25), (d44), (d45), (d46), (d47), (d48), (d49), (d90), (d91), (d92), (d93), (d94), (d95), (d100), (d101), (d104),

more preferably a group of the above-mentioned (d1), (d2), (d5), (d8), (d17), (d18), (d20), (d21), (d22), (d24), (d92), or (d104).

In the formula (1), X is preferably an oxygen atom.

In the formula (1), Y is preferably an oxygen atom or a sulfur atom.

In the formula (1), X is preferably an oxygen atom and Y is preferably a sulfur atom.

In the formula (1), Z is preferably an $OR^4$ group wherein $R^4$ is a hydrogen atom, a $(C_1-C_6)$alkyl group, a $(C_1-C_6)$alkylcarbonyl group or a $(C_1-C_6)$alkoxycarbonyl group, an S(O)n$R^5$ group wherein n is 0, 1 or 2, and $R^5$ is a hydrogen atom, a $(C_1-C_6)$alkyl group, or a phenyl $(C_1-C_6)$alkyl group, or an $N(R^6)R^7$ group wherein $R^6$ and $R^7$ are hydrogen atoms, hydroxyl groups, $(C_1-C_6)$alkyl groups, $(C_1-C_6)$alkoxy groups, $(C_1-C_6)$alkylcarbonyl groups, or $(C_1-C_6)$alkoxycarbonyl groups, and $R^6$ and $R^7$ are the same or different,

more preferably an $OR^4$ group wherein $R^4$ is a hydrogen atom, an S(O)n$R^5$ group wherein n is 0, and $R^5$ is a phenyl$(C_1-C_6)$alkyl group, or an $N(R^6)R^7$ group wherein $R^6$ and $R^7$ are hydrogen atoms, hydroxyl groups, or $(C_1-C_6)$alkoxy groups, and $R^6$ and $R^7$ are the same or different.

In the formula (1), the substituent group A preferably consists of the groups of the above-mentioned (e1), (e2), (e4), (e5), (e6), (e7), (e8), (e9), (e10), (e11), (e12), (e13), (e14), (e17), (e18), (e19), (e20), (e21), (e22), (e23), (e24), (e25), (e26), (e27), (e28), (e29), (e30), (e32), (e33), (e36), (e37), (e38), (e39), (e40), (e41), (e42), (e43), (e44), (e45), (e46),

(e47), and (e48), more preferably the groups of the above-mentioned (e1), (e2), (e4), (e5), (e11), (e12), (e13), (e14), (e17), (e19), (e20), (e21), (e29), (e38), (e40'), (e42), (e43), (e44), (e45'), (e46), and (e48') .

In the formula (1), substituent group B preferably consists of the groups of the above-mentioned (f1), (f2), (f3), (f6), (f7), (f8), (f9), (f10), (f11), (f12), (f13), (f14), (f15), (f16), (f19), (f20), (f21), (f22), (f23), (f25), (f26), (f27), (f28), (f29), (f30), (f31), (f34), (f35), (f38), (f39), (f40), (f41), (f42), (f44), (f45), and (f46),

more preferably the groups of the above-mentioned (f1), (f6), (f16), and (f38).

Various compounds of the present invention can be produced by, for example, the following production methods, and the present invention is not limited thereto.

Production method 1

A compound represented by the formula (1-1) of the present invention can be produced from a known compound represented by the formula (2) by the following step [a]:

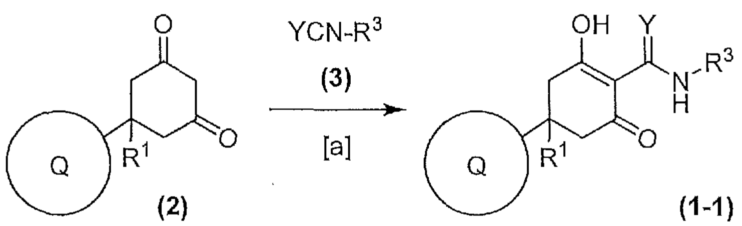


wherein $R^1$, $R^3$, Q, and Y are the same as above.

Production method of step [a]

A compound represented by the formula (1-1) of the present invention can be produced by reacting a compound represented by the formula (2) with a compound represented by the formula (3) in the presence of a base and an inert solvent.

Examples of the base that can be used in this reaction include hydroxides such as lithium hydroxide, sodium hydroxide, potassium hydroxide, calcium hydroxide, and the like, alkoxides such as sodium methoxide, sodium ethoxide, sodium tertiary-butoxide, potassium tertiary-butoxide, and the like, alkali metal hydrides such as sodium hydride, potassium hydride, and the like, carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like, acetates such as lithium acetate, sodium acetate, potassium acetate, and the like, organic bases such as pyridine, picoline, lutidine, triethylamine, tributylamine, N,N-diisopropylethylamine, and the like, and the like. The amount thereof to be used is generally 1-fold mol to 10-fold mol with respect to a compound represented by the formula (2) .

An inert solvent that can be used in this reaction may be any as long as it does not markedly inhibit the progress of this reaction. Examples thereof include inert solvents such as chain or cyclic saturated hydrocarbons such as pentane, hexane, cyclohexane, and the like, aromatic hydrocarbons such as benzene, toluene, xylene, and the like, halogenated aromatic hydrocarbons such as chlorobenzene, dichlorobenzene, and the like, chain or cyclic ethers such as diethyl ether, methyl tertiary-butyl ether, dioxane, tetrahydrofuran, and the like, and the like, nitriles such as acetonitrile, propionitrile, and the like, aprotic polar solvents such as N,N-dimethylformamide, N,N-dimethylacetamide, dimethyl sulfoxide, 1,3-dimethyl-2-imidazolidinone, and the like, alcohols such as methanol, ethanol, propanol, butanol, 2-propanol, and the like, and the like. These inert solvents can be used alone or a mixture of two or more kinds thereof may be used. The amount thereof to be used can be appropriately selected from the range of generally 0.1 to 100 L per 1 mol of a compound represented by the formula (2).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary.

Production method 2

A compound represented by the formula (1-2) of the present invention can be produced from a known compound represented by the formula (2) by the following step [b].

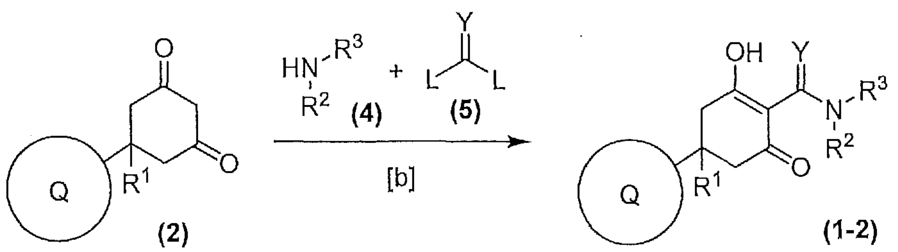

(2) (1-2)

wherein $R^1$, $R^2$, $R^3$, Q, and Y are the same as above, and L is a leaving group such as chlorine, bromine, imidazolyl group, trichloromethoxy group, and the like.

Production method of step [b]

A compound represented by the formula (1-2) can be produced by reacting a compound represented by the formula (2) with a reaction product of a compound represented by the formula (4) and a compound represented by the formula (5) in the presence of an inert solvent and a base.

Examples of the base that can be used in this reaction include hydroxides such as lithium hydroxide, sodium hydroxide, potassium hydroxide, calcium hydroxide, and the like, alkoxides such as sodium methoxide, sodium ethoxide, sodium tertiary-butoxide, potassium tertiary-butoxide, and the like, alkali metal hydrides such as sodium hydride, potassium hydride, and the like, carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like, acetates such as lithium acetate, sodium acetate, potassium acetate, and the like, organic bases such as pyridine, picoline, lutidine, triethylamine, tributylamine, N,N-diisopropylethylamine, and the like, and the like. The amount thereof to be used is generally 1-fold mol to 10-fold mol with respect to a compound represented by the formula (2) .

An inert solvent that can be used in this reaction may be any as long as it does not markedly inhibit the progress of this reaction. Examples thereof include inert solvents such as chain or cyclic saturated hydrocarbons such as pentane, hexane, cyclohexane, and the like, aromatic hydrocarbons such as benzene, toluene, xylene, and the like, halogenated aromatic hydrocarbons such as chlorobenzene, dichlorobenzene, and the like, chain or cyclic ethers such as diethyl ether, methyl tertiary-butyl ether, dioxane, tetrahydrofuran, and the like, and the like, nitriles such as acetonitrile, propionitrile, and the like, aprotic polar solvents such as N,N-dimethylformamide, N,N-dimethylacetamide, dimethyl sulfoxide, 1,3-dimethyl-2-imidazolidinone, and the like, alcohols such as methanol, ethanol, propanol, butanol, 2-propanol, and the like, and the like. These inert solvents can be used alone or a mixture of two or more kinds thereof may be used. The amount thereof to be used can be appropriately selected from the range of generally 0.1 to 100 L per 1 mol of a compound represented by the formula (2).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary.

Production method 3

A compound represented by the formula (1-2) of the present invention can be produced from a compound represented by the formula (2) by the following steps [c], [d], and [e].

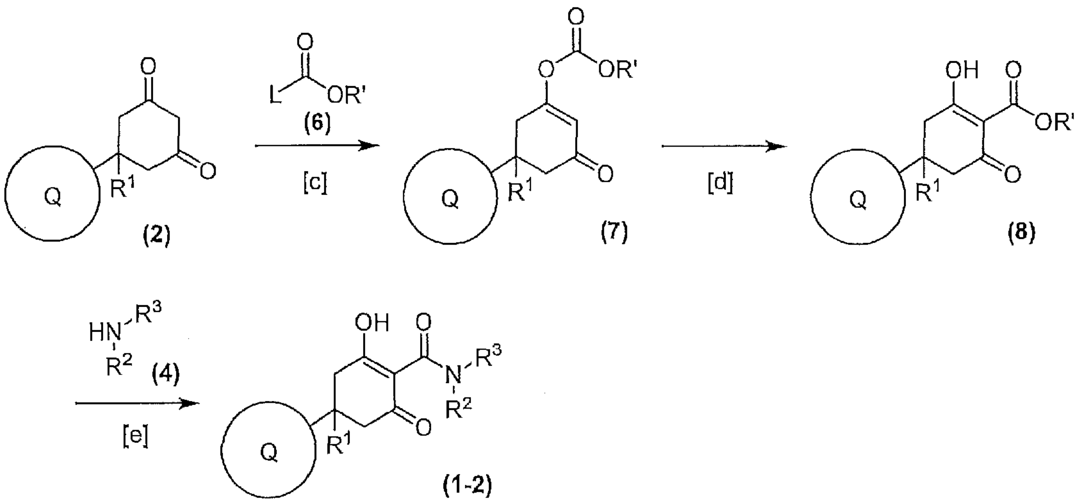


wherein $R^1$, $R^2$, $R^3$, and Q are the same as above, R' is a $(C_1-C_6)$alkyl group such as methyl group, ethyl group, and the like, and L is a leaving group such as halogen atom (e.g., chlorine, bromine, etc.), $(C_1-C_6)$alkylcarbonyloxy group (e.g., acetoxy group, etc.), and the like.

Production method of step [c]

A compound represented by the formula (7) can be produced by reacting a compound represented by the formula (2) with a compound represented by the formula (6) in the presence of a base and an inert solvent.

Examples of the base that can be used in this reaction include hydroxides such as lithium hydroxide, sodium hydroxide, potassium hydroxide, calcium hydroxide, and the like, alkoxides such as sodium methoxide, sodium ethoxide, sodium tertiary-butoxide, potassium tertiary-butoxide, and the like, alkali metal hydrides such as sodium hydride, potassium hydride, and the like, carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like, acetates such as lithium acetate, sodium acetate, potassium acetate, and the like, organic bases such as pyridine, picoline, lutidine, triethylamine, tributylamine, N,N-diisopropylethylamine, and the like, and the like. The amount thereof to be used is generally 1-fold mol to 10-fold mol with respect to a compound represented by the formula (2) .

An inert solvent that can be used in this reaction may be any as long as it does not markedly inhibit the progress of this reaction. Examples thereof include inert solvents such as chain or cyclic saturated hydrocarbons such as pentane, hexane, cyclohexane, and the like, aromatic hydrocarbons such as benzene, toluene, xylene, and the like, halogenated aromatic hydrocarbons such as chlorobenzene, dichlorobenzene, and the like, chain or cyclic ethers such as diethyl ether, methyl tertiary-butyl ether, dioxane, tetrahydrofuran, and the like, and the like, nitriles such as acetonitrile, propionitrile, and the like, aprotic polar solvents such as N,N-dimethylformamide, N,N-dimethylacetamide, dimethyl sulfoxide, 1,3-dimethyl-2-imidazolidinone, and the like, alcohols such as methanol, ethanol, propanol, butanol, 2-propanol, and the like, and the like. These inert solvents can be used alone or a mixture of two or more kinds thereof may be used. The amount thereof to be used can be appropriately selected from the range of generally 0.1 to 100 L per 1 mol of a compound represented by the formula (2).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [d]

A compound represented by the formula (8) can be produced by reacting a compound represented by the formula (7) in the presence of a base and an inert solvent.

Examples of the base that can be used in this reaction include hydroxides such as lithium hydroxide, sodium hydroxide, potassium hydroxide, calcium hydroxide, and the like, alkoxides such as sodium methoxide, sodium ethoxide, sodium tertiary-butoxide, potassium tertiary-butoxide, and the like, alkali metal hydrides such as sodium hydride, potassium hydride, and the like, carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like, acetates such as lithium acetate, sodium acetate, potassium acetate, and the like, organic bases such as pyridine, picoline, lutidine, triethylamine, tributylamine, N,N-diisopropylethylamine, N,N-dimethyl-4-aminopyridine, and the like, and the like. The amount thereof to be used is generally 1-fold mol to 10-fold mol with respect to a compound represented by the formula (7).

An inert solvent that can be used in this reaction may be any as long as it does not markedly inhibit the progress of this reaction. Examples thereof include inert solvents such as chain or cyclic saturated hydrocarbons such as pentane, hexane, cyclohexane, and the like, aromatic hydrocarbons such as benzene, toluene, xylene, and the like, halogenated aromatic hydrocarbons such as chlorobenzene, dichlorobenzene, and the like, chain or cyclic ethers such as diethyl ether, methyl tertiary-butyl ether, dioxane, tetrahydrofuran, and the like, and the like, nitriles such as acetonitrile, propionitrile, and the like, aprotic polar solvents such as N,N-dimethylformamide, N,N-dimethylacetamide, dimethyl sulfoxide, 1,3-dimethyl-2-imidazolidinone, and the like, alcohols such as methanol, ethanol, propanol, butanol, 2-propanol, and the like, and the like. These inert solvents can be used alone or a mixture of two or more kinds thereof may be used. The amount thereof to be used can be appropriately selected from the range of generally 0.1 to 100 L per 1 mol of a compound represented by the formula (7).

The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [e]

A compound represented by the formula (1-2) of the present invention can be produced by reacting a compound

represented by the formula (8) with a compound represented by the formula (4) in the presence of an inert solvent.

An inert solvent that can be used in this reaction may be any as long as it does not markedly inhibit the progress of this reaction. Examples thereof include inert solvents such as chain or cyclic saturated hydrocarbons such as pentane, hexane, cyclohexane, and the like, aromatic hydrocarbons such as benzene, toluene, xylene, and the like, halogenated hydrocarbons such as methylene chloride, chloroform, carbon tetrachloride, and the like, halogenated aromatic hydrocarbons such as chlorobenzene, dichlorobenzene, and the like, chain or cyclic ethers such as diethyl ether, methyl tertiary-butyl ether, dioxane, tetrahydrofuran, and the like, and the like, nitriles such as acetonitrile, propionitrile, and the like, esters such as methyl acetate and the like, ketones such as acetone, methyl ethyl ketone, and the like, aprotic polar solvents such as N,N-dimethylformamide, N,N-dimethylacetamide, dimethyl sulfoxide, 1,3-dimethyl-2-imidazolidinone, and the like, alcohols such as methanol, ethanol, propanol, butanol, 2-propanol, and the like, and the like. These inert solvents can be used alone or a mixture of two or more kinds thereof may be used. The amount thereof to be used can be appropriately selected from the range of generally 0.1 to 100 L per 1 mol of a compound represented by the formula (8).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary.

Production method 4

A compound represented by the formula (1-3) of the present invention can be produced from a compound represented by the formula (1-2) of the present invention by the following steps [f] and [g].

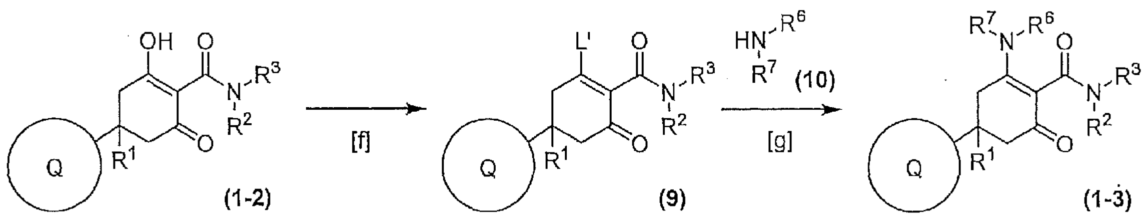


wherein $R^1$, $R^2$, $R^3$, $R^6$, $R^7$, and Q are the same as above, and L' is a halogen atom.

Production method of step [f]

A compound represented by the formula (9) can be produced by reacting a compound represented by the formula (2) with a halogenating agent in the presence of an inert solvent.

Examples of the halogenating agent that can be used in this reaction include thionyl chloride, chlorine, sulfuryl chloride, oxalyl chloride, 1,3-dichloro-5,5-dimethylhydantoin, N-chlorosuccinimide, and the like. The amount thereof to be used is generally 0.5-fold mol to 5-fold mol with respect to a compound represented by the formula (1-2).

An inert solvent that can be used in this reaction may be any as long as it does not markedly inhibit the progress of this reaction. Examples thereof include inert solvents such as halogenated hydrocarbons such as methylene chloride, chloroform, carbon tetrachloride, and the like, halogenated aromatic hydrocarbons such as chlorobenzene, dichlorobenzene, and the like, nitriles such as acetonitrile, propionitrile, and the like, organic acids such as acetic acid, propionic acid, and the like, aprotic polar solvents such as N,N-dimethylformamide, N,N-dimethylacetamide, dimethyl sulfoxide, 1,3-dimethyl-2-imidazolidinone, and the like, water, and the like. These inert solvents can be used alone or a mixture of two or more kinds thereof may be used. The amount thereof to be used can be appropriately selected from the range of generally 0.1 to 100 L per 1 mol of a compound represented by the formula (1-2).

The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [g]

A compound represented by the formula (1-3) can be produced by reacting a compound represented by the formula (9) with a compound represented by the formula (10) in the presence of an inert solvent and a base.

Examples of the base that can be used in this reaction include hydroxides such as lithium hydroxide, sodium hydroxide, potassium hydroxide, calcium hydroxide, and the like, alkoxides such as sodium methoxide, sodium ethoxide, sodium tertiary-butoxide, potassium tertiary-butoxide, and the like, alkali metal hydrides such as sodium hydride, potassium hydride, and the like, carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like, acetates such as lithium acetate, sodium acetate, potassium acetate, and the like, organic bases such as pyridine, picoline, lutidine, triethylamine, tributylamine, N,N-diisopropylethylamine, and the like, and the like. The amount thereof to be used is generally 1-fold mol to 10-fold mol with respect to a compound represented by the formula (9) .

An inert solvent that can be used in this reaction may be any as long as it does not markedly inhibit the progress of this reaction. Examples thereof include inert solvents such as chain or cyclic saturated hydrocarbons such as pentane, hexane, cyclohexane, and the like, aromatic hydrocarbons such as benzene, toluene, xylene, and the like, halogenated aromatic hydrocarbons such as chlorobenzene, dichlorobenzene, and the like, chain or cyclic ethers such as diethyl ether, methyl tertiary-butyl ether, dioxane, tetrahydrofuran, and the like, and the like, nitriles such as acetonitrile, propionitrile, and the like, aprotic polar solvents such as N,N-dimethylformamide, N,N-dimethylacetamide, dimethyl sulfoxide, 1,3-dimethyl-2-imidazolidinone, and the like, alcohols such as methanol, ethanol, propanol, butanol, 2-propanol, and the like, and the like. These inert solvents can be used alone or a mixture of two or more kinds thereof may be used. The amount thereof to be used can be appropriately selected from the range of generally 0.1 to 100 L per 1 mol of a compound represented by the formula (9).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary.

Starting material production method 1

Among the compounds represented by the formula (2) as starting materials, a compound wherein $R^1$ is a hydrogen atom can be produced by the method described in Journal of the Chemical Society, published in 1935, pp. 539-540, or according thereto.

Starting material production method 2

Among the compounds represented by the formula (2) as starting materials, a compound (2-1) wherein $R^1$ is other than a hydrogen atom can be produced by the following production method, namely, from a compound represented by the formula (11) by the following steps [h], [i], and [j].

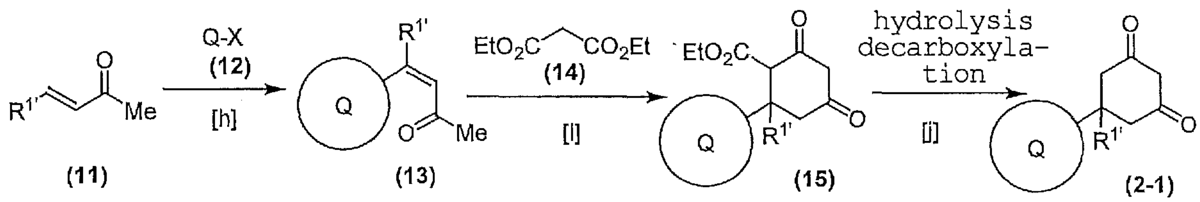


(11) (13) (15) (2-1)

wherein Q is the same as above, $R^{1'}$ is a $(C_1-C_6)$alkyl group, a halo$(C_1-C_6)$alkyl group, a phenyl group, or a substituted phenyl group having, on a ring, 1 to 5 substituents each independently selected from the group consisting of a halogen atom, a $(C_1-C_6)$alkyl group, a halo$(C_1-C_6)$alkyl group, and a $(C_1-C_6)$alkoxy group, and X is a leaving group such as chlorine, bromine, iodine, and the like.

Production method of step [h]

A compound represented by the formula (13) can be produced by reacting a compound represented by the formula (11) with a compound represented by the formula (12) in the presence of a metal catalyst, a base, and an inert solvent.

Examples of the metal catalyst that can be used in this reaction include palladium catalyst, nickel catalyst, iron catalyst, ruthenium catalyst, platinum catalyst, rhodium catalyst, iridium catalyst, and the like. As these metal catalysts, "metal", "supported metal", "metal salt such as metal chloride, bromide, iodide, nitrate, sulfate, carbonate, oxalate, acetate, oxide, and the like", "complex compound such as olefin complex, phosphine complex, amine complex, ammine complex, acetylacetonate complex, and the like" can be used. Palladium catalysts are preferred.

Examples of the palladium catalyst include palladium metals such as palladium black, palladium sponge, and the like, and supported palladium metals such as palladium/alumina, palladium/carbon, palladium/silica, palladium/Y-type zeolite, and the like. In addition, metal salts such as palladium chloride, palladium bromide, palladium iodide, palladium acetate, and the like can be mentioned. In addition, examples thereof include complex compounds such as n-allylpalladium chloride dimer, palladium acetylacetonate, dichlorobis(acetonitrile)palladium, dichlorobis(benzonitrile)palladium, bis(dibenzylideneacetone)palladium, tris(dibenzyl ideneacetone)dipalladium, tris(dibenzylideneacetone)dipalladium (chloroform adduct), dichlorodiaminepalladium, dichlorobis(triphenylphosphine)palladium, dichlorobis(tricyclohexylphosphine)palladium, tetrakis (triphenylphosphine)palladium, dichloro[1,2-bis(diphenylphosphino)ethane]palladium, dichloro[1,3-bis(diphenylphosphino)propane]palladium, dichloro[1,4-bis(diphenylphosphino)butane]palladium, dichloro[1,1'-bis(diphenylphosphino)ferrocene]palladium, diphenylphosphino ferrocenedichloropalladium-dichloromethane complex, and the like. The amount of the metal catalyst to be used can be appropriately selected from the range of 0.001-fold mol to 0.5-fold mol with respect to a compound represented by the formula (11).

These palladium catalysts can be used alone or in combination with tertiary phosphine. Examples of the tertiary phosphine that can be used include triphenylphosphine, trimethylphosphine, triethylphosphine, tributylphosphine, tri(tert-butyl)phosphine, tricyclohexylphosphine, tri-o-tolylphosphine, trioctylphosphine, 9,9-dimethyl-4,5-bis(diphenylphosphino)xanthene, 2-(di-tert-butylphosphino)biphenyl, 2-(dicyclohexylphosphino)biphenyl, 1,2-bis(diphenylphosphino)ethane, 1,3-bis(diphenylphosphino)propane, 1,4-bis(diphenylphosphino)butane, 1,1'-bis(diphenylphosphino)ferrocene, (R)-(+)-2,2'-bis(diphenylphosphino)-1,1'-binaphthyl, (S)-(-)-2,2'-bis(diphenylphosphino)-1,1'-binaphthyl, (±)-2,2'-bis(diphenylphosphino)-1,1'-binaphthyl, and the like. The amount of the tertiary phosphine to be used can be appropriately selected from the range of 0.5-fold mol to 10-fold mol with respect to the metal catalyst.

Examples of the base that can be used in this reaction include inorganic bases such as sodium hydroxide, potassium hydroxide, sodium carbonate, potassium carbonate, cesium carbonate, sodium hydrogen carbonate, potassium hydrogen carbonate, and the like, metal acetates such as sodium acetate, potassium acetate, silver acetate, and the like, alkali metal hydrides such as sodium hydride, potassium hydride, and the like, alkoxides such as sodium methoxide, sodium ethoxide, potassium tertiary-butoxide, and the like. The amount thereof to be used is generally within the range of about 1-fold mol to 5-fold mol with respect to a compound represented by the formula (11).

An inert solvent that can be used in this reaction may be any as long as it does not markedly inhibit the progress of this reaction, and examples thereof include alcohols such as methanol, ethanol, propanol, butanol, 2-propanol, and the like, chain or cyclic ethers such as diethyl ether, tetrahydrofuran, dioxane, 1,2-dimethoxyethane (DME), and the like, aromatic hydrocarbons such as benzene, toluene, xylene, and the like, halogenated hydrocarbons such as methylene chloride, chloroform, carbon tetrachloride, and the like, halogenated aromatic hydrocarbons such as chlorobenzene, dichlorobenzene, and the like, nitriles such as acetonitrile and the like, esters such as ethyl acetate and the like, polar solvents such as N,N-dimethylformamide, N,N-dimethylacetamide, dimethyl sulfoxide, 1,3-dimethyl-2-imidazolidinone, and the like, organic acids acetic acid, propionic acid, and the like, and water. These inert solvents can be used alone or a mixture of two or more kinds thereof may be used. The amount of the inert solvent to be used is not particularly limited as long as it is an amount that dissolves the reaction reagent, and can be appropriately selected from the range of 0.5 L to 100 L per 1 mol of a compound represented by the formula (11).

Since this reaction is an equimolar reaction, each compound may be used in an equimolar amount, but it is also possible to use either compound in excess.

The reaction temperature in this reaction may be generally from about 0°C to the boiling point of the solvent to be used. While the reaction time varies depending on the reaction scale, reaction temperature, and the like, it can be appropriately selected from the range of several minutes to 48 hours. This reaction can also be performed under an inert gas atmosphere of, for example, nitrogen gas or argon gas.

After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [i]

A compound represented by the formula (15) can be produced by reacting a compound represented by the formula (13) with a compound represented by the formula (14) in the presence of a base and an inert solvent.

Examples of the base that can be used in this reaction include inorganic bases such as hydroxides of alkali metal atom such as sodium hydroxide, potassium hydroxide, and the like, hydrides of alkali metal such as sodium hydride, potassium hydride, and the like, alkali metal salts of alcohol such as sodium ethoxide, potassium t-butoxide, and the like, carbonates such as sodium carbonate, potassium carbonate, sodium hydrogen carbonate, and the like, and the like, organic bases such as triethylamine, pyridine, DBU, and the like, and the like. The amount thereof to be used is generally 1-fold mol to 10-fold mol with respect to a compound represented by the formula (13).

An inert solvent that can be used in this reaction may be any as long as it does not markedly inhibit the progress of this reaction. Examples thereof include inert solvents such as chain or cyclic saturated hydrocarbons such as pentane, hexane, cyclohexane, and the like, aromatic hydrocarbons such as benzene, toluene, xylene, and the like, halogenated hydrocarbons such as methylene chloride, chloroform, carbon tetrachloride, and the like, halogenated aromatic hydrocarbons such as chlorobenzene, dichlorobenzene, and the like, chain or cyclic ethers such as diethyl ether, methyl tertiary-butyl ether, dioxane, tetrahydrofuran, and the like, and the like, nitriles such as acetonitrile, propionitrile, and the like, esters such as methyl acetate and the like, ketones such as acetone, methyl ethyl ketone, and the like, aprotic polar solvents such as N,N-dimethylformamide, N,N-dimethylacetamide, dimethyl sulfoxide, 1,3-dimethyl-2-imidazolidinone, and the like, alcohols such as methanol, ethanol, propanol, butanol, 2-propanol, and the like, and the like. These inert solvents can be used alone or a mixture of two or more kinds thereof may be used. The amount thereof to be used can be appropriately selected from the range of generally 0.1 to 100 L per 1 mol of a compound represented by the formula (13).

Since this reaction is an equimolar reaction, each compound may be used in an equimolar amount, but it is also possible to use either compound in excess. The reaction temperature in this reaction may be generally 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [j]

A compound represented by the formula (2-1) can be produced by hydrolyzing a compound represented by the formula (15) in the presence or a base and an inert solvent, and decarboxylating the compound under acidic conditions.

Examples of the base that can be used in this reaction include inorganic bases such as hydroxides of alkali metal atom such as sodium hydroxide, potassium hydroxide, and the like, hydrides of alkali metal such as sodium hydride, potassium hydride, and the like, alkali metal salts of alcohol such as sodium ethoxide, potassium t-butoxide, and the like, carbonates such as sodium carbonate, potassium carbonate, sodium hydrogen carbonate, and the like, and the like, organic bases such as triethylamine, pyridine, DBU, and the like, and the like. The amount thereof to be used is generally 1-fold mol to 10-fold mol with respect to a compound represented by the formula (15).

Examples of the acid that can be used in the decarboxylation of this reaction include hard acids such as hydrochloric acid, sulfuric acid, and the like, organic acids such as formic acid, acetic acid, trifluoroacetic acid, and the like, sulfonic acids such as methanesulfonic acid, trifluoromethanesulfonic acid, p-toluenesulfonic acid, and the like, and the like. The amount thereof to be used may be any amount that renders the reaction mixture acidic:

An inert solvent that can be used in this reaction may be any as long as it does not markedly inhibit the progress of this reaction. Examples thereof include nitriles such as acetonitrile, propionitrile, and the like, aprotic polar solvents such as N,N-dimethylformamide, N,N-dimethylacetamide, dimethyl sulfoxide, 1,3-dimethyl-2-imidazolidinone, and the like, alcohols such as methanol, ethanol, propanol, butanol, 2-propanol, and the like, and the like. These inert solvents can be used alone or a mixture of two or more kinds thereof may be used. The amount thereof to be used can be appropriately selected from the range of generally 0.1 to 100 L per 1 mol of a compound represented by the formula (15) .

The reaction temperature in this reaction may be generally from 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Specific examples of the compound of the present invention are shown in the following. In the following Tables, Me is a methyl group, Et is an ethyl group, i-Pr is an isopropyl group, c-Pr is a cyclopropyl group, n-Bu is a normal butyl group, i-Bu is an isobutyl group, s-Bu is a secondary butyl group, n-Hex is a normal hexyl group, c-Hex is a cyclohexyl

group, Ph is a phenyl group, Bn is a benzyl group, and Ac group is an acetyl group. In the structural formulas, • shows a bonding position. Physical property shows melting point (°C) or $H^1$-NMR. $H^1$-NMR data are shown in the second table.

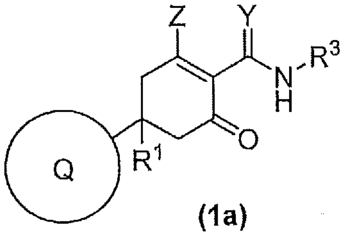

(1a)

[Table 1]

| the first table | | | | | | |
|---|---|---|---|---|---|---|
| compound No. | $R^1$ | Q | R3 | Y | Z | physical property value |
| 1-1 | H | 2-Me-Ph | 4-F-Ph | O | OH | 141-143 |
| 1-2 | H | 3,5-$CF_3$-Ph | 4-F-Ph | O | OH | 174-175 |
| 1-3 | H | 3-Cl-4-$CF_3$-Ph | 4-F-Ph | O | OH | 162-163 |
| 1-4 | H | 3-Cl-4-$CF_3$-Ph | 4-$CF_3$-Ph | O | OH | 166-167 |
| 1-5 | H | 3-OPh-Ph | 4-F-Ph | O | OH | 123-125 |
| 1-6 | H | 4-$CF_3$-Ph | 4-F-Ph | O | OH | 138-139 |
| 1-7 | H | 4-$CF_3$-Ph | 4-CN-Ph | O | OH | 196-201 |
| 1-8 | H | 4-$CF_3$-Ph | 4-OMe-Ph | O | OH | 201-202 |
| 1-9 | H | 4-$CF_3$-Ph | 4-O$CF_3$-Ph | O | OH | 138-139 |
| 1-10 | H | 4-$CF_3$-Ph | Et | O | OH | NMR |
| 1-11 | H | 4-$CF_3$-Ph | c-Hex | O | OH | NMR |
| 1-12 | H | 4-$CF_3$-Ph | Bn | O | OH | NMR |
| 1-13 | H | 4-$CF_3$-Ph | 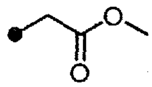 | O | OH | NMR |
| 1-14 | H | 4-$CF_3$-Ph | $CO_2Et$ | O | OH | 146-148 |
| 1-15 | H | 4-$CF_3$-Ph | 4-Me-Ph | O | OH | 224-226 |
| 1-16 | H | 4-$CF_3$-Ph | 2-Me-4-Cl--Ph | O | OH | 180-181 |
| 1-17 | H | 4-$CF_3$-Ph | 2,4-$F_2$-Ph | O | OH | 172-174 |
| 1-18 | H | 4-$CF_3$-Ph | 2,6-$F_2$-Ph | O | OH | 176-178 |
| 1-19 | H | 4-$CF_3$-Ph | 3,4-$F_2$-Ph | O | OH | 162-164 |
| 1-20 | H | 4-$CF_3$-Ph | 2-Cl-Ph | O | OH | 162-164 |
| 1-21 | H | 4-$CF_3$-Ph | 3-Cl-Ph | O | OH | 136-137 |
| 1-22 | H | 4-$CF_3$-Ph | 2,4-$Cl_2$-Ph | O | OH | 197-198 |
| 1-23 | H | 4-$CF_3$-Ph | 2,6-$Cl_2$-Ph | O | OH | 172-174 |
| 1-24 | H | 4-$CF_3$-Ph | 3,4-$Cl_2$-Ph | O | OH | 190-191 |

(continued)

| compound No. | $R^1$ | Q | R3 | Y | Z | physical property value |
|---|---|---|---|---|---|---|
| 1-25 | H | $4-CF_3-Ph$ | $3,5-Cl_2-Ph$ | O | OH | 207-209 |
| 1-26 | H | $4-CF_3-Ph$ | $2,4,6-Cl_3-Ph$ | O | OH | 168-170 |
| 1-27 | H | $4-CF_3-Ph$ | $2-Cl-5-CF_3-Ph$ | O | OH | 172-174 |
| 1-28 | H | $4-CF_3-Ph$ | 4-Br-Ph | O | OH | 179-181 |
| 1-29 | H | $4-CF_3-Ph$ | $2-CF_3-Ph$ | O | OH | 128-129 |
| 1-30 | H | $4-CF_3-Ph$ | $3-CF_3-Ph$ | O | OH | 122-123 |
| 1-31 | H | $4-CF_3-Ph$ | $4-CF_3-Ph$ | O | OH | 168-169 |
| 1-32 | H | $4-CF_3-Ph$ | $3,5-(CF_3)_2-Ph$ | O | OH | NMR |
| 1-33 | H | $4-CF_3-Ph$ | $4-SCF_3-Ph$ | O | OH | 127-129 |
| 1-34 | H | $4-CF_3-Ph$ | 3-CN-Ph | O | OH | 178-180 |
| 1-35 | H | $4-CF_3-Ph$ | $3-NO_2-Ph$ | O | OH | 200-201 |

[Table 2]

| the first table (continued) | | | | | | |
|---|---|---|---|---|---|---|
| compound No. | $R^1$ | Q | R3 | Y | Z | physical property value |
| 1-36 | H | $4-CF_3-Ph$ | 4-Ac-Ph | O | OH | 207-208 |
| 1-37 | H | $4-CF_3-Ph$ | COPh | O | OH | 176-177 |
| 1-38 | H | $4-CF_3-Ph$ | 2-F-Ph | O | OH | 233-236 |
| 1-39 | H | $4-CF_3-Ph$ | 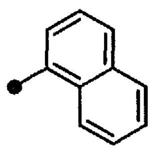 | O | OH | 201-203 |
| 1-40 | H | $4-CF_3-Ph$ | 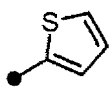 | O | OH | 208-210 |
| 1-41 | H | $4-CF_3-Ph$ | 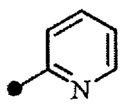 | O | OH | 188-190 |
| 1-42 | H | $4-CF_3-Ph$ | 2-Ph-Ph | O | OH | 134-136 |
| 1-43 | H | $4-CF_3-Ph$ | 3-F-Ph | O | OH | 164-166 |
| 1-44 | H | $4-CF_3-Ph$ | 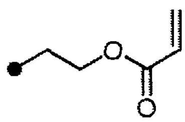 | O | OH . | 134-136 |
| 1-45 | H | $4-CF_3-Ph$ | $2, 3, 4, 5, 6-F_5-Ph$ | O | OH | 162-164 |
| 1-46 | H | $4-CF_3-Ph$ | 4-Ph-Ph | O | OH | 181-183 |

(continued)

| compound No. | $R^1$ | Q | R3 | Y | Z | physical property value |
|---|---|---|---|---|---|---|
| 1-47 | H | 4-$CF_3$-Ph | 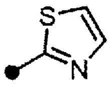 | O | OH | 249-250 |
| 1-48 | H | 4-$CF_3$-Ph | 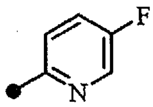 | O | OH | 188-190 |
| 1-49 | H | 4-$CF_3$-Ph | 4-SMe-Ph | O | OH | 197-198 |
| 1-50 | H | 4-$CF_3$-Ph | 4-SOMe-Ph | O | OH | 261-262 |
| 1-51 | H | 4-$CF_3$-Ph | 4-$SO_2$Me-Ph | O | OH | |
| 1-52 | H | 4-$CF_3$-Ph | 4-$SOCF_3$-Ph | O | OH | 199-200 |
| 1-53 | H | 4-$CF_3$-Ph | 4-$SO_2CF_3$-Ph | O | OH | 166-167 |
| 1-54 | H | 4-$CF_3$-Ph | 4-OPh-Ph | O | OH | 171-172 |
| 1-55 | H | 4-$CF_3$-Ph | 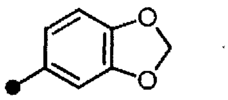 | O | OH | 193-194 |
| 1-56 | H | 4-$CF_3$-Ph | 4-F-Ph | O | ONa | |
| 1-57 | H | 4-$CF_3$-Ph | 4-F-Ph | O | SMe | |
| 1-58 | H | 4-$CF_3$-Ph | 4-F-Ph | O | S(O)Me | |
| 1-59 | H | 4-$CF_3$-Ph | 4-F-Ph | O | $S(O)_2Me$ | |
| 1-60 | H | 4-$CF_3$-Ph | 4-F-Ph | O | OMe | |

[Table 3]

| the first table (continued) | | | | | | |
|---|---|---|---|---|---|---|
| compound No. | $R^1$ | Q | $R^3$ | Y | Z | physical property value |
| 1-61 | H | 4-CN-Ph | 4-F-Ph | O | OH | 204-205 |
| 1-62 | H | 4-$CONH_2$-Ph | 4-F-Ph | O | OH | NMR |
| 1-63 | H | 4-Me-Ph | 4-F-Ph | O | OH | NMR |
| 1-64 | H | 4-(morpholin-4-yl)-Ph | 4-F-Ph | O | OH | 236-238 |
| 1-65 | H | 4-$OCF_3$-Ph | 4-F-Ph | O | OH | 137-138 |
| 1-66 | H | 4-$SCF_3$-Ph | 4-F-Ph | O | OH | 149-150 |
| 1-67 | H | ferrocen-1-yl | 4-F-Ph | O | OH | 161-163 |
| 1-68 | H | 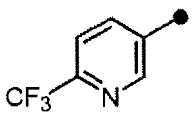 | 4-F-Ph | O | OH | 189-190 |
| 1-69 | H | 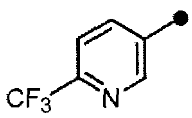 | 4-$CF_3$-Ph | O | OH | 199-200 |

(continued)

| compound No. | $R^1$ | Q | $R^3$ | Y | Z | physical property value |
|---|---|---|---|---|---|---|
| 1-70 | H | 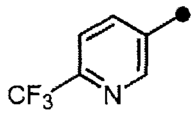 | 4-$OCF_3$-Ph | O | OH | 193-194 |
| 1-71 | H | 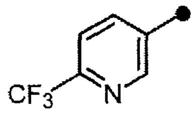 | 4-Cl-Ph | O | OH | 209-210 |
| 1-72 | H | 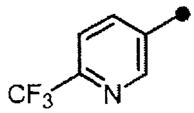 | 4-OMe-Ph | O | OH | 215-216 |
| 1-73 | H | 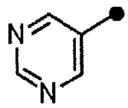 | 4-F-Ph | O | OH | 215-216 |
| 1-74 | H | 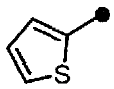 | 4-F-Ph | O | OH | 143-145 |
| 1-75 | H | 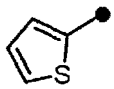 | 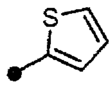 | O | OH | 158-159 |
| 1-76 | H | 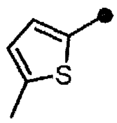 | 4-F-Ph | O | OH | 138-139 |
| 1-77 | H | 3-Cl-4-$CF_3$-Ph | 4-$CF_3$-Ph | S | OH | 60-61 |
| 1-78 | H | 3-Cl-4-$CF_3$-Ph | 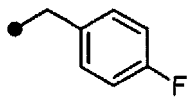 | S | OH | 125 |
| 1-79 | H | 4- (4-F-Ph) -Ph | 4-F-Ph | S | OH | |
| 1-80 | H | 4- (pyrazol-1-yl) -Ph | 4-F-Ph | S | OH | |

[Table 4]

| the first table (continued) | | | | | | |
|---|---|---|---|---|---|---|
| compound No. | $R^1$ | Q | $R^3$ | Y | Z | physical property value |
| 1-81 | H | 4-$CF_3$-Ph | 4-F-Ph | S | OH | 164-165 |
| 1-82 | H | 4-$CF_3$-Ph | Et | S | OH | 141-142 |
| 1-83 | H | 4-$CF_3$-Ph | n-Pr | S | OH | 143-144 |
| 1-84 | H | 4-$CF_3$-Ph | i-Pr | S | OH | 147-149 |
| 1-85 | H | 4-$CF_3$-Ph | n-Bu | S | OH | 98-99 |
| 1-86 | H | 4-$CF_3$-Ph | i-Bu | S | OH | 102-103 |

(continued)

| compound No. | $R^1$ | Q | $R^3$ | Y | Z | physical property value |
|---|---|---|---|---|---|---|
| 1-87 | H | $4-CF_3-Ph$ | s-Bu | S | OH | 104-105 |
| 1-88 | H | $4-CF_3-Ph$ | n-Hex | S | OH | 87-88 |
| 1-89 | H | $4-CF_3-Ph$ | c-Hex | S | OH | 152-153 |
| 1-90 | H | $4-CF_3-Ph$ | 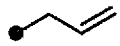 | S | OH | NMR |
| 1-91 | H | $4-CF_3-Ph$ | Bn | S | OH | 114-115 |
| 1-92 | H | $4-CF_3-Ph$ | 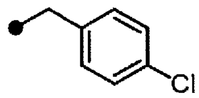 | S | OH | 165-166 |
| 1-93 | H | $4-CF_3-Ph$ | Ph | S | OH | 169-170 |
| 1-94 | H | $4-CF_3-Ph$ | 2-F-Ph | S | OH | NMR |
| 1-95 | H | $4-CF_3-Ph$ | 3-F-Ph | S | OH | 156-158 |
| 1-96 | . H | $4-CF_3-Ph$ | 2-Cl-Ph | S | OH | 142-143 |
| 1-97 | H | $4-CF_3-Ph$ | 4-Cl-Ph | S | OH | 194-196 |
| 1-98 | H | $4-CF_3-Ph$ | $2,4-Cl_2-Ph$ | S | OH | NMR |
| 1-99 | H | $4-CF_3-Ph$ | $3-CF_3-Ph$ | S | OH | 141-142 |
| 1-100 | H | $4-CF_3-Ph$ | 4-CN-Ph | S | OH | 182-185 |
| 1-101 | H | $4-CF_3-Ph$ | 2-Me-Ph | S | OH | 151-152 |
| 1-102 | H | $4-CF_3-Ph$ | 4-Me-Ph | S | OH | 190-192 |
| 1-103 | H | $4-CF_3-Ph$ | $2,6-Me_2-Ph$ | S | OH | 176-178 |
| 1-104 | H | $4-CF_3-Ph$ | $2,6-Et_2-Ph$ | S | OH | 168-169 |
| 1-105 | H | $4-CF_3-Ph$ | 2-OMe-Ph | S | OH | 185-187 |
| 1-106 | H | $4-CF_3-Ph$ | 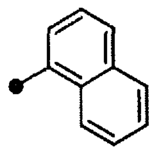 | S | OH | NMR |
| 1-107 | H | $4-CF_3-Ph$ | 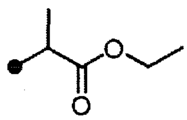 | S | OH | NMR |
| 1-108 | H | $4-CF_3-Ph$ | 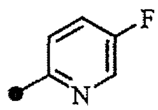 | S | OH | 56-57 |
| 1-109 | H | $4-CF_3-Ph$ | 2-Me-4-c-Pr-Ph | S | OH | |

[Table 5]

| the first table (continued) |
|---|

(continued)

| compound No. | $R^1$ | Q | $R^3$ | Y | Z | physical property value |
|---|---|---|---|---|---|---|
| 1-110 | H | 4-$CF_3$-Ph | 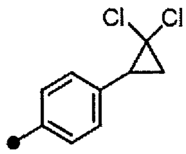 | S | OH | |
| 1-111 | H | 4-$CF_3$-Ph | 4-NH(COMe)-Ph | S | OH | |
| 1-112 | H | 4-$CF_3$-Ph | 4-NH($COCF_3$)-Ph | S | OH | |
| 1-113 | H | 4-$CF_3$-Ph | 4-NH($SO_2$Me)-Ph | S | OH | |
| 1-114 | H | 4-$CF_3$-Ph | 4-NH($SO_2CF_3$)-Ph | S | OH | |
| 1-115 | H | 4-$CF_3$-Ph | 4-$CO_2$Et-Ph | S | OH | |
| 1-116 | H | 4-$CF_3$-Ph | 4-$CONMe_2$-Ph | S | OH | |
| 1-117 | H | 4-$CF_3$-Ph | 4-$CONHCH_2CF_3$-Ph | S | OH | |
| 1-118 | H | 4-$CF_3$-Ph | 4-(4-F-Ph)-Ph | S | OH | |
| 1-119 | H | 4-$CF_3$-Ph | 4-(pyrazol-1-yl)-Ph | S | OH | |
| 1-120 | H | 4-$CO_2$Et-Ph | 4-F-Ph | S | OH | |
| 1-121 | H | 4-$CONHCH_2CF_3$-Ph | 4-F-Ph | S | OH | |
| 1-122 | H | 4-$CONMe_2$-Ph | 4-F-Ph | S | OH | |
| 1-123 | H | 4-c-Pr-Ph | 4-F-Ph | S | OH | |
| 1-124 | H | 4-(morpholin-4-yl)-Ph | 4-F-Ph | S | OH | 210-211 |
| 1-125 | H | 4-$NHCOCF_3$-Ph | 4-F-Ph | S | OH | |
| 1-126 | H | 4-NHCOMe-Ph | 4-F-Ph | S | OH | |
| 1-127 | H | 4-$NHSO_2CF_3$-Ph | 4-F-Ph | S | OH | |
| 1-128 | H | 4-$NHSO_2$Me-Ph | 4-F-Ph | S | OH | |
| 1-129 | H | 4-$NMe_2$-Ph | 4-F-Ph | S | OH | NMR |
| 1-130 | H | 4-$NO_2$-Ph | 4-F-Ph | S | OH | |
| 1-131 | H | 4-$OCF_3$-Ph | 4-F-Ph | S | OH | |
| 1-132 | H | 4-SMe-Ph | 4-F-Ph | S | OH | |
| 1-133 | H | 4-$SO_2CF_3$-Ph | 4-F-Ph | S | OH | |
| 1-134 | H | 4-$SO_2$Me-Ph | 4-F-Ph | S | OH | |
| 1-135 | H | 4-$SOCF_3$-Ph | 4-F-Ph | S | OH | |
| 1-136 | H | 4-SOMe-Ph | 4-F-Ph | S | OH | |
| 1-137 | H | 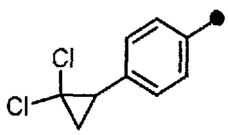 | 4-F-Ph | S | OH | |

(continued)

| compound No. | $R^1$ | Q | $R^3$ | Y | Z | physical property value |
|---|---|---|---|---|---|---|
| 1-138 | H | 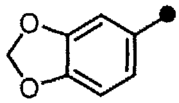 | 4-F-Ph | S | OH | |
| 1-139 | Me | 4-$CF_3$-Ph | 4-F-Ph | O | OH | 126-127 |
| 1-140 | Me | 4-$CF_3$-Ph | 4-F-Ph | S | OH | 96-98 |
| 1-141 | Ph | 4-$CF_3$-Ph | 4-F-Ph | O | OH | 65-67 |

[Table 6]

| the first table (continued) | | | | | | |
|---|---|---|---|---|---|---|
| compound No. | $R^1$ | Q | R3 | Y | Z | physical property value |
| 1-142 | H | 4-$CF_3$-Ph | 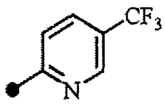 O | | OH | 162-166 |
| 1-143 | H | 4-$CF_3$-Ph | $CH_2CH_2OCH_3$ | O | OH | 59-61 |
| 1-144 | H | 4-$CF_3$-Ph | c-Pr | O | OH | 145-147 |
| 1-145 | H | 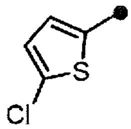 | 4-F-Ph | O | OH | 161-163 |
| 1-146 | H | 4-$CF_3$-Ph | 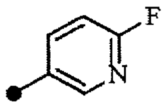 | O | OH | 156-158 |
| 1-147 | H | 4-$CF_3$-Ph | 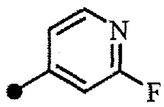 | O | OH | 186-188 |
| 1-148 | H | 4-$CH_3$-Ph | 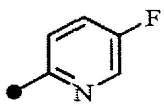 | O | OH | 182-184 |
| 1-149 | H | 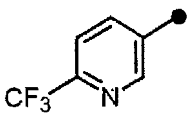 | 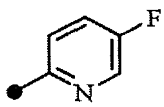 | O | OH | 195-197 |
| 1-150 | H | 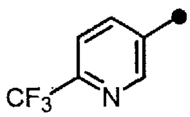 | 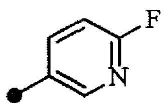 | O | OH | 190-192 |
| 1-151 | H | 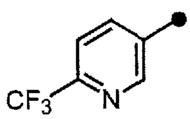 | 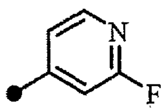 | O | OH | 208-211 |

(continued)

| compound No. | R1 | Q | R3 | Y | Z | physical property value |
|---|---|---|---|---|---|---|
| 1-152 | H | 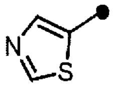 | 4-F-Ph | O | OH | 186-188 |
| 1-153 | H | 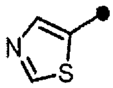 | 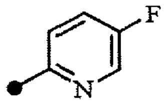 | O | OH | 273-275 |
| 1-154 | H | 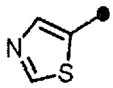 | 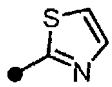 | O | OH | 275-277 |

[Table 7]

| the first table (continued) | | | | | | |
|---|---|---|---|---|---|---|
| compound No. | $R^1$ | Q | $R^3$ | Y | Z | physical property value |
| 1-155 | H | 4-$CF_3$-Ph | 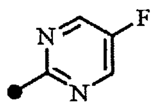 | O | OH | 198-199 |
| 1-156 | H | 4-$CF_3$-Ph | 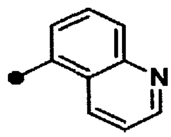 | O | OH | 211-212 |
| 1-157 | H | 4-$CF_3$-Ph | 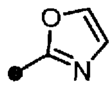 | O | OH | 209-210 |
| 1-158 | H | 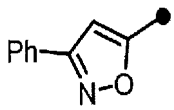 | 4-F-Ph | O | OH | 195-197 |
| 1-159 | H | 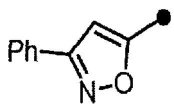 | 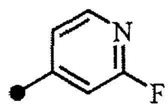 | O | OH | 246-247 |
| 1-160 | H | 4-$CF_3$-Ph | 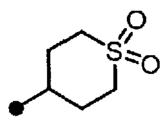 | O | OH | 190-192 |
| 1-161 | H | 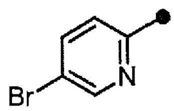 | 4-F-Ph | O | OH | NMR |
| 1-162 | H | 4-$CF_3$-Ph | 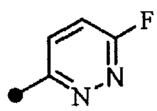 | O | OH | 201-202 |

(continued)

| compound No. | $R^1$ | Q | $R^3$ | Y | Z | physical property value |
|---|---|---|---|---|---|---|
| 1-163 | H | 4-SMe-Ph | 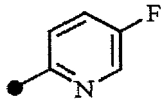 | O | OH | 177-178 |
| 1-164 | H | 4-SOMe-Ph | 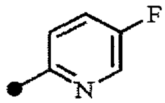 | O | OH | 215-216 |
| 1-165 | H | 4-$SO_2$Me-Ph | 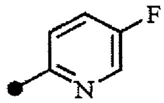 | O | OH | 231-232 |
| 1-166 | H | 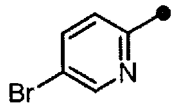 | 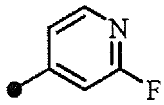 | O | OH | 285-289 |

[Table 8]

| the first table (continued) | | | | | | |
|---|---|---|---|---|---|---|
| compound No. | $R^1$ | Q | $R^3$ | Y | Z | physical property value |
| 1-167 | H | 4-$CF_3$-Ph | 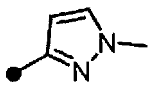 | O | OH | 201-202 |
| 1-168 | H | 4-$CF_3$-Ph | 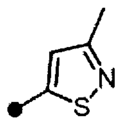 | O | OH | 300 |
| 1-169 | H | 4-$CF_3$-Ph | 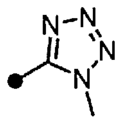 | O | OH | 155-156 |
| 1-170 | H | 4-SMe-Ph | 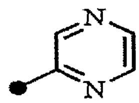 | O | OH | 195-196 |
| 1-171 | H | 4-$CF_3$-Ph | 4-c-Hex-Ph | O | OH | 217-220 |
| 1-172 | H | 4-$CF_3$-Ph | 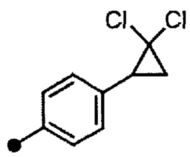 | O | OH | |
| 1-173 | H | 4-$CF_3$-Ph | 4-NH(COMe)-Ph | O | OH | 293-294 |
| 1-174 | H | 4-$CF_3$-Ph | 4-NH($COCF_3$)-Ph | O | OH | |
| 1-175 | H | 4-$CF_3$-Ph | 4-NH($SO_2$Me)-Ph | O | OH | |
| 1-176 | H | 4-$CF_3$-Ph | 4-NH($SO_2CF_3$)-Ph | O | OH | |
| 1-177 | H | 4-$CF_3$-Ph | 4-$CO_2$Me-Ph | O | OH | 230-231 |

(continued)

| compound No. | $R^1$ | Q | $R^3$ | Y | Z | physical property value |
|---|---|---|---|---|---|---|
| 1-178 | H | $4-CF_3-Ph$ | $4-CO_2H-Ph$ | O | OH | 291-292 |
| 1-179 | H | $4-CF_3-Ph$ | $4-CONMe_2-Ph$ | O | OH | |
| 1-180 | H | $4-CF_3-Ph$ | 4-CONH(n-Bu)-Ph | O | OH | |
| 1-181 | H | $4-CF_3-Ph$ | $4-CONHCH_2CF_3-Ph$ | O | OH | |
| 1-182 | H | $4-CF_3-Ph$ | $3-Cl-4-CONHCH_2CF_3-Ph$ | O | OH | |
| 1-183 | H | $4-CF_3-Ph$ | 4-(pyrazol-1-yl)-Ph | O | OH | |
| 1-184 | H | $4-CF_3-Ph$ | $4-SF_5-Ph$ | O | OH | 189-190 |
| 1-185 | H | 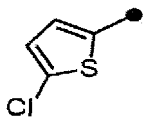 | $4-SO_2Me-Ph$ | O | OH | 232-235 |

[Table 9]

| the first table (continued) | | | | | | |
|---|---|---|---|---|---|---|
| compound No. | $R^1$ | Q | $R^3$ | Y | Z | physical property value |
| 1-186 | H | 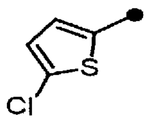 | $4-NH(SO_2Me)-Ph$ | O | OH | 256-259 |
| 1-187 | H | 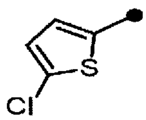 | $4-CH_2OMe-Ph$ | O | OH | 183-185 |
| 1-188 | H | 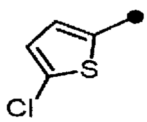 | $3-Cl-4-CONHCH_2CF_3-Ph$ | O | OH | 249-252 |
| 1-189 | H | $4-CF_3-Ph$ | $4-SO_2NHMe-Ph$ | O | OH | 254-257 |
| 1-190 | H | $4-CF_3-Ph$ | 4-S(=N-CN)Me-Ph | O | OH | 137-138 |
| 1-191 | H | $4-CF_3-Ph$ | 4-SO(=N-CN)Me-Ph | O | OH | |
| 1-192 | H | $4-CF_3-Ph$ | $4-S(=N-CN)CF_3-Ph$ | O | OH | |
| 1-193 | H | $4-CF_3-Ph$ | $4-SO(=N-CN)CF_3-Ph$ | O | OH | |
| 1-194 | H | 4-Ph-Ph | 4-F-Ph | O | OH | |
| 1-195 | H | 4- (pyrazol-1-yl)-Ph | 4-F-Ph | O | OH . | |
| 1-196 | H | $4-CO_2Et-Ph$ | 4-F-Ph | O | OH | 163-164 |
| 1-197 | H | $4-CO_2Me-Ph$ | 4-F-Ph | O | OH | 172-173 |
| 1-198 | H | $4-CO_2H-Ph$ | 4-F-Ph | O | OH | 267-268 |
| 1-199 | H | $4-CONHCH_2CF_3-Ph$ | 4-F-Ph | O | OH | |
| 1-200 | H | 4-CONH(n-Bu)-Ph | 4-F-Ph | O | OH | 274-275 |

(continued)

| compound No. | $R^1$ | Q | $R^3$ | Y | Z | physical property value |
|---|---|---|---|---|---|---|
| 1-201 | H | 4-CONMe$_2$-Ph | 4-F-Ph | O | OH | |
| 1-202 | H | 4-c-Pr-Ph | 4-F-Ph | O | OH | |
| 1-203 | H | 4-NHCOCF$_3$-Ph | 4-F-Ph | O | OH | |
| 1-204 | H | 4-NHCOMe-Ph | 4-F-Ph | O | OH | |
| 1-205 | H | 4-NHSO$_2$CF$_3$-Ph | 4-F-Ph | O | OH | |
| 1-206 | H | 4-NHSO$_2$Me-Ph | 4-F-Ph | O | OH | |
| 1-207 | H | 4-NO$_2$-Ph | 4-F-Ph | O | OH | 218-219 |
| 1-208 | H | 4-SMe-Ph | 4-F-Ph | O | OH | 145-146 |
| 1-209 | H | 4-SO$_2$CF$_3$-Ph | 4-F-Ph | O | OH | 191-192 |
| 1-210 | H | 4-SO$_2$Me-Ph | 4-F-Ph | O | OH | 192-193 |

[Table 10]

| the first table (continued) | | | | | | |
|---|---|---|---|---|---|---|
| compound No. | $R^1$ | Q | $R^3$ | Y | Z | physical property value |
| 1-211 | H | 4-SOCF$_3$-Ph | 4-F-Ph | O | OH | 164-165 |
| 1-212 | H | 4-SOMe-Ph | 4-F-Ph | O | OH | 187-188 |
| 1-213 | H | 4-SCF$_3$-Ph | 4-F-Ph | O | OH | 167-168 |
| 1-214 | H | 4-(4-t-BuPhCH$_2$S)-Ph | 4-F-Ph | O | OH | 145-146 |
| 1-215 | H | 4-SO$_2$NHMe-Ph | 4-F-Ph | O | OH | |
| 1-216 | H | 4-S(=N-CN)Me-Ph | 4-F-Ph | O | OH | 179-180 |
| 1-217 | H | 4-SO(=N-CN)Me-Ph | 4-F-Ph | O | OH | |
| 1-218 | H | 4-S(=N-CN)CF$_3$-Ph | 4-F-Ph | O | OH | |
| 1-219 | H | 4-SO(=N-CN)CF$_3$-Ph | 4-F-Ph | O | OH | |
| 1-220 | H | 4-SF$_5$-Ph | 4-F-Ph | O | OH | |
| 1-221 | H | 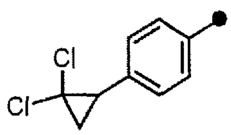 | 4-F-Ph | O | OH | |
| 1-222 | H | 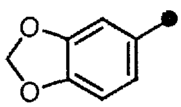 | 4-F-Ph | O | OH | 203-204 |
| 1-223 | H | 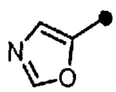 | 4-F-Ph | O | OH | 159-162 |
| 1-224 | H | 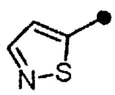 | 4-F-Ph | O | OH | 167-169 |

(continued)

| the first table (continued) | | | | | | |
|---|---|---|---|---|---|---|
| compound No. | $R^1$ | Q | $R^3$ | Y | Z | physical property value |
| 1-225 | H | $4-CF_3-Ph$ | 4-F-Ph | O | S-Bn | 131-132 |
| 1-226 | H | $4-CF_3-Ph$ | 4-F-Ph | O | NHOH | 165-166 |
| 1-227 | H | $4-CF_3-Ph$ | 4-F-Ph | O | NHOMe | 130-131 |
| 1-228 | H | $4-CF_3-Ph$ | 4-F-Ph | O | NHOEt | 182-183 |
| 1-229 | H | 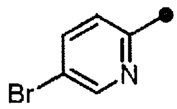 | 4-F-Ph | S | OH | 141-142 |
| 1-230 | H | 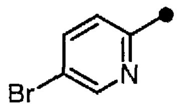 | 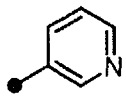 | S | OH | 187-188 |
| 1-231 | H | 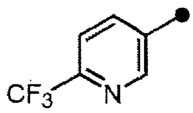 | 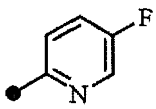 | S | OH | NMR |

[Table 11]

| the first table (continued) | | | | | | |
|---|---|---|---|---|---|---|
| compound No. | $R^1$ | Q | $R^3$ | Y | Z | physical property value |
| 1-232 | H | $4-SO_2Me-Ph$ | 4-Cl-Ph | O | OH | 179-180 |
| 1-233 | H | 4-SOMe-Ph | 4-Cl-Ph | O | OH | 217-218 |
| 1-234 | H | 4-SMe-Ph | 4-Cl-Ph | O | OH | 154-155 |
| 1-235 | H | $4-SCF_3-Ph$ | 4-Cl-Ph | O | OH | 175-176 |
| 1-236 | H | 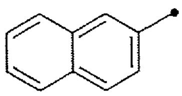 | 4-F-Ph | O | OH | 190-191 |
| 1-237 | H | 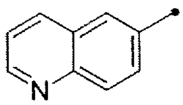 | 4-F-Ph | O | OH | 210-211 |
| 1-238 | H | $4-CF_3-Ph$ | 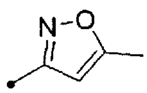 | O | OH | 212-213 |
| 1-239 | H | $4-CF_3-Ph$ | 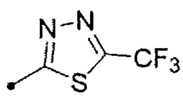 | O | OH | 254-255 |
| 1-240 | H | $4-CF_3-Ph$ | 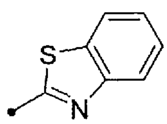 | O | OH | 290-291 |

(continued)

| the first table (continued) | | | | | | |
|---|---|---|---|---|---|---|
| compound No. | $R^1$ | Q | $R^3$ | Y | Z | physical property value |
| 1-241 | H | 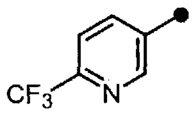 | 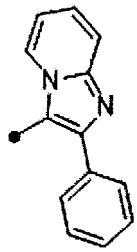 | O | OH | 153-155 |
| 1-242 | H | 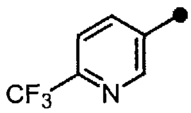 | 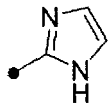 | O | OH | 258-262 ’ |
| 1-243 | H | 4-$CF_3$-Ph | 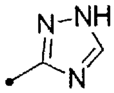 | O | OH | 298-299 |
| 1-244 | H | 4-$CF_3$-Ph | 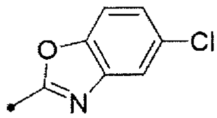 | O | OH | 219-220 |

[Table 12]

| the second table | |
|---|---|
| compound No. | $^1$H-NMR data ($CDCl_3$/TMS, ppm) |
| 1-10 | δ 9.88-9.87 (1H, m), 7.64-7.60 (2H, m), 7.38-7.34 (2H, m), 3.42 (2H, qd), 2.89-2.70 (5H, m), 1.25 (3H, t) |
| 1-11 | δ 9.98-9.92 (1H, m), 7.64-7.60 (2H, m), 7.38-7.34 (2H, m), 3.92-3.83 (1H, m), 3.48-3.39 (1H, m), 2.85-2.81 (2H, m), 2.75-2.69 (2H, m), 1.96-1.89 (2H, m), 1.77-1.71 (2H, m), 1.70-1.58 (1H, m), 1.46-1.22 (6H, m) |
| 1-12 | δ 10.21-10.17 (1H, m), 7.64-7.60 (2H, m), 7.39-7.29 (8H, m), 4.59-4.56 (2H, m), 3.50-3.40 (1H, m), 2.88-2.84 (2H, m), 2.76-2.66 (2H, m) |
| 1-13 | δ 10.11 (1H, t), 7.65-7.61 (2H, m), 7.38-7.34 (2H, m), 4.25 (2H, q), 4.14 (2H, dd), 3.54-3.41 (1H, m), 2.89-2.68 (4H, m), 1.31 (3H, t) |
| 1-32 | δ 12.13 (1H, s), 8.10 (2H, s), 7.67-7.64 (3H, m), 7.41-7.37 (2H, m), 3.57-3.48 (1H, m), 3.00-2.81 (4H, m) |
| 1-62* | δ 11.76 (1H, s), 7.86-7.82 (2H, m), 7.65-7.58 (4H, m), 7.28-7.20 (2H, m), 3.65-3.58 (1H, m), 3.24-2.60 (4H, m) |
| 1-63 | δ 15 (1H, s), 11.79 (1H, s), 7.50-7.57 (2H, m), 7.12-7.21 (4H, m), 7.09-7.20 (2H, m),3.33-3.47(1H, m) 2.70-2.92(4H, m), 2.35 (3H, s) |
| 1-90 | δ 12.38-12.38 (1H, m), 7.65-7.61 (2H, m), 7.38-7.34 (2H, m), 5.97 (1H, ddt), 5.33 (1H, ddt), 5.28 (1H, ddt), 4.29-4.25 (2H, m), 3.44 (1H, tt), 2.99-2.95 (2H, m), 2.85-2.74 (2H, m) |
| 1-94 | δ 13.85 (1H, s), 7.74-7.69 (1H, m), 7.67-7.63 (2H, m), 7.41-7.37 (2H, m), 7.34-7.30 (1H, m), 7.23-7.16 (2H, m), 3.56-3.46 (1H, m), 3.05-2.82 (4H, m) |

(continued)

| the second table | |
|---|---|
| compound No. | $^1$H-NMR data ($CDCl_3$/TMS, ppm) |
| 1-98 | δ 13.90 (1H, s), 7.67-7.63 (3H, m), 7.53-7.51 (1H, m), 7.41-7.37 (2H, m), 7.33-7.30 (1H, m), 3.57-3.47 (1H, m), 3.06-2.83 (4H, m) |
| 1-106 | δ 14.04 (1H, s), 7.94-7.83 (3H, m), 7.69-7.65 (2H, m), 7.64-7.60 (1H, m), 7.57-7.51 (3H, m), 7.44-7.40 (2H, m), 3.62-3.53 (1H, m), 3.09-2.91 (4H, m) |
| 1-107 | δ 10.20-10.12 (1H, m), 7.65-7.60 (2H, m), 7.38-7.34 (2H, m), 4.60 (1H, dq), 4.28-4.17 (2H, m), 3.50-3.40 (1H, m), 2.90-2.71 (4H, m), 1.33-1.27 (3H, m) |
| 1-129 | δ 17.39 (1H, s), 13.96 (1H, s), 6.90-7.40 (8H, m), 3.26-3.37(1H, m), 2.75-3.00 (10H, m) |
| 1-161 | δ11.8 (bs, 1H), 8.64 (d, 1H), 7.80 (dd, 1H), 7.56-7.50 (m, 2H), 7.12 (d, 1H), 7.06 (t, 2H), 3.58-3.47 (m, 1H), 3.19 (dd, 2H), 3.00-2.74 (m, 3H) |
| 1-231 | δ14.37(1H, s), 8.66-8.71(1H, m), 8.36-8.39(1H, m), 8.27-8.33(1H, m), 7.70-7.81(1H, m), 7.47-7.54(1H, m), 3.53-3.63(1H, m), 2.80-3.15(4H, m) |
| In Table 2, "*" shows the measurement results in DMSO-$d_6$. | |

Pest control agent containing the compound represented by the formula (1) or salts thereof of the present invention as an active ingredient can be applied as agrohorticultural insect pest control agents, ectoparasite control agents for animal, and endoparasite control agents for animal. Agrohorticultural insect pest control agents are applicable to controlling various insect pests such as agrohorticultural insect pests, stored grain insect pests, sanitary insect pests, nematodes, and the like, which are injurious to paddy rice, fruit trees, vegetables, other crops, flowers and ornamental plants.

In addition, pest control agents containing the compound represented by the formula (1) or salts thereof of the present invention as an active ingredient are superior in physical properties suitable for various labor-saving application methods, systemic transfer activity, and environmental safety such as appropriate soil persistence and the like.

Furthermore, pest control agents containing the compound represented by the formula (1) or salts thereof of the present invention as an active ingredient have little impact on natural enemies; useful insects such as Apis mellifera, bumblebee, and the like; environmental organisms such as midge and the like.

Furthermore, an ectoparasite control agent for animals and an endoparasite control agent for animals, particularly, an endoparasite control agent for animals, containing a compound represented by the formula (1) or salts thereof of the present invention as active ingredients are superior in safety and pharmacokinetics.

Examples of the above-mentioned insect pest, Nematoda and the like include the following.

Examples of the insect pest of Lepidoptera include Parasa consocia, Anomis mesogona, Papilio xuthus, Matsumuraeses azukivora, Ostrinia scapulalis, Spodoptera exempta, Hyphantria cunea, Ostrinia furnacalis, Pseudaletia separata, Tinea translucens, Bactra furfuryla, Parnara guttata, Marasmia exigua, Sesamia inferens, Brachmia triannulella, Monema flavescens, Trichoplusia ni, Pleuroptya ruralis, Cystidia couaggaria, Lampides boeticus, Cephonodes hylas, Helicoverpa armigera, Phalerodonta manleyi, Eumeta japonica, Pieris brassicae, Malacosoma neustria testacea, Stathmopoda masinissa, Cuphodes diospyrosella, Archips xylosteanus, Agrotis segetum, Tetramoera schistaceana, Papilio machaon hippocrates, Endoclyta sinensis, Lyonetia prunifoliella, Phyllonorycter ringoneella, Cydia kurokoi, Eucoenogenes aestuosa, Lobesia botrana, Latoia sinica, Euzophera batangensis, Phalonidia mesotypa, Spilosoma imparilis, Glyphodes pyloalis, Olethreutes mori, Tineola bisselliella, Endoclyta excrescens, Nemapogon granellus, Synanthedon hector, Cydia pomonella, Plutella xylostella, Cnaphalocrocis medinalis, Sesamia calamistis, Scirpophaga incertulas, Pediasia teterrellus, Phthorimaea operculella, Stauropus fagi persimilis, Etiella zinckenella, Spodoptera exigua, Palpifer sexnotata, Spodoptera mauritia, Scirpophaga innotata, Xestia c-nigrum, Spodoptera depravata, Ephestia kuehniella, Angerona prunaria, Clostera anastomosis, Pseudoplusia includens, Matsumuraeses falcana, Helicoverpa assulta, Autographa nigrisigna, Agrotis ipsilon, Euproctis pseudoconspersa, Adoxophyes orana, Caloptilia theivora, Homona magnanima, Ephestia elutella, Eumeta minuscula, Clostera anachoreta, Heliothis maritima, Sparganothis pilleriana, Busseola fusca, Euproctis subflava, Biston robustum, Heliothis zea, Aedia leucomelas, Narosoideus flavidorsalis, Viminia rumicis, Bucculatrix pyrivorella, Grapholita molesta, Spulerina astaurota, Ectomyelois pyrivorella, Chilo suppressalis, Acrolepiopsis sapporensis, Plodia interpunctella, Hellula undalis, Sitotroga cerealella, Spodoptera litura, Eucosma aporema, Acleris comariana, Scopelodes contractus, Orgyia thyellina, Spodoptera frugiperda, Ostrinia zaguliaevi, Naranga

aenescens, Andraca bipunctata, Paranthrene regalis, Acosmeryx castanea, Phyllocnistis toparcha, Endopiza viteana, Eupoecillia ambiguella, Anticarsia gemmatalis, Cnephasia cinereipalpana, Lymantria dispar, Dendrolimus spectabilis, Leguminivora glycinivorella, Maruca testulalis, Matsumuraeses phaseoli, Caloptilia soyella, Phyllocnistis citrella, Omiodes indicate, Archips fuscocupreanus, Acanthoplusia agnata, Bambalina sp., Carposina niponensis, Conogethes punctiferalis, Synanthedon sp., Lyonetia clerkella, Papilio helenus, Colias erate poliographus, Phalera flavescens, Pieridae such as Pieris rapae crucivora, Pieris rapae, and the like, Euproctis similis, Acrolepiopsis suzukiella, Ostrinia nubilalis, Mamestra brassicae, Ascotis selenaria, Phtheochroides clandestina, Hoshinoa adumbratana, Odonestis pruni japonensis, Triaena intermedia, Adoxophyes orana fasciata, Grapholita inopinata, Spilonota ocellana, Spilonota lechriaspis, Illiberis pruni, Argyresthia conjugella, Caloptilia zachrysa, Archips breviplicanus, Anomis flava, Pectinophora gossypiella, Notarcha derogata, Diaphania indica, Heliothis virescens, Earias cupreoviridis, and the like.

Examples of the insect pest of Hemiptera include Nezara antennata, Stenotus rubrovittatus, Graphosoma rubrolineatum, Trigonotylus coelestialium, etc., Aeschynteles maculatus, Creontiades pallidifer, Dysdercus cingulatus, Chrysomphalus ficus, Aonidiella aurantii, Graptopsaltria nigrofuscata, Blissusleucopterus, Icerya purchasi, Piezodorus hybneri, Lagynotomus elongatus, Thaia subrufa, Scotinophara lurida, Sitobion ibarae, Stariodes iwasakii, Aspidiotus destructor, Taylorilygus pallidulus, Myzusmumecola, Pseudaulacaspis prunicola, Acyrthosiphon pisum, Anacanthocoris striicornis, Ectometopterus micantulus, Eysarcoris lewisi, Molipteryx fuliginosa, Cicadella viridis, Rhopalosophum rufiabdominalis, Saissetia oleate, Trialeurodes vaporariorum, Aguriahana quercus, Lygus spp., Euceraphis punctipennis, Andaspis kashicola, Coccus pseudomagnoliarum, Cavelerius saccharivorus, Galeatus spinifrons, Macrosiphoniella sanborni, Aonidiella citrina, Halyomorpha mista, Stephanitis fasciicarina, Trioza camphorae, Leptocorisa chinensis, Trioza quercicola, Uhlerites latius, Erythroneura comes, Paromius exiguus, Duplaspidiotus claviger, Nephotettix nigropictus, Halticiellus insularis, Perkinsiella saccharicida, Psylla malivorella, Anomomeura mori, Pseudococcus longispinis, Pseudaulacaspis pentagona, Pulvinaria kuwacola, Apolygus lucorum, Togo hemipterus, Toxoptera aurantii, Saccharicoccus sacchari, Geoica lucifuga, Numata muiri, Comstockaspis perniciosa, Unaspis citri, Aulacorthum solani, Eysarcoris ventralis, Bemisia argentifolii, Cicadella spectra, Aspidiotus hederae, Liorhyssus hyalinus, Calophya nigridorsalis, Sogatella furcifera, Megoura crassicauda, Brevicoryne brassicae, Aphis glycines, Leptocorisa oratorius, Nephotettix virescens, Uroeucon formosanum, Cyrtopeltis tennuis, Bemisia tabaci, Lecanium persicae, Parlatoria theae, Pseudaonidia paeoniae, Empoasca onukii, Plautia stall, Dysaphis tulipae, Macrosiphum euphorbiae, Stephanitis pyrioides, Ceroplastes ceriferus, Parlatoria camelliae, Apolygus spinolai, Nephotettix cincticeps, Glaucias subpunctatus, Orthotylus flavosparsus, Rhopalosiphum maidis, Peregrinus maidis, Eysarcoris parvus, Cimex lectularius, Psylla abieti, Nilaparvata lugens, Psylla tobirae, Eurydema rugosum, Schizaphis piricola, Psylla pyricola, Parlatoreopsis pyri, Stephanitis nashi, Dysmicoccus wistariae, Lepholeucaspis japonica, Sappaphis piri, Lipaphis erysimi, Neotoxoptera formosana, Rhopalosophum nymphaeae, Edwardsianarosae, Pinnaspisaspidistrae, Psylla alni, Speusotettix subfusculus, Alnetoidia alneti, Sogatella panicicola, Adelphocoris lineolatus, Dysdercus poecilus, Parlatoria ziziphi, Uhlerites debile, Laodelphax striatellus, Eurydema pulchrum, Cletus trigonus, Clovia punctata, Empoasca sp., Coccus hesperidum, Pachybrachius luridus, Planococcus kraunhiae, Stenotus binotatus, Arboridia apicalis, Macrosteles fascifrons, Dolycoris baccarum, Adelphocoris triannulatus, Viteus vitifolii, Acanthocoris sordidus, Leptocorisa acuta, Macropes obnubilus, Cletus punctiger, Riptortus clavatus, Paratrioza cockerelli, Aphrophora costalis, Lygus disponsi, Lygus saundersi, Crisicoccus pini, Empoasca abietis, Crisicoccus matsumotoi, Aphis craccivora, Megacopta punctatissimum, Eysarcoris guttiger, Lepidosaphes beckii, Diaphorina citri, Toxoptera citricidus, Planococcus citri, Dialeurodes citri, Aleurocanthus spiniferus, Pseudococcus citriculus, Zyginella citri, Pulvinaria citricola, Coccus discrepans, Pseudaonidia duplex, Pulvinaria aurantii, Lecanium corni, Nezara viridula, Stenodema calcaratum, Rhopalosiphum padi, Sitobion akebiae, Schizaphis graminum, Sorhoanus tritici, Brachycaudus helichrysi, Carpocoris purpureipennis, Myzus persicae, Hyalopterus pruni, Aphis farinose yanagicola, Metasalis populi, Unaspis yanonensis, Mesohomotoma camphorae, Aphis spiraecola, Aphis pomi, Lepidosaphes ulmi, Psylla mali, Heterocordylus flavipes, Myzus malisuctus, Aphidonuguis mali, Orientus ishidai, Ovatus malicolens, Eriosoma lanigerum, Ceroplastes rubens, Aphis gossypii, and the like.

Examples of the insect pest of Coleoptera include Xystrocera globosa, Paederus fuscipes, Eucetonia roelofsi, Callosobruchus chinensis, Cylas formicarius, Hypera postica, Echinocnemus squameus, Oulema oryzae, Donacia provosti, Lissorhoptrus oryzophilus, Colasposoma dauricum, Euscepes postfasciatus, Epilachna varivestis, Acanthoscelides obtectus, Diabrotica virgifera virgifera, Involvulus cupreus, Aulacophora femoralis, Bruchus pisorum, Epilachna vigintioctomaculata, Carpophilus dimidiatus, Cassida nebulosa, Luperomorpha tunebrosa, Phyllotreta striolata, Psacothea hilaris, Aeolesthes chrysothrix, Curculio sikkimensis, Carpophilus hemipterus, Oxycetonia jucunda, Diabrotica spp., Mimela splendens, Sitophilus zeamais, Tribolium castaneum, Sitophilus oryzae, Palorus subdepressus, Melolontha japonica, Anoplophora malasiaca, Neatus picipes, Leptinotarsa decemlineata, Diabrotica undecimpunctata howardi, Sphenophorus venatus, Crioceris quatuordecimpunctata, Conotrachelus nenuphar, Ceuthorhynchidius albosuturalis, Phaedon brassicae, Lasioderma serricorne, Sitona japonicus, Adoretus tenuimaculatus, Tenebrio molitor, Basilepta balyi, Hypera nigrirostris, Chaetocnema concinna, Anomala cuprea, Heptophylla picea, Epilachna vigintioctopunctata, Diabrotica longicornis, Eucetonia pilifera, Agriotes spp., Attagenus unicolor japonicus, Pagria signata, Anomala rufocuprea, Palorus ratzeburgii, Alphitobius laevigatus, Anthrenus verbasci, Lyctus brunneus, Tribolium confusum, Medythia

nigrobilineata, Xylotrechus pyrrhoderus, Epitrix cucumeris, Tomicus piniperda, Monochamus alternatus, Popillia japonica, Epicauta gorhami, Sitophilus zeamais, Rhynchites heros, Listroderes costirostris, Callosobruchus maculatus, Phyllobius armatus, Anthonomus pomorum, Linaeidea aenea, Anthonomus grandis, and the like.

Examples of the insect pest of Diptera include Culex pipiens pallens, Pegomya hyoscyami, Liriomyza huidobrensis, Musca domestica, Chlorops oryzae, Hydrellia sasakii, Agromyza oryzae, Hydrellia griseola, Ophiomyia phaseoli, Dacus cucurbitae, Drosophila suzukii, Rhacochlaena japonica, Muscina stabulans, Phoridae such as Megaselia spiracularis and the like, Clogmia albipunctata, Tipula aino, Phormia regina, Culex tritaeniorhynchus, Anopheles sinensis, Hylemya brassicae, Asphondylia sp., Delia platura, Delia antiqua, Rhagoletis cerasi, Culex pipiens molestus Forskal, Ceratitis capitata, Bradysia agrestis, Pegomya cunicularia, Liriomyza sativae, Liriomyza bryoniae, Chromatomyia horticola, Liriomyza chinensis, Culex quinquefasciatus, Aedes aegypti, Aedes albopictus, Liriomyza trifolii, Liriomyza sativae, Dacus dorsalis, Dacus tsuneonis, Sitodiplosis mosellana, Meromuza nigriventris, Anastrepha ludens, Rhagoletis pomonella, and the like.

Examples of the insect pest of Hymenoptera include Pristomyrmex pungens, Bethylidae, Monomorium pharaohnis, Pheidole noda, Athalia rosae, Dryocosmus kuriphilus, Formica fusca japonica, Vespoidea, Athalia infumata infumata, Arge pagana, Athalia japonica, Acromyrmex spp., Solenopsis spp., Arge mali, Ochetellus glaber, and the like.

Examples of the insect pest of Orthoptera include Homorocoryphus lineosus, Gryllotalpa sp., Oxya hyla intricata, Oxya yezoensis, Locusta migratoria, Oxya japonica, Homorocoryphus jezoensis, Teleogryllus emma, and the like.

Examples of the insect pest of Thysanoptera include Selenothrips rubrocinctus, Stenchaetothrips biformis, Haplothrips aculeatus, Ponticulothrips diospyrosi, Thrips flavus, Anaphothrips obscurus, Liothrips floridensis, Thrips simplex, Thrips nigropilosus, Heliothrips haemorrhoidalis, Pseudodendrothrips mori, Microcephalothrips abdominalis, Leeuwenia pasanii, Litotetothrips pasaniae, Scirtothrips citri, Haplothrips chinensis, Mycterothrips glycines, Thrips setosus, Scirtothrips dorsalis, Dendrothrips minowai, Haplothrips niger, Thrips tabaci, Thrips alliorum, Thrips hawaiiensis, Haplothrips kurdjumovi, Chirothrips manicatus, Frankliniella intonsa, Thrips coloratus, Franklinella occidentalis, Thrips palmi, Frankliniella lilivora, Liothrips vaneeckei, and the like.

Examples of the insect pest of Acari include Leptotrombidium akamushi, Tetranychus ludeni, Dermacentor variabilis, Tetranychus truncatus, Ornithonyssus bacoti, Demodex canis, Tetranychus viennensis, Tetranychus kanzawai, Ixodes such as Rhipicephalus sanguineus, and the like, Cheyletus malaccensis, Tyrophagus putrescentiae, Dermatophagoides farinae, Latrodectus hasseltii, Dermacentor taiwanicus, Acaphylla theavagrans, Polyphagotarsonemus latus, Aculops lycopersici, Ornithonyssus sylvairum, Tetranychus urticae, Eriophyes chibaensis, Sarcoptes scabiei, Haemaphysalis longicornis, Ixodes scapularis, Tyrophagus similis, Cheyletus eruditus, Panonychus citri, Cheyletus moorei, Brevipalpus phoenicis, Octodectes cynotis, Dermatophagoides ptrenyssnus, Haemaphysalis flava, Ixodes ovatus, Phyllocoptruta citri, Aculus schlechtendali, Panonychus ulmi, Amblyomma americanum, Dermanyssus gallinae, Rhyzoglyphus robini, Sancassania sp., and the like.

Examples of the insect pest of Isoptera include Reticulitermes miyatakei, Incisitermes minor, Coptotermes formosanus, Hodotermopsis japonica, Reticulitermes sp., Reticulitermes flaviceps amamianus, Glyptotermes kushimensis, Coptotermes guangzhoensis, Neotermes koshunensis, Glyptotermes kodamai, Glyptotermes satsumensis, Cryptotermes domesticus, Odontotermes formosanus, Glyptotermes nakajimai, Pericapritermes nitobei, Reticulitermes speratus, and the like.

Examples of the insect pest of Blattodea include Periplaneta fuliginosa, Blattella germanica, Blatta orientalis, Periplaneta brunnea, Blattella lituricollis, Periplaneta japonica, Periplaneta americana, and the like.

Examples of the Nematoda include Nothotylenchus acris, Aphelenchoides besseyi, Pratylenchus penetrans, Meloidogyne hapla, Meloidogyne incognita, Globodera rostochiensis, Meloidogyne javanica, Heterodera glycines, Pratylenchus coffeae, Pratylenchus neglectus, Tylenchus semipenetrans, and the like.

Examples of the Mollusca include Pomacea canaliculata, Achatina fulica, Meghimatium bilineatum, Lehmannina valentiana, Limax flavus, Acusta despecta sieboldiana, and the like.

The agrohorticultural insect pest control agent of the present invention also has a strong control effect on Tuta absoluta as other insect pest.

The agrohorticultural insect pest control agent containing the compound represented by the formula (1) or salts thereof of the present invention as an active ingredient has a marked control effect on the aforementioned insect pests, which are injurious to paddy field crops, upland crops, fruit trees, vegetables and other crops, flowers and ornamental plants, and the like. Therefore, the desired effect of the agrohorticultural insect pest control agent of the present invention can be exhibited by applying the agents to propagation facility, paddy field, field, fruit trees, vegetables, other crops, seeds of flowers and ornament plants, cultivation carriers such as paddy field water, stem and leaves, soil, and the like, and the like, at a season at which the insect pests are expected to appear, before their appearance or at the time when their appearance is confirmed. Particularly, preferred use form is application utilizing what is called systemic/penetration transferability by allowing absorption of the compound of the present invention from roots via or not via soil, by treating propagation soil for crops, flowers, ornamental plants, and the like, planting hole soil for transplantation, plant foot, irrigation water, culture water for hydroponic culture, and the like.

The useful plants, for which the agrohorticultural insect pest control agent of the present invention can be used, are not specifically limited, and examples thereof include plants such as cereals (e.g. rice, barley, wheat, rye, oat, corn, etc.), legume (soybean, adzuki bean, fava bean, bean, kidney bean, peanut, etc.), fruit trees and fruits (apple, citrus fruits, pear, grapes, peach, plum, cherry fruit, walnut, sweet chestnut, almond, banana, etc.), vegetables (cabbage, tomato, spinach, broccoli, lettuce, onion, green onion (chive, shallot), green pepper, eggplant, strawberry, pepper, okra, green chive, etc.), root vegetables (carrot, potato, sweet potato, tannia, radish, turnip, lotus root, burdock, garlic, Allium bakeri, etc.), crop for processing (cotton, hemp, beet, hop, sugar cane, sugar beet, olive, rubber, coffee, tobacco, tea, etc.), gourd (pumpkin, cucumber, watermelon, oriental melon, melon, etc.), feed crop (orchard grass, sorghum, timothy, clover, alfalfa, etc.), grass (Korean lawn grass, bent grass, etc.), ornamental plants such as spices (lavender, rosemary, thyme, parsley, pepper, ginger, etc.), flowers and ornamental plants (chrysanthemum, rose, carnation, orchid, tulip, lily, etc.), garden tree (gingko, Japanese cherry, aucuba, etc.), forest tree (Abies sachalinensis, Picea jezoensis, pines, Thujopsis dolabrata, Japanese cedar, Japanese cypress, eucalyptus, etc.), and the like.

The above-mentioned "plant" also includes plants imparted with resistance to herbicides, for example, HPPD inhibitors such as isoxaflutole and the like, ALS inhibitors such as imazethapyr, thifensulfuron methyl, and the like, EPSP synthetase inhibitors such as glyphosate and the like, glutamine synthetase inhibitors such as glufosinate and the like, acetyl CoA carboxylase inhibitors such as sethoxydim and the like, broxynil, dicamba, 2,4-D, and the like, by a classical breeding method or gene recombination technique.

Examples of the "plant" imparted with resistance by a classical breeding method include colza, wheat, sunflower, and rice resistant to imidazolinone ALS inhibitory herbicides such as imazethapyr and the like, which are already commercially available as a trade name of "Clearfield" (registered trade mark). Similarly, soybean resistant to sulfonylurea ALS inhibitory herbicides such as thifensulfuron methyl and the like by a classical breeding method is already commercially available as a trade name of "STS soybean". Similarly, examples of the plant imparted with resistance to acetyl CoA carboxylase inhibitors such as trione oxime-based and aryloxyphenoxypropionic acid-based herbicides and the like, by a classical breeding method include SR corn and the like.

The plant imparted with resistance to acetyl CoA carboxylase inhibitors is described in Proc. Natl. Acad. Sci. USA, vol. 87, pages 7175 - 7179 (1990) and the like. In addition, mutated acetyl CoA carboxylase resistant to acetyl CoA carboxylase inhibitors is reported in Weed Science, vol. 53, pages 728 - 746 (2005) and the like, and a plant resistant to acetyl CoA carboxylase inhibitors can be created by introducing such mutated acetyl CoA carboxylase gene into a plant by a gene recombination technique or introducing a mutation involved in imparting resistance into acetyl CoA carboxylase of the plant. Furthermore, a plant resistant to acetyl CoA carboxylase inhibitor, ALS inhibitor, and the like can be created by introducing a mutation of site-specific amino acid substitution into the acetyl CoA carboxylase gene, ALS gene, and the like of the plant by introducing a base-substituted and mutation-introducing nucleic acid represented by a chimeraplasty technique (Gura T. 1999. Repairing the Genome's Spelling Mistakes. Science 285: 316-318.) into the plant cell. The agrohorticultural insect pest control agent of the present invention can also be used for these plants.

Furthermore, as the toxins produced in such genetically modified plants, insecticidal proteins derived from Bacillus cereus or Bacillus popilliae; insecticidal proteins such as $\delta$-endotoxins (e.g. CrylAb, CrylAc, CrylF, CrylFa2, Cry2Ab, Cry3A, Cry3Bbl, Cry9C, etc.), VIP1, VIP2, VIP3, VIP3A, and the like, which are derived from Bacillus thuringiensis; insecticidal proteins derived from nematodes; toxins produced by animals, such as scorpion toxin, spider toxin, bee toxin, insect-specific neurotoxins, and the like; filamentous fungi toxins; plant lectins; agglutinin; protease inhibitors such as trypsin inhibitor, serine protease inhibitor, patatin, cystatin, papain inhibitor, and the like; ribosome-inactivating proteins (RIP) such as ricin, corn-RIP, abrin, rufin, sapolin, priodin, and the like; steroid metabolic enzymes such as 3-hydroxysteroid oxidase, ecdysteroid-UDP-glucosyltransferase, cholesterol oxidase, and the like; ecdysone inhibitors; HMG-CoA reductase; ion channel inhibitors such as a sodium channel inhibitor, calcium channel inhibitor, and the like; juvenile hormone esterase; diuretic hormone acceptor; stilbene synthetase; bibenzyl synthetase; chitinase; glucanase, and the like can be mentioned.

The toxins produced in such genetically modified plants also include hybrid toxins, partially deficient toxins, and modified toxins of insecticidal proteins, such as $\delta$-endotoxin proteins (e.g. CrylAb, CrylAc, CrylF, CrylFa2, Cry2Ab, Cry3A, Cry3Bbl, Cry9C, Cry34Ab, and Cry35Ab), VIPl, VIP2, VIP3, VIP3A, and the like. The hybrid toxins are produced by a novel combination of the different domains of such a protein by adopting recombination technology. The known partially deficient toxin is CrylAb, in which a part of amino acid sequence is deficient. In the modified toxins, one or a plurality of amino acids of a natural toxin is/are replaced.

Examples of such toxins and genetically modified plants capable of synthesizing such toxins are described in EP-A-0 374 753, WO 93/07278, WO 95/34656, EP-A-0 427 529, EP-A-451 878, WO 03/052073, and the like.

The toxins contained in such genetically modified plants impart resistance to insect pests of Coleoptera, insect pests of Hemipteria, insect pests of Diptera, insect pests of Lepidoptera, and Nematoda to the plants. The agrohorticultural insect pest control agent of the present invention can also be used in combination with or by systematization with such technique.

For control of various insect pests, the agrohorticultural insect pest control agent of the present invention need

only be applied to the plant expected to allow occurrence of insect pest and nematode, as it is or after being appropriately diluted with or suspended in water or the like, in an amount effective for control of the insect pest or nematode. For example, for insect pest and nematode that occur in fruit trees, cereals, vegetables, and the like, the agent is sprayed on the stem and leaf parts, or absorbed from the root by a seed treatment such as dipping the seeds in a drug, dressing of seeds, Calper treatment, and the like, a soil treatment such as soil whole layer blending, planting row application, bed soil blending, cell seedling treatment, planting hole treatment, plant foot/base treatment, top dress, nursery box treatment of rice, application on water surface, and the like, or the like. In addition, the agrohorticultural insect pest control agent can also be used by application to a culture fluid for culture fluid (hydroponic) culture, application by smoking, tree stem injection, or the like.

The agrohorticultural insect pest control agent of the present invention need only be applied to the place expected to allow development of insect pests, as it is or after being appropriately diluted with or suspended in water or the like, in an amount effective for control of the insect pests. For example, spraying on grain-storage insect pests, house insect pests, hygiene insect pests, forest insect pests, and the like, as well as application, smoking, bait to house and architectural materials and the like can also be employed for use.

As methods for the seed treatment, a method comprising penetration of a liquid agent obtained by diluting or not a liquid formulation or a solid formulation by dipping seeds in the agent, a method including adhering a solid formulation or liquid formulation to the surfaces of seeds by admixing and dressing the seeds with the formulation, a method comprising coating seeds by admixing the seeds with adhesive carriers such as resin, polymer, and the like, a method comprising applying near the seeds simultaneously with planting, and the like can be mentioned.

The "seed" to be subjected to the seed treatment means a plant body in an early stage of growth, which is used for propagation, and examples thereof include seeds, as well as bulb, tuber, seed potato, germ, propagule, scaly bulb, a plant body for vegetative propagation by cutting propagation, and the like.

The "soil" and "cultivation carrier" for plant when practicing the method of use of the present invention mean supports for cultivation of plants, particularly supports for growing roots, and the material is not particularly limited. The material may be any as long as plants can grow therein and specific materials include, for example, soils, nursery mat, water, and the like. As a specific material, for example, sand, pumice, vermiculite, diatomaceous earth, agar, gelled substance, polymer substance, rock wool, glass wool, wood chip, bark, and the like can also be used.

As a method of application to crop plant stem and leaves, grain storage insect pests, house insect pests, sanitary insect pests, forest insect pests, or the like, a method including appropriately diluting a liquid formulation such as emulsion, flowable formulation, and the like, or a solid formulation such as a wettable powder, water dispersible granule, and the like with water, and spraying same, a method of spraying a dust, smoking, and the like can be mentioned.

For application to the soil, for example, a method comprising application of a liquid formulation to the plant foot of plant body, nursery for raising seedling and the like with or without dilution in water, a method comprising application of granules to the plant foot of plant body or nursery for raising seedling, a method comprising application of dust, wettable powder, water dispersible granule, granule, and the like before sowing or transplanting to allow them to be incorporated into the entire soil, a method comprising application of dust, wettable powder, water dispersible granule, granule, and the like to planting hole, planting row, and the like before sowing or planting, and the like can be mentioned.

In the case of the paddy rice nursery box, the formulation may vary depending on the timing of, for example, application on sowing, application during greening, application during transplanting, and the like. The formulations of dust, water dispersible granule, granule, and the like can be employed. Application is also possible by incorporation into the grove soil, wherein the grove soil may be mixed with dust, water dispersible granule, granule, and the like and, for example, incorporation into bed soil, incorporation into cover soil, incorporation into the entire grove soil, and the like can be employed. Simply, a grove soil and various formulations may be applied in alternate layers.

As a method of application to a paddy field, a solid formulation such as jumbo, pack, granule, water dispersible granule, and the like, a liquid formulation such as flowable, emulsion, and the like are generally sprayed on a flooded paddy field. Alternatively, during rice planting, a suitable formulation can also be applied or injected to soil directly or after blending with a fertilizer. Moreover, by utilizing a liquid agent such as emulsion, flowable, and the like to a water inlet and the flow source of water into paddy fields such as an irrigation apparatus and the like, a saving application along with the supply of water can also be performed.

For upland crops, treatment of seeds, a cultivation carrier to be placed near the plant body, and the like during the period of from sowing to raising seedling is possible. For plants to be directly sown in the field, a direct treatment of seeds, a treatment of a plant foot of the plant under cultivation, and the like are preferred. An application of granules, a soil injection treatment with a liquid agent with or without dilution with water, and the like can be performed. It is also a preferred treatment to blend granules with a cultivation carrier before seeding, and seed the blend.

For a treatment on sowing or during raising seedling of a cultivated plant to be transplanted, a direct treatment of seeds, a soil injection treatment of nursery for raising seedling with a liquid agent, and a dispersal treatment thereof with granules are preferred. In addition, treatment of a planting hole with granules and mixing of a cultivation carrier to be placed near the transplantation site with the granules during fix planting are also preferred treatments.

The agrohorticultural insect pest control agent of the present invention is generally prepared into conveniently usable forms according to an ordinary manner for preparation of agrochemicals.

That is, the compound represented by the formula (1) or salts thereof of the present invention are blended with a suitable inert carrier and, optionally, an adjuvant in a proper proportion and prepared into a suitable preparation form such as a suspension, emulsion, liquid formulation, wettable powder, granular wettable powder, granules, dust, tablets, pack, or the like through dissolution, dispersion, suspension, mixing, impregnation, adsorption, or sticking.

The agrohorticultural insect pest control agent of the present invention can contain, besides active ingredients as necessary, additional components generally used for pesticide preparation. Examples of the additional component include carriers such as solid carrier, liquid carrier, and the like, surfactant, dispersant, wetting agent, binder, tackifier, thickener, colorant, spreader, sticker, antifreezing agent, anticaking agent, disintegrant, decomposition inhibitor, and the like. Where necessary, preservative, plant detritus, and the like may be used as other additional components. These addition components may be used alone or in a mixture of two or more kinds thereof.

Examples of the solid carrier include natural minerals such as quartz, clay, kaolinite, pyrophyllite, sericite, talc, bentonite, acid clay, attapulgite, zeolite, diatomaceous earth, and the like, inorganic salts such as calcium carbonate, ammonium sulfate, sodium sulfate, potassium chloride, and the like, organic solid carriers such as synthetic silicic acid, synthetic silicate, starch, cellulose, vegetable powder (e.g., sawdust, coconut shell, corn cob, tobacco stalk, etc.), and the like, plastic carriers such as polyethylene, polypropylene, poly(vinylidene chloride), and the like, urea, hollow inorganic bodies, hollow plastic bodies, fumed silica (white carbon), and the like. These may be used alone or in a mixture of two or more kinds thereof.

Examples of the liquid carrier include alcohols including monohydric alcohols such as methanol, ethanol, propanol, isopropanol, butanol, and the like, and polyvalent alcohols such as ethylene glycol, diethylene glycol, propylene glycol, hexylene glycol, polyethylene glycol, polypropylene glycol, glycerol, and. the like, polyhydric alcohol compounds such as propylene glycol ether and the like, ketones such as acetone, methyl ethyl ketone, methyl isobutyl ketone, diisobutyl ketone, cyclohexanone, and the like, ethers such as ethyl ether, dioxane, ethylene glycol monoethyl ether, dipropyl ether, THF, and the like, aliphatic hydrocarbons such as normal paraffin, naphthene, isoparaffin, kerosene, mineral oil, and the like, aromatic hydrocarbons such as benzene, toluene, xylene, solvent naphtha, alkylnaphthalene, and the like, halogenated hydrocarbons such as dichloromethane, chloroform, carbon tetrachloride, and the like, esters such as ethyl acetate, diisopropyl phthalate, dibutyl phthalate, dioctyl phthalate, dimethyl adipate, and the like, lactones such as γ-butyrolactone and the like, amides such as dimethylformamide, diethylformamide, dimethylacetamide, N-alkylpyrrolidinone, and the like, nitriles such as acetonitrile and the like, sulfur compounds such as dimethyl sulfoxide and the like, vegetable oils such as soybean oil, rapeseed oil, cottonseed oil, castor oil, and the like, water, and the like. These may be used alone or in a mixture of two or more kinds thereof.

Examples of the surfactant to be used as a dispersant or moistening agent include nonionic surfactants such as sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkyl aryl ether, polyoxyethylene alkyl phenyl ether, polyoxyethylene dialkylphenyl ether, polyoxyethylene alkyl phenyl ether-formalin condensate, polyoxyethylene-polyoxypropylene block copolymer, polyoxystyrene-polyoxyethylene block copolymer, alkyl polyoxyethylene-polypropylene block copolymer ether, polyoxyethylenealkylamine, polyoxyethylene fatty acid amide, polyoxyethylene fatty acid bisphenyl ether, polyalkylene benzyl phenyl ether, polyoxyalkylene styryl phenyl ether, acetylene diol, polyoxyalkylene-added acetylene diol, polyoxyethylene ether-type silicone-, ester-type silicone, and fluorine-surfactants, polyoxyethylene castor oil, hydrogenated polyoxyethylene castor oil, and the like, anionic surfactants such as alkyl sulfate salt, polyoxyethylene alkyl ether sulfate salt, polyoxyethylene alkyl phenyl ether sulfate salt, polyoxyethylene styryl phenyl ether sulfate salt, alkylbenzenesulfonate salt, alkylarylsulfonate salt, lignin sulfonate salt, alkylsulfosuccinate salt, naphthalenesulfonate salt, alkylnaphthalenesulfonate salt, salt of formalin condensate of naphthalenesulfonic acid, salt of formalin condensate of alkylnaphthalenesulfonic acid, fatty acid salt, polycarboxylate salt, polyacrylate salt, N-methyl-fatty acid sarcosinate, resin acid salt, polyoxyethylene alkyl ether phosphate salt, polyoxyethylene alkyl phenyl ether phosphate salt, and the like, cationic surfactants such as laurylamine hydrochloride, stearylamine hydrochloride, oleylamine hydrochloride, stearylamine acetate salt, stearylaminopropylamine acetate salt, alkylamine salts including alkyltrimethylammonium chloride and alkyldimethylbenzalkonium chloride and the like, ampholytic surfactants such as amino acid- and betaine-surfactants and the like, and the like. These surfactants may be used alone or in a mixture of two or more kinds thereof.

Examples of the binder and tackifier include carboxymethylcellulose or salt thereof, dextrin, water-soluble starch, xanthan gum, guar gum, sucrose, polyvinylpyrrolidone, gum arabic, polyvinyl alcohol, polyvinyl acetate, sodium polyacrylate, polyethylene glycol having an average molecular weight of 6,000 to 20,000, polyethylene oxide having an average molecular weight of 100,000 to 5,000,000, phospholipid (e.g. cephalin, lecithin, etc.), cellulose powder, dextrin, processed starch, polyaminocarboxylic acid chelate compound, crosslinked polyvinylpyrrolidone, maleic acid-styrene copolymer, (meth)acrylic acid copolymer, half ester of polyhydric alcohol polymer and dicarboxylic anhydride, water-soluble salt of polystyrenesulfonic acid, paraffin, terpene, polyamide resin, polyacrylic acid salt, polyoxyethylene, wax,

polyvinylalkyl ether, alkylphenol formaldehyde condensate, synthetic resin emulsion, and the like.

Examples of the thickener include water-soluble polymers such as xanthan gum, guar gum, diutan gum, carboxymethylcellulose, polyvinylpyrrolidone, carboxyvinyl polymer, acrylic polymer, starch compound, and polysaccharide, inorganic fine powders such as high-purity bentonite and fumed silica (white carbon), and the like.

Examples of the colorant include inorganic pigments such as iron oxide, titanium oxide, and Prussian blue, organic dyes such as an alizarin dye, azo dye, and metal phthalocyanine dye, and the like.

Examples of the antifreezing agent include polyvalent alcohols such as ethylene glycol, diethylene glycol, propylene glycol, glycerol, and the like, and the like.

Examples of the aids for anticaking or disintegrating include polysaccharides such as starch, alginic acid, mannose, galactose, and the like, polyvinylpyrrolidone, fumed silica (white carbon), ester gum, petroleum resin, sodium tripolyphosphate, sodium hexametaphosphate, metal stearate, cellulose powder, dextrin, methacrylate copolymer, polyvinylpyrrolidone, polyaminocarboxylic acid chelate compound, sulfonated styrene-isobutylene-maleic anhydride copolymer, and starch-polyacrylonitrile graft copolymer, and the like.

Examples of the decomposition inhibitor include desiccants such as zeolite, calcined lime, and magnesium oxide, antioxidants such as phenol compound, amine compound, sulfur compound, phosphoric acid compound, and the like, ultraviolet absorbers such as salicylic acid compound, benzophenone compound, and the like, and the like.

Examples of the preservative include potassium sorbate, 1,2-benzothiazolin-3-one, and the like.

Further, functional spreading agents, active enhancers for metabolic decomposition inhibitors such as piperonyl butoxide and the like, anti-freezing agents such as propylene glycol and the like, antioxidants such as BHT and the like, ultraviolet absorbers, and other aids may also be used as necessary.

The content of the compound as an active ingredient may be increased or decreased as necessary, and the compound may be used in a proportion appropriately chosen from the range of 0.01 to 90 parts by weight per 100 parts by weight of the agrohorticultural insect control agent of the present invention. For example, in the case of dusts, granules, emulsion, or wettable powders, the suitable content is from 0.01 to 50 parts by weight (0.01 to 50 wt% of the total weight of the agrohorticultural insect control agent).

The amount of use of the agrohorticultural insect pest control agent of the present invention varies depending on various factors such as purpose, target insect pest, growth state of plant, tendency of insect pest outbreak, weather, environmental conditions, dosage form, application method, application site, application time, and the like. It may be appropriately chosen from the range of 0.001 g to 10 kg, preferably 0.01 g to 1 kg, in terms of the compound as an active ingredient, per 10 ares depending on the purpose.

The agrohorticultural insect pest control agent of the present invention may be used in admixture with other agrohorticultural insecticides, acaricides, nematicides, fungicides, biological control agents, or the like in order to expand control target insect pests and the period suitable for control, or to reduce the drug amount. Furthermore, it may be used in admixture with herbicides, plant growth regulators, fertilizers, and the like, depending on the usage situation.

Examples of other agrohorticultural insecticide, acaricide and nematicide to be used for such purpose include 3,5-xylyl methylcarbamate (XMC), fenobucarb (BPMC), Bt toxin insecticide compound, CPCBS (chlorfenson), DCIP (dichlorodiisopropyl ether), D-D (1,3-Dichloropropene), DDT, NAC, O-4-dimethylsulfamoylphenyl O,O-diethyl phosphorothioate (DSP), O-ethyl O-4-nitrophenyl phenylphosphonothioate (EPN), tripropylisocyanurate (TPIC), acrinathrin, azadirachtin, acynonapyr, azinphos-methyl, acequinocyl, acetamiprid, acetoprole, acephate, abamectin, afidopyropen, avermectin-B, amidoflumet, amitraz, alanycarb, aldicarb, aldoxycarb, aldrin, alpha-endosulfan, alpha-cypermethrin, albendazole, allethrin, isazofos, isamidofos, isoamidofos, isoxathion, isocycloseram, isofenphos, isoprocarb (MIPC), epsilon-metofluthrin, epsilon-momfluorothrin, ivermectin, imicyafos, imidacloprid, imiprothrin, indazapyroxamet, indoxacarb, esfenvalerate, ethiofencarb, ethion, ethiprole, etoxazole, ethofenprox, ethoprophos, etrimfos, emamectin, emamectin-benzoate, endosulfan, empenthrin, oxazosulfyl, oxamyl, oxydemeton-methyl, oxydeprofos (ESP), oxibendazole, oxfendazole, Potassium oleate, sodium oleate, cadusafos, kappa-bifenthrin, cartap, carbaryl, carbosulfan, carbofuran, gamma-cyhalothrin, xylylcarb, quinalphos, kinoprene, chinomethionat, cloethocarb, clothianidin, clofentezine, chromafenozide, chlorantraniliprole, chlorethoxyfos, chlordimeform, chlordane, chlorpyrifos, chlorpyrifos-methyl, chlorphenapyr, chlorfenson, ch1orfenvinphos, chlorfluazuron, chlorobenzilate, chlorobenzoate, chloroprallethrin, Kelthane (dicofol), salithion, cyhalodiamide, cyanophos (CYAP), diafenthiuron, diamidafos, cyantraniliprole, theta-cypermethrin, dienochlor, cyetpyrafen, cyenopyrafen,- dioxabenzofos, diofenolan, sigma-cypermethrin, cyclaniliprole, dichlofenthion (ECP), cycloprothrin, dichlorvos (DDVP), dicloromezotiaz, disulfoton, dinotefuran, cyhalodiamide, cyhalothrin, cyphenothrin, cyfluthrin, diflubenzuron, cyflumetofen, diflovidazin, cyproflanilide, cyhexatin, cypermethrin, dimethylvinphos, dimethoate, dimpropyridaz, dimefluthrin, silafluofen, cyromazine, spidoxamat, spinetoram, spinosad, spirobudifen, spirodiclofen, spirotetramat, spiropidion, spiromesifen, sulfluramid, sulprofos, sulfoxaflor, zeta-cypermethrin, diazinon, tau-fluvalinate, dazomet, thiacloprid, thiamethoxam, tioxazafen, thiodicarb, thiocyclam, thiosultap, thiosultap-sodium, thionazin, thiometon, tiorantraniliprole, deet, dieldrin, tetrachlorantraniliprole, tyclopyrazoflor, tetrach1orvinphos, tetradifon, tetraniliprole, tetramethylfluthrin, tetramethrin, tebupirimfos, tebufenozide, tebufenpyrad, tefluthrin, teflubenzuron, demeton-S-methyl, temephos, deltamethrin, terbufos, doramectin, tralopyril, tralomethrin, transfluthrin, triazamate, triazuron, trichlamide,

trichlorphon (DEP), trifluenfuronate, triflumezopyrium, triflumuron, tolfenpyrad, naled (BRP), nicofluprole, nithiazine, nitenpyram, novaluron, noviflumuron, hydroprene, vaniliprole, vamidothion, parathion, parathion-methyl, halfenprox, halofenozide, bistrifluron, bisultap, hydramethylnon, hydroxy propyl starch, binapacryl, pyflubumide, bifenazate, bifenthrin, pymetrozine, pyraclorfos, pyrafluprole, pyridafenthion, pyridaben, pyridalyl, pyrifluquinazon, pyriprole, pyriproxyfen, pirimicarb, pyrimidifen, pyriminostrobin, pirimiphos-methy1, pyrethrins, fipronil, fenazaquin, fenamiphos, bromopropylate, fenitrothion (MEP), fenoxycarb, fenothiocarb, phenothrin, fenobucarb, fensulfothion, fenthion (MPP), phenthoate (PAP), fenvalerate, fenpyroximate, fenpropathrin, fenbendazole, fenmezoditiaz, fosthiazate, formetanate, butathiofos, buprofezin, furathiocarb, prallethrin, fluacrypyrim, fluazaindolizine, fluazinam, fluazuron, fluensulfone, fluxametamide, fluchlordiniliprole, flucycloxuron, flucythrinate, fluvalinate, flupyradifurone, flufiprole, flupyradifurone, flupyroxystrobin, flupyrazofos, flupyrimin, flufenerim, flufenoxystrobin, flufenoxuron, flufenzine, flufenoprox, fluproxyfen, flubrocythrinate, fluhexafon, flubendiamide, flupentiofenox, flumethrin, flurimfen, prothiofos, protrifenbute, flonicamid, propaphos, propargite (BPPS), profenofos, broflanilide, profluthrin, propoxur (PHC), flometoquin, $\alpha$-alpha-bromadiolone, bromopropylate, beta-cyfluthrin, hexaflumuron, hexythiazox, heptafluthrin, heptenophos, permethrin, benclothiaz, bendiocarb, benzpyrimoxan, bensu1tap, benzoximate, benfuracarb, phoxim, phosalone, fosthiazate, fosthietan, phosphamidon, phosphocarb, phosmet (PMP), polynactins, formetanate, formothion, phorate, machine oil, malathion, milbemycin, milbemycin-A, milbemectin, mecarbam, mesulfenfos, methomyl, metaldehyde, metaflumizone, methamidophos, metam-ammonium, metam-sodium, methiocarb, methidathion (DMTP), methylisothiocyanate, methylneodecanamide, methylparathion, metoxadiazone, methoxychlor, methoxyfenozide, metofluthrin, methoprene, metolcarb, meperfluthrin, mevinphos, monocrotophos, monosultap, momfluorothrin, lambda-cyhalothrin, ryanodine, lufenuron, rescalure, resmethrin, lepimectin, rotenone, levamisol hydrochloride, fenbutatin oxide, morantel tartarate, methyl bromide, cyhexatin, calcium cyanamide, calcium polysulfide, sulfur, nicotine-sulfate, and the like.

Examples of the agrohorticultural fungicide to be used for such purposes include aureofungin, azaconazole, azithiram, acypetacs, acibenzolar, acibenzolar-S-methyl, azoxystrobin, anilazine, amisulbrom, ampropylfos, ametoctradin, allyl alcohol, aldimorph, amobam, isotianil, isovaledione, isopyrazam, isofetamid, isoflucypram, isoprothiolane, ipconazole, ipfentrifluconazole, ipflufenoquin, iprodione, iprovalicarb, iprobenfos, imazalil, iminoctadine, metam, iminoctadine-albesilate, iminoctadine-triacetate, imibenconazole, inpyrfluxam, uniconazole, uniconazole-P, echlomezole, edifenphos, etaconazole, ethaboxam, ethirimol, etem, ethoxyquin, etridiazole, enestroburin, enoxastrobin, epoxiconazole, oxadixyl, oxathiapiprolin, oxycarboxin, copper-8-quinolinolate, oxytetracycline, copper-oxinate, oxpoconazole, oxpoconazole-fumarate, oxolinic acid, octhilinone, ofurace, orysastrobin, carbam (metam-sodium), kasugamycin, carbamorph, carpropamid, carbendazim, carboxin, carvone, quinazamid, quinacetol, quinoxyfen, quinofumelin, chinomethionat, quinomethionate, captafol, captan, kiralaxyl, quinconazole, quintozene, guazatine, cufraneb, cuprobam, coumoxystrobin, glyodin, griseofulvin, climbazole, cresol, kresoxim-methyl, chlozolinate, clotrimazole, chlobenthiazone, chloraniformethan, chloranil, chlorquinox, chloropicrin, chlorfenazole, chloroinconazide, chlorodinitronaphthalene, chlorothalonil, chloroneb, salicylanilide, zarilamid, cyazofamid, diethyl pyrocarbonate, diethofencarb, cyclafuramid, diclocymet, dichlozoline, diclobutrazol, cyclobutrifluram, dichlofluanid, cycloheximide, dichlobentiazox, diclomezine, dicloran, dichlorophen, dichlone, disulfiram, ditalimfos, dithianon, diniconazole, diniconazole-M, zineb, dinocap, dinocton, dinosulfon, dinoterbon, dinobuton, dinopenton, dipymetitrone, dipyrithione, diphenylamine, difenoconazole, cyflufenamid, diflumetorim, cyproconazole, cyprodinil, cyprofuram, cypendazole, simeconazole, dimethirimol, dimethomorph, cymoxanil, dimoxystrobin, ziram, silthiofam, streptomycin, spiroxamine, sultropen, sedaxane, zoxamide, dazomet, thiadiazin, tiadinil, thiadifluor, thiabendazole, tioxymid, thiochlorfenphim, thiophanate, thiophanate-methyl, thifluzamide, thicyofen, thioquinox, thiram, decafentin, tecnazene, tecloftalam, tecoram, tetraconazole, debacarb, dehydroacetic acid, tebuconazole, tebufloquin, dodicin, dodine, dodecylbenzenesulfonic acid bis(ethylenediamine) copper(II) complex salt (DBEDC), dodemorph, drazoxolon, triadimenol, triadimefon, triazbutil, triazoxide, triamiphos, triarimol, trichlamide, triclopyricarb, tricyclazole, triticonazole, tridemorph, tributyltin oxide, triflumizole, trifloxystrobin, triforine, tolylfluanid, tolclofos-methyl, tolprocarb, natamycin, nabam, nitrostyrene, nitrothal-isopropyl, nuarimol, copper nonylphenol sulfonate, halacrinate, validamycin, valifenalate, harpin protein, picarbutrazox, bixafen, picoxystrobin, picobenzamide, pydiflumetofen, bithionol, bitertanol, hydroxyisoxazole, hydroisoxazole-potassium, binapacryl, biphenyl, piperalin, hymexazol, pyraoxystrobin, pyracarbolid, pyraclostrobin, pyraziflumid, pyrazophos, pyrapropoyne, pyrametostrobin, pyriofenone, pyridinitril, pydiflumetofen, pyrisoxazole, pyridachlometyl, pyrifenox, pyribencarb, pyriminostrobin, pyrimethanil, pyroxychlor, pyroxyfur, pyroquilon, vinclozolin, ferbam, famoxadone, fenapanil, fenamidone, fenaminosulf, fenaminstrobin, fenarimol, fenitropan, fenoxanil, ferimzone, ferbam, fentin, fenpiclonil, fenpicoxamid, fenpyrazamine, fenbuconazole, fenfuram, fenpropidin, fenpropimorph, fenhexamid, phthalide, buthiobate, butylamine, bupirimate, fuberidazole, blasticidin-S, furametpyr, furalaxyl, fluacrypyrim, fluazinam, fluindapyr, fluoxastrobin, fluoxapiprolin, fluoxytioconazole, fluotrimazole, fluopicolide, Fluopimomide, fluopyram, fluoroimide, furcarbanil, fluxapyroxad, fluquinconazole, furconazole, furconazole-cis, fludioxonil, flusilazole, flusulfamide, flutianil, flutolanil, flutriafol, flufenoxadiazam, flufenoxystrobin, furfural, flubeneteram, furmecyclox, flumetylsulforim, flumetover, flumorph, proquinazid, prochloraz, procymidone, prothiocarb, prothioconazole, pronitridine, propamocarb, propiconazole, propineb, furophanate, probenazole, bromuconazole, florylpicoxamid, hexachlorobutadiene, hexaconazole, hexylthiofos, bethoxazin, benalaxyl, benalaxyl-M, benodanil, benomyl, pefurazoate, benquinox, penconazole, benzamorf, pencycuron, benzohydroxamic acid, benzovindiflupyr, bentaluron, benthiazole, benthiavalicarb, benthiavalicarb-isopropyl, penthiopyrad, penflufen, boscalid, phosdiphen, fosetyl, fosetyl-Al, polyoxins, polyoxorim, polycarbamate, folpet, formaldehyde, machine oil, maneb, mancozeb, mandipropamid, mandestrobin, myclozolin, myclobutanil, mildiomycin, milneb, mecarbinzid, methasulfocarb, metazoxolon, metam, metam-sodium, metalaxyl, metalaxyl-M, metarylpicoxamid, metiram, methyl isothiocyanate, mepthyldinocap, Metyltetraprole, metconazole, metsulfovax, methfuroxam, metominostrobin, metrafenone, mepanipyrim, mefenoxam, mefentrifluconazole, meptyldinocap, mepronil, mebenil, iodomethane, rabenzazole, methyl bromide, benzalkonium chloride, inorganic antimicrobial agents such as basic copper chloride, basic copper sulfate, silver, and the like, sodium hypochlorote, cupric hydroxide, wettable sulfur, calcium polysulfide, potassium hydrogen carbonate, sodium hydrogen carbonate, sulfur, copper sulfate anhydride, nickel dimethyldithiocarbamate, copper compounds such as copper 8-hydroxyquinoline (oxine copper) and the like, zinc sulfate, copper sulfate pentahydrate, and the like.

Similarly, examples of the herbicide include 1-naphthylacetamide, 2,4-PA, 2,3,6-TBA, 2,4,5-T, 2,4,5-TB, 2,4-D, 2,4-DB, 2,4-DEB, 2,4-DEP, 3,4-DA, 3,4-DB, 3,4-DP, 4-CPA, 4-CPB, 4-CPP, MCP, MCPA, MCPA thioethyl, MCPB, ioxynil, aclonifen, azafenidin, acifluorfen, aziprotryne, azimsulfuron, asulam, acetochlor, atrazine, atraton, anisuron, anilofos, aviglycine, abscisic acid, amicarbazone, amidosulfuron, amitrole, aminocyclopyrachlor, aminopyralid, amibuzin, amiprophos-methyl, ametridione, ametryn, alachlor, allidochlor, alloxydim, alorac, iofensulfuron, isouron, isocarbamid, isoxachlortole, isoxapyrifop, isoxaflutole, isoxaben, isocil, isonoruron, isoproturon, isopropalin, isopolinate, isomethiozin, inabenfide, ipazine, iptriazopyrid, ipfencarbazone, iprymidam, imazaquin, imazapic, imazapyr, imazamethapyr, imazamethabenz, imazamethabenz-methyl, imazamox, imazethapyr, imazosulfuron, indaziflam, indanofan, indolebutyric acid, uniconazole-P, eglinazine, esprocarb, ethametsulfuron, ethametsulfuron-methyl, ethalfluralin, ethiolate, ethychlozate-ethyl, ethidimuron, etinofen, ethephon, ethoxysulfuron, ethoxyfen, etnipromid, ethofumesate, etobenzanid, epyrifenacil, epronaz, erbon, endothal, oxadiazon, oxadiargyl, oxaziclomefone, oxasulfuron, oxapyrazon, oxyfluorfen, oryzalin, orthosulfamuron, orbencarb, cafenstrole, cambendichlor, carbasulam, carfentrazone, carfentrazone-ethyl, karbutilate, carbetamide, carboxazole, quizalofop, .quizalofop-P, quizalofop-ethyl, xylachlor, quinoclamine, quinonamid, quinclorac, quinmerac, cumyluron, clacyfos, cliodinate, glyphosate, glufosinate, glufosinate-P, credazine, clethodim, cloxyfonac, clodinafop, clodinafop-propargyl, chlorotoluron, clopyralid, cloproxydim, cloprop, chlorbromuron, clofop, clomazone, chlomethoxyni1, chlomethoxyfen, clomeprop, chlorazifop, chlorazine, chloranocryl, chloramben, cloransulam, cloransulam-methyl, chloridazon, chlorimuron, chlorimuron-ethyl, chlorsulfuron, chlorthal, chlorthiamid, chlortoluron, chlornitrofen, chlorfenac, chlorfenprop, chlorbufam, chlorphthalim, chlorflurazole, chlorflurenol, chlorprocarb, chlorpropham, chlormequat, chloreturon, chloroxynil, chloroxuron, chlorotoluron, chloropon, saflufenacil, cyanazine, cyanatryn, di-allate, diuron, diethamquat, dioxopyritrione, dicamba, cycluron, cycloate, cycloxydim, diclosulam, cyclosulfamuron, cyclopyranil, cyclopyrimorate, dichlorprop, dichlorprop-P, dichlobenil, diclofop, diclofop-methyl, dichlormate, dichloralurea, diquat, cisanilide, disul, siduron, dithiopyr, dinitramine, cinidon-ethyl, dinosam, cinosulfuron, dinoseb, dinoterb, dinofenate, dinoprop, cyhalofop-butyl, cypyrafluone, diphenamid, difenoxuron, difenopenten, difenzoquat, cybutryne, cyprazine, cyprazole, diflufenican, diflufenzopyr, dipropetryn, cypromid, cyperquat, gibberellin, simazine, dimexano, dimesulfazet, dimethachlor, dimidazon, dimethametryn, dimethenamid, simetryn, simeton, dimepiperate, dimefuron, cinmethylin, swep, sulglycapin, sulcotrione, sulfallate, sulfentrazone, sulfosulfuron, sulfometuron, sulfometuron-methyl, secbumeton, sethoxydim, sebuthylazine, terbacil, daimuron, dazomet, dalapon, thiazafluron, thiazopyr, tiafenacil, thiencarbazone, thiencarbazone-methyl, tiocarbazil, tioclorim, thiobencarb, thidiazimin, thidiazuron, thifensulfuron, thifensulfuron-methyl, desmedipham, desmetryn, tetflupyrolimet, tetrafluron, thenylchlor, tebutam, tebuthiuron, terbumeton, tepraloxydim, tefuryltrione, tembotrione, delachlor, terbacil, terbucarb, terbuchlor, terbuthylazine, terbutryn, topramezone, tralkoxydim, triaziflam, triasulfuron, triafamone, tri-allate, trietazine, tricamba, triclopyr, tridiphane, tritac, tritosulfuron, tripyrasulfone, trifludimoxazin, triflusulfuron, triflusulfuron-methyl, trifluralin, trifloxysulfuron, tripropindan, tribenuron, tribenuron-methyl, trifop, trifopsime, trimeturon, tolpyralate, naptalam, naproanilide, napropamide, nicosulfuron, nitralin, nitrofen, nitrofluorfen, nipyraclofen, neburon, norflurazon, noruron, barban, paclobutrazol, paraquat, parafluron, haloxydine, halauxifen, haloxyfop, haloxyfop-P, haloxyfop-methyl, halosafen, halosulfuron, halosulfuron-methyl, bilanafos, bixlozone, picloram, picolinafen, bicyclopyrone, bispyribac, bispyribac-sodium, pydanon, pinoxaden, bipyrazone, bifenox, piperophos, hymexazol, pyraclonil, pyrasulfotole, pyrazoxyfen, pyrazosulfuron, pyrazosulfuron-ethyl, pyrazolate, bilanafos, pyraflufen, pyraflufen-ethyl, pyriclor, pyridafol, pyrithiobac, pyrithiobac-sodium, pyridate, pyriftalid, pyributicarb, pyriflubenzoxim, pyribenzoxim, pyrimisulfan, primisulfuron, pyriminobac-methyl, pyroxasulfone, pyroxsulam, fenasulam, phenisopham, fenuron, fenoxasulfone, fenoxaprop, fenoxaprop-P, fenoxaprop-ethyl, phenothio1, fenoprop, phenobenzuron, fenquinotrione, fenthiaprop, fenteracol, fentrazamide, fenpyrazone, phenmedipham, phenmedipham-ethyl, butachlor, butafenacil, butamifos, buthiuron, buthidazole, butylate, buturon, butenachlor, butroxydim, butralin, butroxydim, flazasulfuron, flamprop, furyloxyfen, prynachlor, primisulfuron-methyl, fluazifop, fluazifop-P, fluazifop-butyl, fluazolate, fluroxypyr, fluothiuron, fluometuron, fluoroglycofen, flurochloridone, fluorodifen, fluoronitrofen, fluoromidine, flucarbazone, flucarbazone-sodium, fluchloraminopyr, fluchloraminopyr-tefuryl, fluchloralin, flucetosulfuron, fluthiacet, fluthiacet-methyl, flusulfinam, flupyrsulfuron, flufenacet, flufenican, flufenoximacil, flufenpyr, flupropacil, flupropanate, flupoxam, flumioxazin, flumiclorac, flumiclorac-pentyl, flumipropyn, flumezin, fluometuron, flumetsulam, fluridone, flurtamone, fluroxypyr, pretilachlor, proxan, proglinazine, procyazine, prodiamine, prosulfalin, prosulfuron, prosulfocarb, propaquizafop, propachlor, propazine, propanil, propyzamide, propisochlor, prohydrojasmon, propyrisulfuron, propham, profluazol, profluralin, prohexadione-calcium, propoxycarbazone, propoxycarbazone-sodium, profoxydim, bromacil, brompyrazon, prometryn, prometon, bromoxynil, bromofenoxim, bromobutide, bromobonil, florasulam, florpyrauxifen, hexachloroacetone, hexazinone, pethoxamid, benazolin, penoxsulam, pebulate, beflubutamid, beflubutamid-M, vernolate, perfluidone, bencarbazone, benquitrione, benzadox, benzipram, benzylaminopurine, benzthiazuron, benzfendizone, bensulide, bensulfuron-methyl, benzoylprop, benzobicyclon, benzofenap, benzofluor, bentazone, pentanochlor, benthiocarb, pendimethalin, pentoxazone, benfluralin, benfuresate, fosamine, fomesafen, foramsulfuron, forchlorfenuron, maleic hydrazide, mecoprop, mecoprop-P, medinoterb, mesosulfuron, mesosulfuron-methyl, mesotrione, mesoprazine, methoprotryne, metazachlor, methazole, metazosulfuron, methabenzthiazuron, metamitron, metamifop, metam, methalpropalin, methiuron, methiozolin, methiobencarb, methyldymron, metoxuron, metosulam, metsulfuron, metsu1furon-methy1, metflurazon, metobromuron, metobenzuron, methometon, metolachlor, metribuzin, mepiquatchloride, mefenacet, mefluidide, monalide, monisouron, monuron, monochloroacetic acid, monolinuron, molinate, morfamquat, iodosulfuron, iodosulfuron-methyl-sodium, iodobonil, iodomethane, lactofen, lancotrione, linuron, rimisoxafen, rimsulfuron, lenacil, rhodethanil, calcium peroxide, methyl bromide, and the like.

The same effect as above can be expected by the use in admixture with biological control agents, for example, viral formulations obtained from Nuclear polyhedrosis virus (NPV), Granulosis virus (GV), Cytoplasmic polyhedrosis virus (CPV), Entomopoxi virus (EPV), and the like, microbial pesticides utilized as insecticides or nematicides, such as Monacrosporium phymatophagum, Steinernema carpocapsae, Steinernema kushidai, Pasteuria penetrans, and the like, microbial pesticides utilized as fungicides, such as Trichoderma lignorum, Agrobacterium radiobactor, nonpathogenic Erwinia carotovora, Bacillus subtilis, and the like, biological control agents utilized as herbicides, such as Xanthomonas campestris and the like, and the like.

Furthermore, it is also possible to use biological control agents in combination, for example, natural enemies such as Encarsia formosa, Aphidius colemani, Aphidoletes aphidimyza, Diglyphus isaea, Dacnusa sibirica, Phytoseiulus persimilis, Amblyseius cucumeris, Orius sauteri, and the like, microbial pesticides such as Beauveria brongniartii and the like, or pheromones such as .(Z)-10-tetradecenyl acetate, (E,Z)-4,10-tetradecadieniel acetate, (Z)-8-dodecenyl acetate, (Z)-11-tetradecenyl acetate, (Z)-13-icosen-10-one, 14-methyl-1-octadecene, and the like.

Furthermore, the compound represented by the formula (1) or a salt thereof of the present invention is also suitable for controlling parasites in animals. Animal parasites live in the body or on the body surface of host animals and cause disadvantages to the host animals. They are broadly divided into ectoparasites that live on the body surface of the host animal and endoparasites that live inside the body of the host animal. The compound represented by the formula (1) or a salt thereof of the present invention can also be applied as an ectoparasite control agent for animals or an endoparasite control agent for animals.

When the compound represented by the formula (1) or a salt thereof of the present invention is applied as an ectoparasite control agent for animals or endoparasite control agent for animals, examples of the animal to which it is applicable include, but are not limited to, farm animals such as pig, horse, cows sheep, goat, rabbit, camel, buffalo, deer, mink, chinchillas, and the like, pet animals such as dog, cat, small bird, monkey, and the like, laboratory animals such as rat, mouse, golden hamster, guinea pig, and the like, poultry such as chicken, wild duck, aigamo duck, quail, duck, goose, turkey, and the like, human, and the like. In addition, it is also applicable to farmed fish such as sea bream, flounder, tuna, yellowtail, eel, and the like, crustaceans and shellfish such as shrimp, crab, scallop, oyster, Japanese littleneck, hard clam, and the like.

Examples of the ectoparasite that can be controlled by the ectoparasite control agent for animals of the present invention include, but are not limited to, Rhipicephalus (Rhipicaphalus (Boophilus) microplus, Rhipicephalus sanguineus, etc.), Amblyomma, Dermacentor, Haemaphysalis, Hyalomma, Ixodes, Rhipicentor, Margaropus, Argas, Otobius, Ornithodoros, Chorioptes (Chorioptes bovis etc.), Psoroptes (Psoroptes ovis, etc.), Cheyletiella, Dermanyssus (Dermanyssus gallinae etc.), Ortnithonyssus, Demodex (Demodex canis etc.), Sarcoptes (Sarcoptes scabiei etc.), Psorergates, Siphonaptera (Ctenocephalides felis, Ctenocephalides canis etc.), Diptera (Musca spp, Gasterophilus intestinalis, Oestrus ovis etc.), Phthiraptera (Bovicola Ovis, Bovicola Bovis etc.), Lepidoptera, Coleoptera, Homoptera, Haematopota, Tabunus, Haematobia (Haematobia irritans etc.), Stomoxys, Lucilia, Lepeophtheirus, and the like.

Further specifically, examples of the Acari include Ixodoidea such as Boophilus microplus, Rhipicephalus sanguineus, Haemaphysalis longicornis, Haemaphysalis flava, Haemaphysaliscampanulata, Haemaphysalis concinna, Haemaphysalisjaponica, Haemaphysalis kitaokai, Haemaphysalis ias, Ixodes ovatus, Ixodes nipponensis, Ixodespersulcatus, Amblyommatestudinarium, Haemaphysalis megaspinosa, Dermacentor reticulatus, and Dermacentor taiwanesis, Dermanyssus gallinae, Ornithonyssus such as Ornithonyssus sylviarum, and Ornithonyssus bursa, Trombicula such as Eutrombicula wichmanni, Leptotrombidium akamushi, Leptotrombidium pallidum, Leptotrombidium fuji, Leptotrombidium tosa, Neotrombicula autumnalis, Eutrombicula alfreddugesi, andHelenicula miyagawai, Cheyletidae such as Cheyletiella yasguri, Cheyletiella parasitivorax, and Cheyletiella blakei, Psoroptes cuniculi, Chorioptes bovis, Otodectes cynotis, Sarcoptes such as Sarcoptes scabiei, and Notoedres cati, Demodicidae such as Demodex canis, and

the like.

Examples of the Siphonaptera include Pulicidae, Ceratephyllus, and the like. Examples of the flea belonging to Pulicidae include Ctenocephalidescanis, Ctenocephalides felis, Pulex irritans, Echidnophaga gallinacea, Xenopsylla cheopis, Leptopsylla segnis, Nosopsyllus fasciatus, Monopsyllus anisus, and the like.

In addition, ectoparasites that can be controlled by the ectoparasite control agent for animals of the present invention include Anoplura such as Haematopinus eurysternus, Haematopinus asini, Dalmalinia ovis, Linognathus vituli, Haematopinus suis, Phthirus pubis, and Pediculus capitis, biting lices such as Trichodectes canis, blood-sucking dipterous insect pests such as Tabanus trigonus, Culicoides schultzei, Simulium ornatum, and the like.

The outside for animals of the present invention can be used by blending, according to the preparation formulation generally used, with a suitable solid carrier or liquid carrier, as well as adjuvant and the like as necessary, at suitable proportions, and preparing same into a suitable dosage form such as liquid, emulsifier, cream, ointment, suspension, or aerosol according to the purpose of use by dissolving, suspending, mixing, impregnating, adsorbing or attaching. The amount of the active ingredient in these preparations may be in the range of from 0.01 to 80.0 parts by weight per 100 parts by weight of the preparation. As the solid carrier or liquid carrier to be used, carriers commonly used for veterinary drugs may be used, and liquid carriers are preferably used in view of the easiness of treatment on target animals. Examples of the liquid carrier include alcohols such as methyl alcohol, ethyl alcohol, isopropyl alcohol, tertiary-butyl alcohol, benzyl alcohol, and the like, propylene carbonate, N-methyl-2-pyrrolidone, water, and the like. Where necessary, an adjuvant can be used, and as the adjuvant, surfactant, antioxidant, emulsifier, and the like can be used. For example, surfactants such as polyoxyethylene alkylaryl ether, polyoxyethylene sorbitan monolaurate, alkylallylsorbitan monolaurate, alkylbenzenesulfonate, alkylnaphthalenesulfonic acid, lignin sulfonate higher alcohol sulfate ester salt, glycol monoalkyl ether, glycols, and the like, emulsifiers such as sorbitan monoeulenate, sorbitan monolaurate, monoglyceride caprylate, monoglyceride caprate, monoglyceride isostearate, propylene glycol monocaprylate, and the like, and antioxidants such as BHA, BHT, and the like can be mentioned.

The ectoparasite control agent for animals of the present invention may be administered to animals by a route that affords the desired effect, such as topical, oral, parenteral, or subcutaneous route, and is not particularly limited, but topical administration is preferred. Topical administration includes administration by a spot-on treatment in which a liquid drug is dropped onto the skin of the dorsal scapula of the target animal, a pour-on treatment in which a liquid drug is applied along the mid-dorsal line of the target animal, spray-on and spray lace in which a liquid aerosol drug is used for treatment by spray or the like, medicinal bath (dip), a treatment method using an ear tag or a collar carrying a drug. The application dose for the treatment can be appropriately selected from the range of generally about 0.1 to 500 mg, in terms of the compound as an active ingredient, and generally about 0.01 to 20 ml, in terms of the control agent of the present invention, per 1 kg of body weight of the target animal.

Where necessary, other active ingredients can be used in combination. Examples thereof include afoxolaner, fluralaner, lotilaner, surolaner, mivorilaner, modoflaner, tigolaner, umifoxolaner, albendazole, cambendazole, fenbendazole, flubendazole, mebendazole, oxifendazole, parabendazole, thiabendazole, triclabendazole, amitraz, demiditraz, clorsulon, closantel, oxyclonazide, lafoxanide, cyphenothrin, flumethrin, permethrin, cyromazine, derquantel, diamphenetide, dicyclanil, dinotefuran, imidacloprid, nitenpyram, thiamethoxam, abamectin, doramectin, emamectin, eprinomectin, ivermectin, moxidectin, selamectin, milbemycin oxime, emodepside, epsiplantel, fipronil, fluazuron, fluhexafon, indoxacarb, levamisole, lufenuron, metaflumizone, methoprene, monepantel, morantel, niclosamide, nitroscanate, nitroxinil, novaluron, oxantel, praziquantel, pyrantel, pyriprole, pyriproxyfen, cisapronil, spinosad, spinetoram, triflumezopyrim, and the like. When used in combination, a preparation of two or more kinds of active ingredients mixed together before administration may be used, or two or more different preparations may be administered separately.

The endoparasites that can be controlled by the endoparasite control agent for animals of the present invention are largely classified into Protozoa and Helminth. Examples of Protozoa that parasitize humans include, but are not limited to, amebas (Rhizopoda) such as Entamoeba and the like, Mastigophora such as Leishmania, Trypanosoma, Trichomonas, and the like, Sporozoea such as Plasmodium, Toxoplasma, and the like, and Ciliophora such as Balantidium coli and the like, and examples of Helminth that parasitizes humans include Nematoda such as roundworm, anisakis, dog roundworm, hairworm, pinworm, hookworm (Zubini hookworm, American hookworm, Brazilian hookworm, etc.), Schistosoma, Gnathostom, Brugia (filaria, Bancroft filaria, Brugia malayi, etc.), onchocerca, medina worm, Trichinella, Strongyloides, and the like, Acannthocephala such as Macracanthorhynchus hirudinaceus, Gordiacea such as hairworm and the like, Hirudinea such as Hirudo nipponia and the like, Trematoda such as Schistosoma japonicu, Schistosoma mansoni, Schistosoma haematobium, Clonorchis sinensis, Heterophyes, Fasciolidae, Paragonimus, and the like, and Cestoda such as Diphyllobothrium latum, Spirometra mansoni, Sparganum proliferum, Diplogonoporus, Taenia, (Taeniarhynchus, Taenia solium, hydatid (Echinococcus) etc.), MOYO JOCHU, Dipylidium caninum, Mesocestoides, monkey Anoplocephala, Nybelinia surmenicola, and the like.

Protozoa that parasitize animals belonging to mammals other than human, or birds are exemplified by, but not limited to, Apicomplexa including Cocidia such as Eimeria, Isospora, Toxoplasma, Neospora, Sarcocystis, Besnoitia, Hammondia, Cryptosporidium, Caryospora, and the like, Haemosporina such as Leucocytozoon, Plasmodium, and the

like, Piroplasma such as Theileria, Anaplasma, Eperythrozoon, Heamobartonella, Rhrlichia, and the like, and the like, other Apicomplexa including Hepatozoon, Haemogregarina, and the like, Microspora including Encephalitozoon, Nosema, and the like, Mastigophora including Trypanosomatid such as Trypanosoma, Leishmania, and the like, Trichomonadida such as Chilomastix, Trichomonas, Monocercomonas, Histomonas, and the like, Diplomonadida such as Hexamita, Giardia, and the like, Sarcodina including amebas such as Entamoeba and the like, and the like, Ciliophora including Balantidium, Buxtonella, Entodinium, and the like,

Helminth that parasitizes animals belonging to mammals other than human, or birds is exemplified by Nematoda including Ascarida such as Ascaris, Toxocara, Toxascaris, Parascaris, Ascaridia, Heterakis, Anisakis., and the like, Oxyurida such as Oxyuris, Passalurus, and the like, Strongylida such as Strongylus, Haemonchus, Ostertagia, Trichostrongylus, Cooperia, Nematodirus, Hystrongylus, Oesophagostomum, Chabertia, Ancylostoma, Uncinaria, Necator, Bunostomum, Dictyocaulus, Metastrongylus, Filaroides, Aelurostrongulus, Angiostrongylus, Syngamus, Stephanurus, and the like, Rhabditida such as Strongyloides, Micronema, and the like, Spirurida such as Thelazia, Oxyspirura, Spirocerca, Gongylonema, Draschia, Habronema, Ascarops, Physaloptera, Gnathostoma, and the like, Filariida such as Dirofilaria, Setaria, Dipetalonema, Parafilaria, Onchocerca, and the like, Enoplida such as Parafilaria, Stephanofilaria, Trichuris, Capillaria, Trichosomoides, Trichinella, Dioctophyma, and the like,

Trematoda including Fasciolata such as Fasciola, Fasciolopsis, and the like, Paramphistomatidae such as Homalogaster and the like, Dicrocoelata such as Eurytrema, Dicrocoelium, and the like, Diplostomata such as Pharyngostomum, Alaria, and the like, Echinostomata such as Echinostoma, Echinochasmus, and the like, Troglotrematoidea such as Paragonimus, Nanophyetus, and the like, Opisthorchiida such as Clonorchis and the like, Heterophyida such as Heterophyes, Metagonimus, and the like, Plagiorchiida such as Prosthogonimus and the like, Schistosoma such as Schistoma and the like, and the like, Cestoda including Pseudophylidea such as Diphyllobothrium, Spirometra, and the like, Cyclophyllidea such as Anoplocephara, Paranoplocephala, Moniezia, Dipylidium, Mesocestoides, Taenia pisiformis, Tenia, Hydatigera, Multiceps, Echinococcus, Echinococcus, Taenia, Taeniarhynchus, Hymenolepis, Vampirolepis, Raillietina, Amoebotaenia, and the like, and the like, Acannthocephala including Macracanthorhynchus, Moniliformis, and the like, Linguatulida including Linguatula and the like, and various other parasites.

Helminth is exemplified by, but not limited to, other names of Enoplida of Nematoda including Trichuris spp., Capillaria spp., Trichomosoides spp., Trichinella spp., and the like, Rhabditia including Micronema spp., Strongyloides spp., and the like, Strongylida including Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp. and the like,

Oxyurida including Oxyuris spp., Enterobius spp., Passalurus spp., Syphacia spp., Aspiculuris spp., Heterakis spp., and the like, Ascaridia including Ascaris spp., Toxascaris spp., Toxocara spp., Parascaris spp., Anisakis spp., Ascaridia spp., and the like, Spiruride including Gnathosma spp., Physaloptera spp., Thelazia spp., Gongylonema spp., Habronema spp., Parabronema spp., Draschia spp., Dracunculus spp., and the like, Filariida including Stephanofilaria spp., Parafilaria spp., Setaria spp., Loa spp., Dirofilaria spp., Litomosoides spp., Brugia spp., Wuchereria spp., Onchocerca spp., and the like,

Acanthocephala including Filicollis spp., Moniliformis spp., Macracanthorhynchus spp., Prosthenorchis spp., and the like, Monogenea subclass of Trematoda including Gyrodactylus spp., Dactylogyrus spp., Polystoma spp., and thelike, Digenea subclass including Diplostomum spp., Posthodiplostomum spp., Schistosoma spp., Trichobilharzia spp., Ornithbilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantcotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonimus spp., and the like,

Pseudophyllidea.including Diphyllobothrium spp., Spirometra spp., Schistocephalus spp., Ligula spp., Bothridium spp., and Diplogonoporus spp., and Cyclophyllidea including parasites belonging to species such as Mesocestoides spp., Anoplocephala spp., Paranoplocehala spp., Moniezia spp., Thysanosomsa spp., Thysaniezia spp., Avitellina spp., Stilesia spp., Cittotaenia spp., Andyra spp., Bertiella spp., taenia spp., Echinococcus spp., Hydatigera spp., Davainea spp., Raillietina spp., MOYO JOCHU spp. (Hymenolepis spp.), Echinolepis spp., Echinocotyle spp., Diorchis spp., Dipylidium spp., Joyeuxiella spp., Diplopylidium spp., and the like, and various other parasites belonging to Acannthocephala or Linguatulida.

The endoparasite control agent for animals of the present invention is effective in controlling not only parasites

living in the bodies of intermediate hosts and final hosts, but also parasites in the living bodies of reservoir hosts. Furthermore, the compound represented by the formula (1) of the present invention exhibits a control effect on parasites at all developmental stages. For example, Protozoa include cyst, precystic form, vegetative form, or asexually reproductive stage meristem, ameboid form, sexual reproductive stage gametocyte, gamete, fusant, sporophyte, and the like. Nematoda include egg, larva, and adult. Furthermore, the compound of the present invention not only exterminates parasites within a living body, but also prevents parasite infection when applied to the environment, which is a route of infection. For example, soil-borne infection from soils in fields and parks, percutaneous infection from water systems such as river, lake, swamp, rice paddy, and the like, oral infection from the feces of animals such as dog, cat, and the like, oral infection from raw meat of saltwater fish, freshwater fish, crustaceans, shellfish, livestock, and the like, infection from mosquito, horsefly, fly, cockroach, tick, flea, louse, assassin bug, chigger, and the like, and the like can be prevented.

The endoparasite control agent for animals of the present invention can be administered to humans, mammals other than human, or birds as a pharmaceutical product for the purpose of treating or preventing parasitic diseases. The administration method can be either oral administration or parenteral administration. In the case of oral administration, it can be administered by incorporating into capsule, tablet, pill, dust, granule, fine granule, powder, syrup, enteric tablet, suspension, paste or veterinary liquid drink or feed. In the case of parenteral administration, it is administered in a dosage form that maintains mucosa or transdermal absorption, such as injection, drip infusion, suppository, emulsion, suspension, drop, ointment, cream, liquid, lotion, spray, aerosol, cataplasm, tape.

When the endoparasite control agent for animals of the present invention is used as a pharmaceutical product for animals belonging to human, mammals other than human, or birds, the most appropriate amount (effective amount) of the active ingredient varies depending on whether it is for treatment or prevention, type of infected parasite, type and degree of infection, dosage form and the like. Generally, in the case of oral administration, it is within the range of about 0.0001 to 10000 mg/kg body weight per day. In the case of parenteral administration, it is within the range of about 0.0001 to 10000 mg/kg body weight per day, which is administered in a single dose or in divided doses.

The concentration of the active ingredient in the endoparasite control agent for animals of the present invention is generally about 0.001 to 100% by mass, preferably about 0.001 to 99%, further preferably about 0.005 to 20% by mass. The endoparasite control agent for animals of the present invention may be a composition to be administered as it is, or may be a highly concentrated composition that is diluted to an appropriate concentration before when in use.

In addition, other active ingredients described above can also be used in combination as necessary for the purpose of reinforcing or supplementing the effects of the endoparasite control agent for animals of the present invention. When used in combination, a preparation obtained by mixing two or more kinds of active ingredients.before administration may be used, or two or more kinds of different preparations may be administered separately.

Production Example 1

Production of N-(2-fluorophenyl)-5-hydroxy-3-oxo-4'-(trifluoromethyl)-1,2,3,6-tetrahydro-[1,1'-biphenyl]-4-carboxamide (compound No. 1-38)

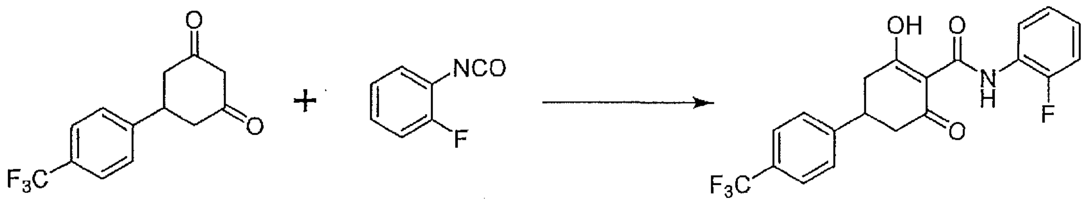

To 5-(4-(trifluoromethyl)phenyl)cyclohexane-1,3-dione (200 mg, 0.78 mmol), 2-fluorophenylisocyanate (160 mg, 1.17 mmol), and N,N-dimethylacetamide (5 mL) was added 60 wt% sodium hydride (40.6 mg, 1.01 mmol), and the mixture was stirred at room temperature for 2 hr. After completion of the reaction, the reaction mixture was poured into 1N hydrochloric acid, and the mixture was extracted with ethyl acetate and washed with water. The obtained organic layer was dried over anhydrous magnesium sulfate and concentrated under reduced pressure. The obtained residue was purified by silica gel column chromatography to give N-(2-fluorophenyl)-5-hydroxy-3-oxo-4'-(trifluoromethyl)-1,2,3,6-tetrahydro-[1,1'-biphenyl]-4-carboxamide (44.0 mg, 0.11 mmol),

yield: 14%
physical property: melting point 233-236°C

Production Example 2

Production of N-(5-fluoropyridin-2-yl)-5-hydroxy-3-oxo-4'-(trifluoromethyl)-1,2,3,6-tetrahydro-[1,1'-biphenyl]-4-carboxamide (compound No. 1-48)

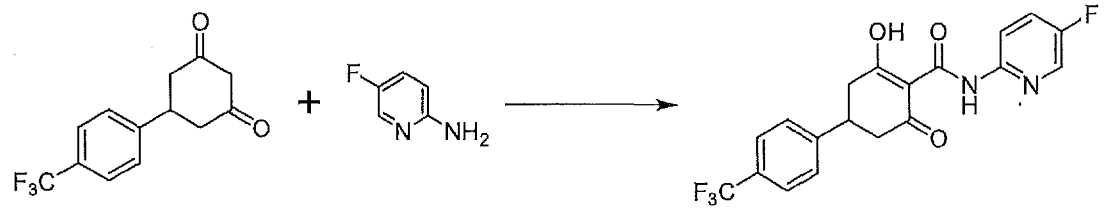

5-Fluoropyridine-2-amine (220 mg, 2.34 mmol), carbodiimidazole (379 mg, 2.34 mmol), and N,N-dimethylacetamide (6 mL) were stirred at 75°C for 1 hr 30 min. Then, 5-(4-(trifluoromethyl)phenyl)cyclohexane-1,3-dione (400 mg, 1.56 mmol) was added, and the mixture was stirred for 2 hr 30 min at room temperature. The reaction solution was poured into 1N hydrochloric acid, and the mixture was extracted with ethyl acetate and washed with water. The obtained organic layer was dried over anhydrous magnesium sulfate and concentrated under reduced pressure. Silica gel column chromatography gave N-(5-fluoropyridin-2-yl)-5-hydroxy-3-oxo-4'-(trifluoromethyl)-1,2,3,6-tetrahydro-[1,1'-biphenyl]-4-carboxamide (40.0 mg, 0.0880 mmol),

yield: 6%
physical property: melting point 188-190°C

Production Example 3

Production of 5-hydroxy-3-oxo-N-(thiazol-2-yl)-4'-(trifluoromethyl)-1,2,3,6-tetrahydro-[1,1'-biphenyl]-4-carboxamide (compound No. 1-47)

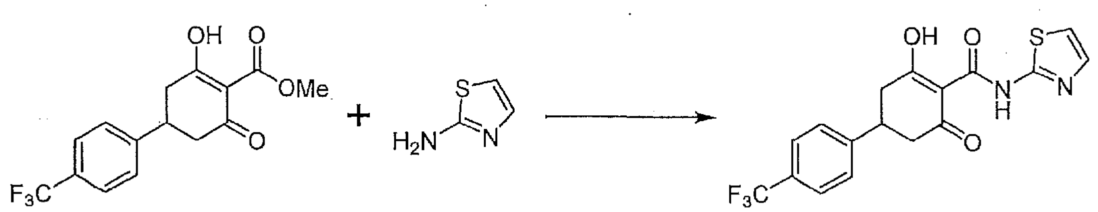

To methyl 5-hydroxy-3-oxo-4'-(trifluoromethyl)-1,2,3,6-tetrahydro-[1,1'-biphenyl]-4-carboxylate (0.218 g, 0.669 mmol) and 2-aminothiazole (0.113 g, 1.13 mmol) was added toluene (7 mL) and the mixture was stirred under refluxing for 2 hr. After completion of the reaction, the reaction solution was poured into 1N hydrochloric acid, and the mixture was extracted with ethyl acetate and washed with water. The obtained organic layer was dried over anhydrous magnesium sulfate and concentrated under reduced pressure. The obtained residue was purified by silica gel column chromatography to give 5-hydroxy-3-oxo-N-(thiazol-2-yl)-4'-(trifluoromethyl)-1,2,3,6-tetrahydro-[1,1'-biphenyl]-4-carboxamide (36 mg, 0.095 mmol).

yield: 14%
physical property: melting point 249-250°C

Production Example 4

Production of N-(4-fluorophenyl)-5-(methoxyamino)-3-oxo-4'-(trifluoromethyl)-1,2,3,6-tetrahydro-[1,1'-biphenyl]-4-carboxamide (compound No. 1-227)

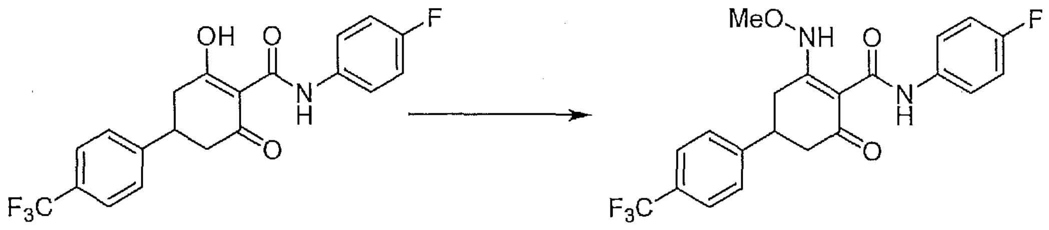

N-(4-fluorophenyl)-5-hydroxy-3-oxo-4'-(trifluoromethyl)-1,2,3,6-tetrahydro-[1,1'-biphenyl]-4-carboxamide (0.10 g, 0.25 mmol), chloroform (1 mL), N,N-dimethylformamide DMF one drop, and oxalyl chloride (50 mg, 0.38 mmol) were stirred at room temperature for 4 hr. The solvent was evaporated under reduced pressure. To the residue were successively added O-methylhydroxylamine hydrochloride (40 mg, 0.50 mmol), ethanol (2 mL), and triethylamine (0.12 g, 1.3 mmol), and the mixture was stirred at room temperature overnight. After completion of the reaction, the mixture was partitioned between ethyl acetate and brine. The organic layer was dried over anhydrous sodium sulfate and filtered. The solvent was evaporated under reduced pressure. The residue was purified by silica gel column chromatography to give N-(4-fluorophenyl)-5-(methoxyamino)-3-oxo-4'-(trifluoromethyl)-1,2,3,6-tetrahydro-[1,1'-biphenyl]-4-carboxamide (40 mg, 0.14 mmol).

yield: 37%
physical property: melting point 130-131°C

Reference Example 1

Production of methyl 5-hydroxy-3-oxo-4'-(trifluoromethyl)-1,2,3,6-tetrahydro-[1,1'-biphenyl]-4-carboxylate

Reference Example 1-1

Production of methyl (5-oxo-4'-(trifluoromethyl)-1,2,5,6-tetrahydro-[1,1'-biphenyl]-3-yl)carbonate

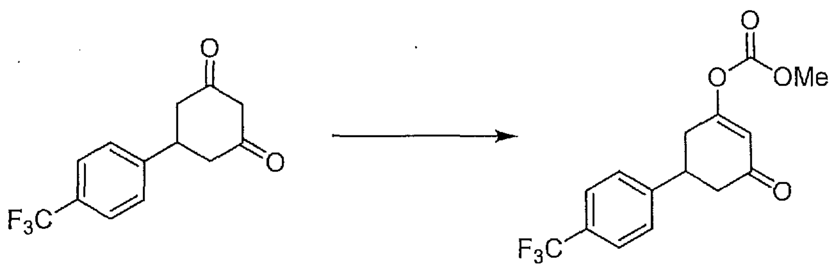

To a solution of 5-(4-(trifluoromethyl)phenyl)cyclohexane-1,3-dione (1.08 g, 4.20 mmol), methyl chloroformate (0.595 g, 6.30 mmol), and N,N-dimethylacetamide (5 mL) was added triethylamine (0.551 g, 5.50 mmol), and the mixture was stirred at room temperature for 2 hr. After completion of the reaction, the reaction solution was poured into 1N hydrochloric acid, and the mixture was extracted with ethyl acetate and washed with water. The obtained organic layer was dried over anhydrous magnesium sulfate and concentrated under reduced pressure. The obtained residue was purified by silica gel column chromatography to give methyl (5-oxo-4'-(trifluoromethyl)-1,2,5,6-tetrahydro-[1,1'-biphenyl]-3-yl)carbonate (1.17 g, 3.73 mmol),

yield: 89%
physical property: $^1$H-NMR ($CDCl_3$): 7.62 (d, 2H), 7.38 (d, 2H), 6.16 (d, 1H), 3.89 (s, 3H), 3.50 (s, 1H), 2.85-2.96 (m, 1H), 2.61-2.82 (m, 3H)

Reference Example 1 -2

Production of methyl 5-hydroxy-3-oxo-4'-(trifluoromethyl)-1,2,3,6-tetrahydro-[1,1'-biphenyl]-4-carboxylate

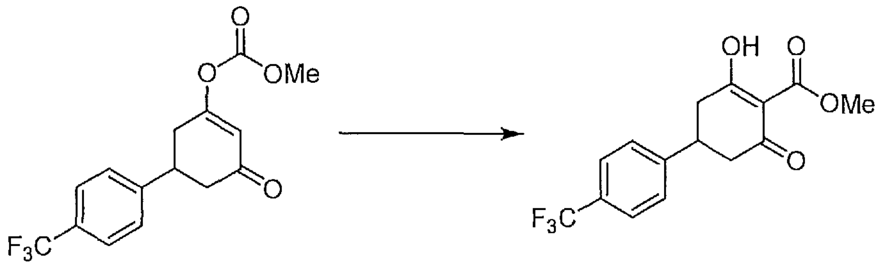

To methyl .(5-oxo-4'-(trifluoromethyl)-1,2,5,6-tetrahydro-[1,1'-biphenyl]-3-yl)carbonate (518 mg, 1.65 mmol) were added N,N-dimethyl-4-aminopyridine (80.0 mg, 0.65 mmol) and tetrahydrofuran (10 mL), and the mixture was reacted at room temperature for 3 hr. After completion of the reaction, the reaction solution was poured into 1N hydrochloric acid, and the mixture was extracted with ethyl acetate and washed with water. The obtained organic layer was dried over anhydrous magnesium sulfate and concentrated under reduced pressure. The obtained residue was purified by silica gel column chromatography to give methyl 5-hydroxy-3-oxo-4'-(trifluoromethyl)-1,2,3,6-tetrahydro-[1,1'-biphenyl]-4-carboxylate (73.8 mg, 0.235 mmol),

yield: 15%
physical property: melting point 126°C

Reference Example 2

Production of 5-phenyl-5-(4-(trifluoromethyl)phenyl)cyclohexane-1,3-dione

Reference Example 2-1

Production of (Z)-4-phenyl-4-(4-(trifluoromethyl)phenyl)but-3-en-2-one

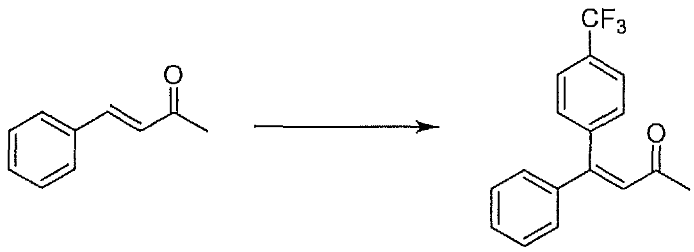

Under an argon atmosphere, to (E)-4-phenylbut-3-en-2-one (2.92 g, 20.0 mmol), 1-iodo-4-trifluoromethylbenzene (8.20 g, 20.0 mmol), palladium(II) acetate (50.0 mg, 0.200 mmol), and silver(I) acetate (3.40 g, 20.0 mmol) was added acetic acid (30 mL), and the mixture was stirred at 115°C for 3 hr. After completion of the reaction, Celite filtration was performed, and the filtrate was washed with ethyl acetate and neutralized with 5% sodium bicarbonate water. After concentration under reduced pressure, the obtained residue was purified by silica gel column chromatography to give (Z)-4-phenyl-4-(4-(trifluoromethyl)phenyl)but-3-en-2-one (5.25 g, 18.1 mmol),

yield: 91%
physical property: $^1$H-NMR ($CDCl_3$): 7.56-7.62 (m, 2H), 7.38-7.46 (m, 5H), 7.18-7.22 (m, 2H), 6.58 (s, 1H), 1.92 (s, 3H)

Reference Example 2-2

Production of 5-phenyl-5-(4-(trifluoromethyl)phenyl)cyclohexane-1,3-dione

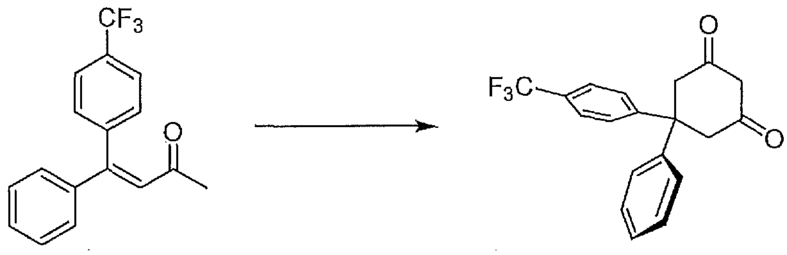

60% Sodium hydride (0.600 g, 15.0 mmol) was added to normal butanol (6 mL) at 0°C over 30 min. After warming to room temperature, diethyl malonate (2.40 g, 15.0 mmol) was added dropwise over 15 min, then a solution of (Z)-4-phenyl-4-(4-(trifluoromethyl)phenyl)but-3-en-2-one (18.5 g, 86.0 mmol) in toluene (10 mL) was added dropwise over 5 min, and the mixture was stirred with heating under reflux for 12 hr. After completion of the reaction, the reaction solution was poured into 1N hydrochloric acid, and the mixture was extracted with ethyl acetate and washed with water. The obtained organic layer was dried over anhydrous magnesium sulfate and concentrated under reduced pressure. Then, a 2N sodium hydroxide aqueous solution (10 mL)was added and the mixture was stirred at 60°C for 4 hr.

The reaction solution was poured into 6N hydrochloric acid, and the mixture was extracted with ethyl acetate and washed with water. The obtained organic layer was dried over anhydrous magnesium sulfate and concentrated under reduced pressure. The obtained residue was produced by silica gel column chromatography to give 5-phenyl-5-(4-(trifluoromethyl)phenyl)cyclohexane-1,3-dione (0.116 g, 0.350 mmol).
yield: 7%

Formulation Examples are shown below which are not limitative. In the Formulation Examples, parts indicate parts by weight.

Formulation Example 1.

| | |
|---|---|
| compound of the present invention | 10 parts |
| xylene | 70 parts |
| N-methylpyrrolidone | 10 parts |
| mixture of polyoxyethylene nonyl phenyl ether and calcium alkylbenzenesulfonate | 10 parts |

The above are uniformly mixed and dissolved to give an emulsion.

Formulation Example 2.

| | |
|---|---|
| compound of the present invention | 3 parts |
| clay powder | 82 parts |
| diatomaceous earth powder | 15 parts |

The above are uniformly mixed and pulverized to give a dust.

Formulation Example 3.

| | |
|---|---|
| compound of the present invention | 5 parts |
| mixed powder of bentonite and clay | 90 parts |
| calcium lignin sulfonate | 5 parts |

The above are uniformly mixed, an appropriate amount of water is added, and the mixture is kneaded, granulated, and dried to give granules.

Formulation Example 4.

| | |
|---|---|
| compound of the present invention | 20 parts |
| kaolin and synthetic highly dispersed silica | 75 parts |
| mixture of polyoxyethylene nonyl phenyl ether and calcium alkylbenzenesulfonate | 5 parts |

The above are uniformly mixed and pulverized to give a wettable powder.

Examples of biological tests are shown below; however, the present invention is not limited thereto.

Experimental Example 1. Insecticidal test against Plutella xylostella

Adults of Plutella xylostella were released on Chinese cabbage seedlings to spawn, and two days after release, the Chinese cabbage seedlings with eggs were immersed in a drug solution containing a compound represented by the formula (1) of the present invention as an active ingredient diluted to 500 ppm, for about 30 sec, air-dried, and left standing in a thermostatic chamber at 25°C. Six days after the immersion in the drug solution, the number of surviving insects was examined. The corrected mortality was calculated by the following equation and the assessment was performed according to the criteria described below. In triplicate using 10 per plot.

[numerical formula 1]

$$\text{corrected mortality (\%)} = \frac{\text{(survival rate in untreated plot} - \text{survival rate in treated plot)}}{\text{(survival rate in untreated plot)}} \times 100$$

assessment criteria.
A ··· mortality 100%
B ··· mortality 99% to 90%
C ··· mortality 89% to 80%
D ··· mortality 79% to 50%
E ··· mortality 49% to 0%

As a result, the compound represented by the formula (1) of the present invention showed a significant insecticidal activity, and particularly the compounds of compound Nos. 1-1, 1-2, 1-3, 1-6, 1-7, 1-8, 1-9, 1-11, 1-12, 1-15, 1-16, 1-17, 1-18, 1-19, 1-21, 1-22, 1-23, 1-30, 1-34, 1-35, 1-36, 1-38, 1-39, 1-40, 1-43, 1-45, 1-48, 1-49, 1-52, 1-53, 1-54, 1-55, 1-61, 1-62, 1-63, 1-65, 1-66, 1-68, 1-69, 1-70, 1-71, 1-75, 1-76, 1-77, 1-81, 1-93, 1-94, 1-95, 1-99, 1-100, 1-108, 1-140, 1-142, 1-144, 1-145, 1-146, 1-147, 1-148, 1-149, 1-151, 1-152, 1-155, 1-156, 1-158, 1-161, 1-162, 1-164, 1-165, 1-167, 1-184, 1-207, 1-208, 1-209, 1-210, 1-211, 1-212, 1-213, 1-222, 1-223, 1-225, 1-229, 1-231, 1-232, 1-233, 1-234, and 1-235 showed superior activity against Plutella xylostella with a rating of assessment D or higher.

Experimental Example 2. Insecticidal test against Laodelphax striatellus

A compound represented by the formula (1) or salts thereof of the present invention were dispersed in water and diluted to give a 500 ppm drug solution. Rice seedlings (variety: Nihonbare) were immersed in the drug solution for

30 seconds and air-dried, after which the seedlings were placed in a glass test tube and inoculated with 10 three-instar Laodelphax striatellus, and the test tube was plugged with a cotton plug. Eight days after the inoculation, the numbers of surviving insects and dead insects were examined. The corrected mortality was calculated by the following equation and the assessment was performed according to the criteria of Experimental Example 1.

[numerical formula 2]

$$\text{corrected mortality (\%)} = \frac{\text{(survival rate in untreated plot - survival rate in treated plot)}}{\text{(survival rate in untreated plot)}} \times 100$$

As a result, the compound represented by the formula (1) of the present invention showed a significant insecticidal activity, and particularly the compounds of compound Nos. 1-9, 1-68, 1-84, 1-149, 1-150, 1-152, 1-161, 1-164, 1-173, and 1-225 showed superior activity against Laodelphax striatellus with a rating of assessment D or higher.

Experimental Example 3. Insecticidal test against Frankliniella occidentalis

Female adult Frankliniella occidentalis were inoculated onto Phaseolus vulgaris leaf pieces, and after allowing them to lay eggs for one day, the female adults were removed. Three days later, hatched larvae on the leaf pieces were counted, and a drug solution containing the compounds listed in the first table to the third table as active ingredients diluted to 500 ppm was sprayed. Four days after the treatment, the number of surviving larvae of this species was examined.. The corrected mortality was calculated by the following equation and the assessment was performed according to the criteria of Experimental Example 1. In duplicate.

[numerical formula 3]

$$\text{corrected mortality (\%)} = \frac{\text{survival rate in untreated plot - survival rate in treated plot}}{\text{survival rate in untreated plot}} \times 100$$

As a result, the compound represented by the formula (1) of the present invention showed a significant insecticidal activity, and particularly the compounds of compound Nos. 1-9, 1-139, and 1-150 showed superior activity against Frankliniella occidentalis with a rating of assessment D or higher.

Experimental Example 4. Influence evaluation test on the growth of Dirofilaria immitis

A given prepared solution, 8 to 20 L-3 stage larvae of Dirofilaria immitis, and a DMSO-diluted solution of the compound of the present invention were added to each well of a 24-well plate to a final concentration of 10 μM. Seven days later, the larvae that had grown to the L-4 stage were counted, and the development inhibition rate was calculated from the L3 stage larva to the L4-stage larva.

As a result, among the compounds represented by the formula (1) of the present invention, the compounds of 1-2, 1-3, 1-6, 1-12, 1-37, 1-39, 1-40, 1-42, 1-46, 1-47, 1-48, 1-49, 1-54, 1-55, 1-63, 1-64, 1-67, 1-68, 1-73, 1-74, 1-81, 1-108, 1-124, 1-141, 1-142, 1-143, 1-144, 1-145, 1-146, 1-147, 1-148, 1-149, 1-150, 1-151, 1-152, 1-153, 1-154, 1-155, 1-156, 1-157, 1-158, 1-160, 1-161, 1-163, 1-164, 1-165, 1-166, 1-177, 1-208, 1-210, 1-212, 1-226, and 1-227 showed a development inhibition rate of not less than 50%.

[Industrial Applicability]

The compound of the present invention and a salt thereof have superior effects as pest control agents.

## Claims

1. A compound represented by the formula (1)

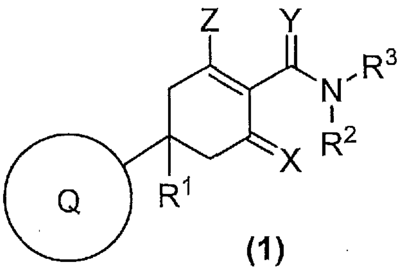

(1)

wherein

$R^1$ is
(a1) a hydrogen atom; (a2) a $(C_1-C_6)$alkyl group; (a3) a halo$(C_1-C_6)$alkyl group; (a4) a phenyl group; or (a5) a substituted phenyl group having, on a ring, 1 to 5 substituents each independently selected from the group consisting of a halogen atom, a $(C_1-C_6)$alkyl group, a halo $(C_1-C_6)$ alkyl group, and a $(C_1-C_6)$alkoxy group,
$R^2$ is
(b1) a hydrogen atom; (b2) a $(C_1-C_6)$alkyl group; (b3) a $(C_3-C_6)$cycloalkyl group; (b4) a $(C_1-C_6)$alkoxy group; or (b5) a $(C_1-C_6)$alkylcarbonyl group,
$R^3$ is
(c1) a $(C_1-C_6)$alkyl group; (c2) a $(C_2-C_6)$alkenyl group; (c3) a $(C_2-C_6)$alkynyl group; (c4) a $(C_3-C_6)$cycloalkyl group; (c5) a halo$(C_1-C_6)$alkyl group; (c6) a halo $(C_2-C_6)$alkenyl group; (c7) a halo$(C_2-C_6)$alkynyl group; (c8) a substituted $(C_1-C_6)$alkyl group having 1 to 3 substituents each independently selected from the group consisting of a cyano group, a $(C_3-C_6)$cycloalkyl group, a $(C_1-C_6)$alkoxy group, a $(C_1-C_6)$alkylthio group, a halo $(C_3-C_6)$ cycloalkyl group, a halo$(C_1-C_6)$alkoxy group, a halo$(C_1-C_6)$alkylthio group, a $(C_1-C_6)$alkoxycarbonyl group, a $(C_2-C_6)$alkenylcarbonyloxy group, a carboxamido group, a phenylcarbonyl group and a di$(C_1-C_6)$alkylamino group; (c9) a substituted $(C_3-C_6)$cycloalkyl group having, on a ring, 1 to 3 substituents each independently selected from the group consisting of a cyano group, a $(C_3-C_6)$cycloalkyl group, a $(C_1-C_6)$ alkoxy group, a $(C_1-C_6)$ alkylthio group, a $(C_1-C_6)$ alkyl group, a halo $(C_3-C_6)$ cycloalkyl group, a halo $(C_1-C_6)$ alkoxy group, a halo$(C_1-C_6)$alkylthio group and a carboxamido group; (c10) a $(C_1-C_6)$alkylsulfonyl group; (c11) an N-$(C_1-C_6)$alkylaminosulfonyl group; (c12) a phenylcarbonylamino group; (c13) a substituted phenylcarbonylamino group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group and a halo $(C_1-C_6)$ alkoxy group; (c14) a piperidinyl group; (c15) a substituted piperidinyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$ alkylsulfonyl group, a halo $(C_1-C_6)$ alkyl group, a halo $(C_1-C_6)$alkoxy group, a $(C_1-C_6)$alkoxycarbonyl group and a phenyl$(C_1-C_6)$alkoxycarbonyl group; (c16) a morpholinyl group; (c17) a substituted morpholinyl group having, on a ring, 1 to 3 substituents.each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo$(C_1-C_6)$alkoxy group; (c18) a substituted phenyl group having, on a ring, 1 to 5 substituents each independently selected from the group consisting of (a) a halogen atom, (b) a cyano group, (c) a nitro group, (d) a hydroxyl group, (e) a carboxyl group, (f) a $(C_1-C_6)$ alkyl group, (g) a $(C_1-C_6)$ alkoxy group, (h) a $(C_3-C_6)$cycloalkyl group, (i) a $(C_1-C_6)$alkylthio group, (j) a $(C_1-C_6)$alkylsulfinyl group, (k) a $(C_1-C_6)$alkylsulfonyl group, (l) a halo $(C_1-C_6)$ alkyl group, (m) a halo $(C_1-C_6)$ alkoxy group, (n) a halo $(C_3-C_6)$ cycloalkyl group, (o) a halo $(C_1-C_6)$ alkylthio group, (p) a halo $(C_1-C_6)$ alkylsulfinyl group, (q) a halo $(C_1-C_6)$ alkylsulfonyl group, (r) a $(C_1-C_6)$alkylcarbonyl group, (s) a $(C_1-C_6)$alkoxycarbonyl group, (t) a $(C_1-C_6)$ alkylcarbonylamino group, (u) a halo $(C_1-C_6)$alkylcarbonylamino group, (v) a $(C_1-C_6)$alkylsulfonylamino group, (w) a halo $(C_1-C_6)$ alkylsulfonylamino group, (x) an N,N-di$(C_1-C_6)$alkylcarboxamido group, (y) an N-$(C_1-C_6)$alkylcarboxamido group, (z) an N-halo$(C_1-C_6)$alkylcarboxamido group, (aa) an N-$(C_1-C_6)$alkylaminosulfonyl group, (ab) a $(C_1-C_6)$alkoxy$(C_1-C_6)$alkyl group, (ac) an $SF_5$ group, (ad) an $R^{10}$-($R^{11}$-N=)O=S group wherein $R^{10}$ is a $(C_1-C_6)$alkyl group, a $(C_3-C_6)$cycloalkyl group, a halo $(C_1-C_6)$ alkyl group, or a $(C_1-C_6)$ alkoxy$(C_1-C_6)$ alkyl group, and $R^{11}$ is a hydrogen atom, a cyano group, a $(C_1-C_6)$alkyl group, a $(C_3-C_6)$cycloalkyl group, a halo $(C_1-C_6)$ alkyl group, a $(C_1-C_6)$ alkylcarbonyl group, or a halo $(C_1-C_6)$ alkylcarbonyl group, (ae) an $R^{10}$- ($R^{11}$-N=) S group wherein $R^{10}$ and $R^{11}$ are the same as above, (af) a pyrazolyl group, (ag) a phenoxy group, (ah) a phenyl group,

and (ai) a substituted phenyl group having, on a ring, 1 to 5 substituents each independently selected from the group consisting of a halogen atom, a cyano group, a $(C_1-C_6)$ alkyl group, a $(C_1-C_6)$ alkoxy group, a $(C_3-C_6)$cycloalkyl group, a $(C_1-C_6)$alkylthio group, a $(C_1-C_6)$alkylsulfinyl group, a $(C_1-C_6)$ alkylsulfonyl group, a halo $(C_1-C_6)$alkyl group and a halo $(C_1-C_6)$ alkoxy group, or (aj) a methylenedioxy group formed by two adjacent substituents together and optionally substituted by 1 or 2 substituents selected from a halogen atom, a phenyl group and a $(C_1-C_6)$alkyl group, provided that an atom adjacent to a bonding atom in the pyrazolyl group is not substituted by the $(C_1-C_6)$alkylsulfonyl group, the halo$(C_1-C_6)$alkylsulfonyl group, the N-$(C_1-C_6)$alkylaminosulfonyl group, or the $R^{10}$-($R^{11}$-N=)O=S group wherein $R^{10}$ and $R^{11}$ are the same as above; (c19) a pyridyl group; (c20) a substituted pyridyl group having, on a ring, 1 to 4 substituents each independently selected from the group consisting of a halogen atom, a cyano group, a nitro group, a $(C_1-C_6)$ alkyl group, a $(C_1-C_6)$alkoxy group, a $(C_3-C_6)$ cycloalkyl group, a $(C_1-C_6)$ alkylthio group, a $(C_1-C_6)$alkylsulfinyl group, a $(C_1-C_6)$ alkylsulfonyl group, a halo$(C_1-C_6)$ alkyl group, a halo $(C_1-C_6)$ alkoxy group, a halo $(C_1-C_6)$ alkylthio group, a halo $(C_1-C_6)$ alkylsulfinyl group and a halo$(C_1-C_6)$alkylsulfonyl group; (c21) an N-oxo-pyridyl group; (c22) a substituted N-oxo-pyridyl group having, on a ring, 1 to 4 substituents each independently selected from the group consisting of a halogen atom, a cyano group, a nitro group, a $(C_1-C_6)$alkyl group, a $(C_1-C_6)$ alkoxy group, a $(C_3-C_6)$ cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group, a halo$(C_1-C_6)$alkoxy group, a halo$(C_1-C_6)$alkylthio group,. a halo$(C_1-C_6)$alkylsulfinyl group and a halo $(C_1-C_6)$ alkylsulfonyl group; (c23) a pyridazinyl group; (c24) a substituted pyridazinyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c25) a pyrimidinyl group; (c26) a substituted pyrimidinyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c27) a pyrazinyl group; (c28) a substituted pyrazinyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo $(C_1-C_6)$ alkoxy group; (c29) a triazinyl group; (c30) a substituted triazinyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$ alkylthio group, a $(C_1-C_6)$ alkylsulfinyl group, a $(C_1-C_6)$alkylsulfonyl group, a halo $(C_1-C_6)$ alkyl group and a halo$(C_1-C_6)$alkoxy group; (c31) a tetrahydrofuranyl group; (c32) a substituted tetrahydrofuranyl group having, on a ring, 1 to 3 substituents each independently selected from the group consisting of a halogen atom, a cyano group, a $(C_1-C_6)$alkyl group, a $(C_1-C_6)$ alkoxy group, a $(C_3-C_6)$ cycloalkyl group, a $(C_1-C_6)$ alkylthio group, a $(C_1-C_6)$alkylsulfinyl group, a $(C_1-C_6)$alkylsulfonyl group, a halo $(C_1-C_6)$ alkyl group and a halo $(C_1-C_6)$ alkoxy group; (c33) an oxazolinyl group; (c34) a substituted oxazolinyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$ alkylsulfonyl group, a halo $(C_1-C_6)$ alkyl group and a halo$(C_1-C_6)$alkoxy group; (c35) a thiazolinyl group; (c36) a substituted thiazolinyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$ alkylsulfonyl group, a halo$(C_1-C_6)$alkyl group and a halo $(C_1-C_6)$ alkoxy group; (c37) an oxazolyl group; (c38) a substituted oxazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c39) a thiazolyl group; (c40) a substituted thiazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$ alkylthio group, a $(C_1-C_6)$ alkylsulfinyl group, a $(C_1-C_6)$ alkylsulfonyl group, a halo $(C_1-C_6)$ alkyl group and a halo$(C_1-C_6)$alkoxy group; (c41) an isoxazolyl group; (c42) a substituted isoxazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$ cycloalkyl group, a $(C_1-C_6)$ alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c43) an isothiazolyl group; (c44) a substituted isothiazolyl group having, on a ring, 1 or 2 substituents each independently selected from the group consisting of a halogen atom, a cyano group, a $(C_1-C_6)$alkyl group, a $(C_1-C_6)$ alkoxy group, a $(C_3-C_6)$ cycloalkyl group, a $(C_1-C_6)$ alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c45) an

imidazolyl group; (c46) a substituted imidazolyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo $(C_1-C_6)$alkoxy group; (c47) a pyrazolyl group; (c48) a substituted pyrazolyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$ alkylsulfonyl group, a halo$(C_1-C_6)$alkyl group and a halo $(C_1-C_6)$ alkoxy group; (c49) a triazolyl group; (c50) a substituted triazolyl group having, on a ring, 1 or 2 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo $(C_1-C_6)$alkoxy group; (c51) a tetrazolyl group; (c52) a substituted tetrazolyl group having, on a ring, one substituent selected from the group consisting of a halogen atom, a cyano group, a $(C_1-C_6)$alkyl group, a $(C_1-C_6)$alkoxy group, a $(C_3-C_6)$ cycloalkyl group, a $(C_1-C_6)$ alkylthio group, a $(C_1-C_6)$ alkylsulfinyl group, a $(C_1-C_6)$ alkylsulfonyl group, a halo$(C_1-C_6)$ alkyl group and a halo $(C_1-C_6)$ alkoxy group; (c53) a thiadiazolyl group; (c54) a substituted thiadiazolyl group having, on a ring, one substituent selected from the group consisting of a halogen atom, a cyano group, a $(C_1-C_6)$ alkyl group, a $(C_1-C_6)$ alkoxy group, a $(C_3-C_6)$ cycloalkyl group, a $(C_1-C_6)$ alkylthio group, a $(C_1-C_6)$alkylsulfinyl group, a $(C_1-C_6)$alkylsulfonyl group, a halo$(C_1-C_6)$alkyl group and a halo $(C_1-C_6)$alkoxy group; (c55) a quinolinyl group; (c56) a substituted quinolinyl group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$ alkylsulfinyl group, a $(C_1-C_6)$alkylsulfonyl group, a halo $(C_1-C_6)$ alkyl group and a halo$(C_1-C_6)$alkoxy group; (c57) a benzoxazolyl group; (c58) a substituted benzoxazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$ alkylthio group, a $(C_1-C_6)$alkylsulfinyl group, a $(C_1-C_6)$alkylsulfonyl group, a halo $(C_1-C_6)$alkyl group and a halo$(C_1-C_6)$alkoxy group; (c59) a benzothiazolyl group; (c60) a substituted benzothiazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$ cycloalkyl group, a $(C_1-C_6)$ alkylthio group, a $(C_1-C_6)$alkylsulfinyl group, a $(C_1-C_6)$alkylsulfonyl group, a halo$(C_1-C_6)$ alkyl group and a halo $(C_1-C_6)$alkoxy group; (c61) a benzimidazolyl group; (c62) a substituted benzimidazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$ cycloalkyl group, a $(C_1-C_6)$ alkylthio group, a $(C_1-C_6)$alkylsulfinyl group, a $(C_1-C_6)$alkylsulfonyl group, a halo $(C_1-C_6)$ alkyl group and a halo$(C_1-C_6)$alkoxy group; (c63) a benzothiadiazolyl group; (c64) a substituted benzothiadiazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$ cycloalkyl group, a $(C_1-C_6)$ alkylthio group, a $(C_1-C_6)$ alkylsulfinyl group, a $(C_1-C_6)$ alkylsulfonyl group, a halo$(C_1-C_6)$alkyl group and a halo $(C_1-C_6)$alkoxy group; (c65) an indazolyl group; (c66) a substituted indazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo $(C_1-C_6)$alkoxy group; (c67) an indanyl group; (c68) a substituted indanyl group having, on a ring, 1 to 4 substituents each independently selected from the group consisting of a halogen atom, a cyano group, a $(C_1-C_6)$ alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$ alkoxy group; (c69) a tetrahydronaphthyl group; (c70) a substituted tetrahydronaphthyl group having, on a ring, 1 to 4 substituents each independently selected from the group consisting of a halogen atom, a cyano group, a $(C_1-C_6)$ alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$ alkoxy group; (c71) a tetrahydrofuranyl$(C_1-C_6)$alkyl group; (c72) a substituted tetrahydrofuranyl$(C_1-C_6)$alkyl group having, on a ring, 1 to 3 substituents each independently selected from the group consisting of a halogen atom, a cyano group, a $(C_1-C_6)$ alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group and a halo $(C_1-C_6)$alkoxy group; (c73) a phenyl$(C_1-C_6)$alkyl group; (c74) a substituted phenyl$(C_1-C_6)$ alkyl group having, on a ring, 1 to 5 substituents each independently selected from the group -consisting of a halogen atom, a cyano group, a $(C_1-C_6)$ alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c75) a $(C_1-C_6)$alkoxycarbonyl group; (c76) a phenylcarbonyl group; (c77) a thienyl group; (c78) a substituted thienyl group having, on a ring, 1 to 3 substituents each independently selected from the group consisting of a halogen atom, a cyano group, a nitro group, a $(C_1-C_6)$

alkyl group, a ($C_1$-$C_6$) alkoxy group, a ($C_3$-$C_6$) cycloalkyl group, a ($C_1$-$C_6$)alkylthio group, a ($C_1$-$C_6$)alkylsulfinyl group, a ($C_1$-$C_6$) alkylsulfonyl group, a halo ($C_1$-$C_6$)alkyl group, a halo ($C_1$-$C_6$) alkoxy group, a halo ($C_1$-$C_6$) alkylthio group, a halo ($C_1$-$C_6$) alkylsulfinyl group and a halo ($C_1$-$C_6$) alkylsulfonyl group; (c79) a naphthyl group; (c80) a substituted naphthyl group having, on a ring, 1 to 7 substituents each independently selected from the group consisting of a halogen atom, a cyano group, a nitro group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$) alkylsulfonyl group, a halo ($C_1$-$C_6$) alkyl group, a halo($C_1$-$C_6$) alkoxy group, a halo ($C_1$-$C_6$) alkylthio group, a halo($C_1$-$C_6$)alkylsulfinyl group and a halo ($C_1$-$C_6$) alkylsulfonyl group; (c81) a 1,1-dioxidetetrahydro-2H-thiopyranyl group; (c82) an imidazopyridyl group; or (c83) a substituted imidazopyridyl group having, on a ring, 1 to 5 substituents each independently selected from the group consisting of a halogen atom, a cyano group, a nitro group, a ($C_1$-$C_6$)alkyl group, a ($C_1$-$C_6$) alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group, a halo ($C_1$-$C_6$)alkoxy group, a halo ($C_1$-$C_6$)alkylthio group, a halo ($C_1$-$C_6$) alkylsulfinyl group, a halo ($C_1$-$C_6$) alkylsulfonyl group and a phenyl group,
Q is

(d1) a substituted phenyl group having, on a ring, 1 to 5 substituents each independently selected from substituent group A;

(d2) a naphthyl group; (d3) a substituted naphthyl group having, on a ring, 1 to 7 substituents each independently selected from substituent group B; (d4) a pyridyl group; (d5) a substituted pyridyl group having, on a ring, 1 to 4 substituents each independently selected from substituent group B; (d6) a pyridazinyl group; (d7) a substituted pyridazinyl group having, on a ring, 1 to 3 substituents each independently selected from substituent group B; (d8) a pyrimidinyl group; (d9) a substituted pyrimidinyl group having, on a ring, 1 to 3 substituents each independently selected from substituent group B; (d10) a pyrazinyl group; (d11) a substituted pyrazinyl group having, on a ring, 1 to 3 substituents each independently selected from substituent group B; (d12) a triazinyl group; (d13) a substituted triazinyl group having, on a ring, 1 or 2 substituents each independently selected from substituent group B; (d14) a 2-oxopyridyl group; (d15) a substituted 2-oxopyridyl group having, on a ring, 1 to 4 substituents each independently selected from substituent group B; (d16) an isoxazolyl group; (d17) a substituted isoxazolyl group having, on a ring, 1 or 2 substituents each independently selected from substituent group B; (d18) an oxazolyl group; (d19) a substituted oxazolyl group having, on a ring, 1 or 2 substituents each independently selected from substituent group B; (d20) a thienyl group; (d21) a substituted thienyl group having, on a ring, 1 to 3 substituents each independently selected from substituent group B; (d22) an isothiazolyl group; (d23) a substituted isothiazolyl group having, on a ring, 1 or 2 substituents each independently selected from substituent group B; (d24) a thiazolyl group; (d25) a substituted thiazolyl group having, on a ring, 1 or 2 substituents each independently selected from substituent group B; (d26) an oxadiazolyl group; (d27) a substituted oxadiazolyl group having, on a ring, one substituent each independently selected from substituent group B; (d28) a thiadiazolyl group;

(d29) a substituted thiadiazolyl group having, on a ring, one substituent each independently selected from substituent group B;

(d30) a pyrrolyl group; (d31) a substituted pyrrolyl group having, on a ring, 1 to 4 substituents each independently selected from substituent group B; (d32) an imidazolyl group; (d33) a substituted imidazolyl group having, on a ring, 1 to 3 substituents each independently selected from substituent group B;

(d34) a triazolyl group; (d35) a substituted triazolyl group having, on a ring, 1 or 2 substituents each independently selected from substituent group B; (d36) a tetrazolyl group; (d37) a substituted tetrazolyl group having, on a ring, one substituent each independently selected from substituent group B; (d38) a benzofuranyl group; (d39) a substituted benzofuranyl group having, on a ring, 1 to 5 substituents each independently selected from substituent group B; (d40) a benzoxazolyl group; (d41) a substituted benzoxazolyl group having, on a ring, 1 to 4 substituents each independently selected from substituent group B;

(d42) a benzisoxazolyl group; (d43) a substituted benzisoxazolyl group having, on a ring, 1 to 4 substituents each independently selected from substituent group B; (d44) a benzothienyl group;

(d45) a substituted benzothienyl group having, on a ring, 1 to 5 substituents each independently selected from substituent group B;

(d46) a benzothiazolyl group; (d47) a substituted benzothiazolyl group having, on a ring, 1 to 4 substituents each independently selected from substituent group B; (d48) a benzisothiazolyl group;

(d49) a substituted benzisothiazolyl group having, on a ring, 1 to 4 substituents each independently selected from substituent group B; (d50) an indolyl group; (d51) a substituted indolyl group having, on a ring, 1 to 6 substituents each independently selected from substituent group B; (d52) an isoindolyl group; (d53) a substituted isoindolyl group having, on a ring, 1 to 6 substituents each independently selected from substituent group B;

(d54) an indazolyl group; (d55) a substituted indazolyl group having, on a ring, 1 to 5 substituents each independently selected from substituent group B; (d56) a benzimidazolyl group; (d57) a substituted benzimidazolyl group having, on a ring, 1 to 5 substituents each independently selected from substituent group B; (d58) a benzotriazolyl group; (d59) a substituted benzotriazolyl group having, on a ring, 1 to 4 substituents each independently selected from substituent group B; (d60) a furopyridyl group;
(d61) a substituted furopyridyl group having, on a ring, 1 to 4 substituents each independently selected from substituent group B;
(d62) a thienopyridyl group; (d63) a substituted thienopyridyl group having, on a ring, 1 to 4 substituents each independently selected from substituent group B; (d64) an indolizinyl group;
(d65) a substituted indolizinyl group having, on a ring, 1 to 6 substituents each independently selected from substituent group B;
(d66) a pyrrolopyridyl group; (d67) a substituted pyrrolopyridyl group having, on a ring, 1 to 5 substituents each independently selected from substituent group B; (d68) a pyrrolopyrimidinyl group; (d69) a substituted pyrrolopyrimidinyl group having, on a ring, 1 to 5 substituents each independently selected from substituent group B; (d70) an oxazolopyridyl group; (d71) a substituted oxazolopyridyl group having, on a ring, 1 to 3 substituents each independently selected from substituent group B;
(d72) an isoxazolopyridyl group; (d73) a substituted isoxazolopyridyl group having, on a ring, 1 to 3 substituents each independently selected from substituent group B; (d74) a thiazolopyridyl group; (d75) a substituted thiazolopyridyl group having, on a ring, 1 to 3 substituents each independently selected from substituent group B; (d76) an isothiazolopyridyl group; (d77) a substituted isothiazolopyridyl group having, on a ring, 1 to 3 substituents each independently selected from substituent group B;
(d78) an imidazopyridyl group; (d79) a substituted imidazopyridyl group having, on a ring, 1 to 5 substituents each independently selected from substituent group B; (d80) an imidazopyrimidinyl group; (d81) a substituted imidazopyrimidinyl group having, on a ring, 1 to 5 substituents each independently selected from substituent group B; (d82) a pyrazolopyridyl group; (d83) a substituted pyrazolopyridyl group having, on a ring, 1 to 5 substituents each independently selected from substituent group B;
(d84) a pyrazolopyrimidinyl group; (d85) a substituted pyrazolopyrimidinyl group having, on a ring, 1 to 5 substituents each independently selected from substituent group B; (d86) a triazolopyridyl group; (d87) a substituted triazolopyridyl group having, on a ring, 1 to 4 substituents each independently selected from substituent group B; (d88) a triazolopyrimidinyl group; (d89) a substituted triazolopyrimidinyl group having, on a ring, 1 to 4 substituents each independently selected from substituent group B;
(d90) a quinoxalinyl group; (d91) a substituted quinoxalinyl group having, on a ring, 1 to 5 substituents each independently selected from substituent group B; (d92) a quinolinyl group; (d93) a substituted quinolinyl group having, on a ring, 1 to 6 substituents each independently selected from substituent group B;
(d94) an isoquinolinyl group; (d95) a substituted isoquinolinyl group having, on a ring, 1 to 6 substituents each independently selected from substituent group B; (d96) a cinnolinyl group; (d97) a substituted cinnolinyl group having, on a ring, 1 to 5 substituents each independently selected from substituent group B;
(d98) a phthalazinyl group; (d99) a substituted phthalazinyl group having, on a ring, 1 to 5 substituents each independently selected from substituent group B; (d100) a quinazolinyl group; (d101) a substituted quinazolinyl group having, on a ring, 1 to 5 substituents each independently selected from substituent group B;
(d102) a naphthyridinyl group; (d103) a substituted naphthyridinyl group having, on a ring, 1 to 5 substituents each independently selected from substituent group B; or (d104) a ferrocenyl group, X and Y are each independently an oxygen atom or a sulfur atom, Z is an $OR^4$ group wherein $R^4$ is a hydrogen atom, a $(C_1-C_6)$ alkyl group, a $(C_1-C_6)$ alkylcarbonyl group or a $(C_1-C_6)$ alkoxycarbonyl group, an $S(O)nR^5$ group wherein n is 0, 1 or 2, and $R^5$ is a hydrogen atom, a $(C_1-C_6)$ alkyl group or a phenyl $(C_1-C_6)$ alkyl group, or an $N(R^6)R^7$ group wherein $R^6$ and $R^7$ are hydrogen atoms, hydroxyl groups, $(C_1-C_6)$ alkyl groups, $(C_1-C_6)$alkoxy groups, $(C_1-C_6)$ alkylcarbonyl groups or $(C_1-C_6)$alkoxycarbonyl groups, and $R^6$ and $R^7$ are the same or different,

substituent group A consists of

(e1) a cyano group; (e2) a nitro group; (e3) a hydroxyl group;
(e4) a carboxyl group; (e5) a $(C_1-C_6)$alkyl group; (e6) a $(C_2-C_6)$ alkenyl group; (e7) a $(C_2-C_6)$ alkynyl group; (e8) a $(C_3-C_6)$cycloalkyl group; (e9) a substituted $(C_3-C_6)$cycloalkyl group having, on a ring, 1 to 3 substituents each independently selected from the group consisting of a halogen atom, a cyano group, a $(C_1-C_6)$ alkyl group, a $(C_1-C_6)$ alkoxy group and a $(C_1-C_6)$ alkylcarbonyl group; (e10) a $(C_1-C_6)$alkoxy$(C_1-C_6)$alkyl group; (e11) a $(C_1-C_6)$alkylthio group; (e12) a $(C_1-C_6)$alkylsulfinyl group; (e13) a $(C_1-C_6)$alkylsulfonyl group; (e14) a halo $(C_1-C_6)$ alkyl group; (e15) a halo $(C_2-C_6)$ alkenyl group; (e16) a halo $(C_2-C_6)$ alkynyl group;

(e17) a halo ($C_1$-$C_6$)alkoxy group; (e18) a halo ($C_3$-$C_6$)cycloalkyl group; (e19) a halo($C_1$-$C_6$)alkylthio group; (e20) a halo($C_1$-$C_6$)alkylsulfinyl group; (e21) a halo ($C_1$-$C_6$) alkylsulfonyl group; (e22) an $SF_5$ group; (e23) a ($C_1$-$C_6$)alkylcarbonylamino group; (e24) a halo($C_1$-$C_6$)alkylcarbonylamino group; (e25) a ($C_1$-$C_6$)alkylsulfonylamino group; (e26) a halo ($C_1$-$C_6$) alkylsulfonylamino group; (e27) an N,N-di($C_1$-$C_6$)alkylcarboxamido group; (e28) an N-halo($C_1$-$C_6$)alkylcarboxamido group; (e29) a ($C_1$-$C_6$)alkoxycarbonyl group;

(e30) an N-($C_1$-$C_6$)alkylaminosulfonyl group; (e31) an N,N-di($C_1$-$C_6$)alkylaminosulfonyl group; (e32) a pyrazolyl group; (e33) a substituted pyrazolyl group having, on a ring, 1 to 3 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$) alkoxy group; (e34) an oxadiazolyl group; (e35) a substituted oxadiazolyl group having, on a ring, one substituent each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo ($C_1$-$C_6$) alkyl group and a halo ($C_1$-$C_6$) alkoxy group; (e36) a phenyl group; (e37) a substituted phenyl group having, on a ring, 1 to 5 substituents each independently selected from the group consisting of a halogen atom, a cyano group, a ($C_1$-$C_6$)alkyl group, a ($C_1$-$C_6$) alkoxy group, a ($C_3$-$C_6$)cycloalkyl group, a ($C_1$-$C_6$) alkylthio group, a ($C_1$-$C_6$)alkylsulfinyl group, a ($C_1$-$C_6$)alkylsulfonyl group, a halo ($C_1$-$C_6$) alkyl group and a halo ($C_1$-$C_6$)alkoxy group; (e38) a phenoxy group; (e39) a substituted phenoxy group having, on a ring, 1 to 5 substituents each independently selected from the group consisting of a halogen atom, a cyano group, a ($C_1$-$C_6$)alkyl group, a ($C_1$-$C_6$) alkoxy group, a ($C_3$-$C_6$) cycloalkyl group, a ($C_1$-$C_6$) alkylthio group, a ($C_1$-$C_6$)alkylsulfinyl group, a ($C_1$-$C_6$)alkylsulfonyl group, a halo($C_1$-$C_6$)alkyl group and a halo ($C_1$-$C_6$) alkoxy group; (e40) a methylenedioxy group formed by two adjacent substituents together and optionally substituted by 1 or 2 substituents selected from the group consisting of a halogen atom, a phenyl group and a ($C_1$-$C_6$)alkyl group; (e41) an $R^8$-($R^9$-N=)O=S group wherein $R^8$ is a ($C_1$-$C_6$)alkyl group, a ($C_3$-$C_6$)cycloalkyl group, a halo ($C_1$-$C_6$) alkyl group, or a ($C_1$-$C_6$)alkoxy($C_1$-$C_6$)alkyl group, and $R^9$ is a hydrogen atom, a cyano group, a ($C_1$-$C_6$) alkyl group, a ($C_3$-$C_6$) cycloalkyl group, a halo($C_1$-$C_6$)alkyl group, a ($C_1$-$C_6$)alkylcarbonyl group, or a halo($C_1$-$C_6$)alkylcarbonyl group; (e42) a carboxamido group; (e43) a morpholinyl group; (e44) an N,N-di($C_1$-$C_6$)alkylamino group; (e45) an $R^8$-($R^9$-N=)S group wherein $R^8$ and $R^9$ are the same as above; (e46) an N-($C_1$-$C_6$)alkylcarboxamido group; (e47) a phenyl ($C_1$-$C_6$) alkylthio group; and (e48) a substituted phenyl ($C_1$-$C_6$)alkylthio group having, on a ring, 1 to 5 substituents each independently selected from the group consisting of a halogen atom, a cyano group, a ($C_1$-$C_6$) alkyl group, a ($C_1$-$C_6$)alkoxy group, a ($C_3$-$C_6$) cycloalkyl group, a ($C_1$-$C_6$) alkylthio group, a ($C_1$-$C_6$) alkylsulfinyl group, a ($C_1$-$C_6$)alkylsulfonyl group, a halo ($C_1$-$C_6$) alkyl group and a halo($C_1$-$C_6$)alkoxy group, and

substituent group B consists of

(f1) a halogen atom; (f2) a cyano group; (f3) a nitro group; (f4) a hydroxyl group; (f5) a carboxyl group; (f6) a ($C_1$-$C_6$)alkyl group;

(f7) a ($C_2$-$C_6$)alkenyl group; (f8) a ($C_2$-$C_6$)alkynyl group; (f9) a ($C_1$-$C_6$)alkoxy group; (f10) a ($C_3$-$C_6$) cycloalkyl group; (f11) a substituted ($C_3$-$C_6$)cycloalkyl group having, on a ring, 1 to 3 substituents each independently selected from the group consisting of a halogen atom, a cyano group, a ($C_1$-$C_6$) alkyl group, a ($C_1$-$C_6$)alkoxy group and a ($C_1$-$C_6$)alkylcarbonyl group; (f12) a ($C_1$-$C_6$) alkoxy ($C_1$-$C_6$) alkyl group; (f13) a ($C_1$-$C_6$)alkylthio group; (f14) a ($C_1$-$C_6$)alkylsulfinyl group; (f15) a ($C_1$-$C_6$)alkylsulfonyl group;

(f16) a halo ($C_1$-$C_6$) alkyl group; (f17) a halo ($C_2$-$C_6$) alkenyl group;

(f18) a halo ($C_2$-$C_6$)alkynyl group; (f19) a halo ($C_1$-$C_6$) alkoxy group;

(f20) a halo ($C_3$-$C_6$) cycloalkyl group; (f21) a halo ($C_1$-$C_6$) alkylthio group; (f22) a halo ($C_1$-$C_6$) alkylsulfinyl group; (f23) a halo ($C_1$-$C_6$)alkylsulfonyl group; (f24) an $SF_5$ group; (f25) a ($C_1$-$C_6$)alkylcarbonylamino group; (f26) a halo ($C_1$-$C_6$)alkylcarbonylamino group; (f27) a ($C_1$-$C_6$)alkylsulfonylamino group; (f28) a halo ($C_1$-$C_6$)alkylsulfonylamino group; (f29) an N,N-di($C_1$-$C_6$)alkylcarboxamido group; (f30) an N-halo ($C_1$-$C_6$) alkylcarboxamido group; (f31) a ($C_1$-$C_6$)alkoxycarbonyl group; (f32) an N-($C_1$-$C_6$)alkylaminosulfonyl group; (f33) an N,N-di ($C_1$-$C_6$) alkylaminosulfonyl group; (f34) a pyrazolyl group; (f35) a substituted pyrazolyl group having, on a ring, 1 to 3 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo ($C_1$-$C_6$) alkyl group and a halo($C_1$-$C_6$)alkoxy group; (f36) an oxadiazolyl group; (f37) a substituted oxadiazolyl group having, on a ring, one substituent each independently selected from the group consisting of a halogen atom, a cyano group, a ($C_1$-$C_6$)alkyl group, a ($C_1$-$C_6$)alkoxy group, a ($C_3$-$C_6$) cycloalkyl group, a ($C_1$-$C_6$) alkylthio

group, a $(C_1-C_6)$alkylsulfinyl group, a $(C_1-C_6)$ alkylsulfonyl group, a halo$(C_1-C_6)$alkyl group and a halo $(C_1-C_6)$ alkoxy group; (f38) a phenyl group;

(f39) a substituted phenyl group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$ cycloalkyl group, a $(C_1-C_6)$ alkylthio group, a $(C_1-C_6)$ alkylsulfinyl group, a $(C_1-C_6)$ alkylsulfonyl group, a halo $(C_1-C_6)$ alkyl group and a halo $(C_1-C_6)$alkoxy group; (f40) a phenoxy group; (f41) a substituted phenoxy group having, on a ring, 1 to 5 substituents each independently selected from the group consisting of a halogen atom, a cyano group, a $(C_1-C_6)$alkyl group, a $(C_1-C_6)$ alkoxy group, a $(C_3-C_6)$ cycloalkyl group, a $(C_1-C_6)$ alkylthio group, a $(C_1-C_6)$alkylsulfinyl group, a $(C_1-C_6)$alkylsulfonyl group, a halo $(C_1-C_6)$ alkyl group and a halo $(C_1-C_6)$alkoxy group; (f42) a methylenedioxy group formed by two adjacent substituents together and optionally substituted by 1 or 2 substituents selected from the group consisting of a halogen atom, a phenyl group and a $(C_1-C_6)$alkyl group; (f43) an $R^8$-($R^9$-N=)O=S group wherein $R^8$ is a $(C_1-C_6)$ alkyl group, a $(C_3-C_6)$ cycloalkyl group, a halo $(C_1-C_6)$ alkyl group, or a $(C_1-C_6)$alkoxy$(C_1-C_6)$alkyl group, and $R^9$ is a hydrogen atom, a cyano group, a $(C_1-C_6)$alkyl group, a $(C_3-C_6)$ cycloalkyl group, a halo $(C_1-C_6)$ alkyl group, a $(C_1-C_6)$ alkylcarbonyl group, or a halo$(C_1-C_6)$alkylcarbonyl group; (f44) a carboxamido group; (f45) a morpholinyl group; and (f46) an N,N-di $(C_1-C_6)$ alkylamino group, provided that a compound represented by the following structural formula is excluded:

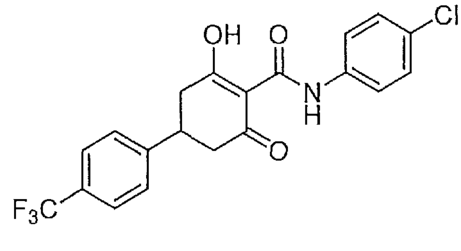

,

and salts thereof.

2. The compound and salts thereof according to claim 1, wherein

$R^1$ is
(a1) a hydrogen atom; (a2) a $(C_1-C_6)$alkyl group; (a3) a halo$(C_1-C_6)$alkyl group; (a4) a phenyl group; or (a5) a substituted phenyl group having, on a ring, 1 to 5 substituents each independently selected from the group consisting of a halogen atom, a $(C_1-C_6)$alkyl group, a halo $(C_1-C_6)$ alkyl group, and a $(C_1-C_6)$alkoxy group,
$R^2$ is
(b1) a hydrogen atom; or (b2) a $(C_1-C_6)$alkyl group,
$R^3$ is
(c1) a $(C_1-C_6)$alkyl group; (c2) a $(C_2-C_6)$alkenyl group; (c3) a $(C_2-C_6)$alkynyl group; (c4) a $(C_3-C_6)$cycloalkyl group; (c5) a halo $(C_1-C_6)$alkyl group; (c8) a substituted $(C_1-C_6)$alkyl group having 1 to 3 substituents each independently selected from the group consisting of a cyano group, a $(C_3-C_6)$ cycloalkyl group, a $(C_1-C_6)$ alkoxy group, a $(C_1-C_6)$ alkylthio group, a halo $(C_3-C_6)$cycloalkyl group, a halo $(C_1-C_6)$ alkoxy group, a halo $(C_1-C_6)$ alkylthio group, a $(C_1-C_6)$alkoxycarbonyl group, a $(C_2-C_6)$alkenylcarbonyloxy group, a carboxamido group, a phenylcarbonyl group and a di$(C_1-C_6)$alkylamino group; (c9) a substituted $(C_3-C_6)$cycloalkyl group having, on a ring, 1 to 3 substituents each independently selected from the group consisting of a cyano group, a $(C_3-C_6)$cycloalkyl group, a $(C_1-C_6)$ alkoxy group, a $(C_1-C_6)$ alkylthio group, a $(C_1-C_6)$ alkyl group, a halo $(C_3-C_6)$ cycloalkyl group, a halo$(C_1-C_6)$alkoxy group, a halo$(C_1-C_6)$alkylthio group and a carboxamido group; (c18) a substituted phenyl group having, on a ring, 1 to 5 substituents each independently selected from the group consisting of (a) a halogen atom, (b) a cyano group, (c) a nitro group, (d) a hydroxyl group, (e) a carboxyl group, (f) a $(C_1-C_6)$ alkyl group, (g) a $(C_1-C_6)$ alkoxy group, (h) a $(C_3-C_6)$cycloalkyl group, (i) a $(C_1-C_6)$alkylthio group, (j) a $(C_1-C_6)$alkylsulfinyl group, (k) a $(C_1-C_6)$alkylsulfonyl group, (l) a halo $(C_1-C_6)$ alkyl group, (m) a halo$(C_1-C_6)$alkoxy group, (n) a halo $(C_3-C_6)$ cycloalkyl group, (o) a halo $(C_1-C_6)$ alkylthio group, (p) a halo $(C_1-C_6)$ alkylsulfinyl group, (q) a halo$(C_1-C_6)$alkylsulfonyl group, (r) a $(C_1-C_6)$alkylcarbonyl group, (s) a $(C_1-C_6)$alkoxycarbonyl group, (t) a $(C_1-C_6)$alkylcarbonylamino group, (u) a halo$(C_1-C_6)$alkylcarbonylamino group, (v) a $(C_1-C_6)$alkylsulfonylamino group, (w) a halo $(C_1-C_6)$ alkylsulfonylamino group, (x) an N,N-di$(C_1-C_6)$alkylcarboxamido group, (y) an N-$(C_1-C_6)$alkylcarboxamido group, (z) an N-halo$(C_1-C_6)$alkylcarboxamido group, (aa) an N-$(C_1-C_6)$ alkylaminosulfonyl group, (ab) a $(C_1-C_6)$ alkoxy $(C_1-C_6)$ alkyl group, (ac) an $SF_5$ group, (ad) an $R^{10}$-($R^{11}$-N=)O=S group

wherein $R^{10}$ is a $(C_1\text{-}C_6)$alkyl group, a $(C_3\text{-}C_6)$cycloalkyl group, a halo$(C_1\text{-}C_6)$alkyl group, or a $(C_1\text{-}C_6)$ alkoxy $(C_1\text{-}C_6)$ alkyl group, and $R^{11}$ is a hydrogen atom, a cyano group, a $(C_1\text{-}C_6)$alkyl group, a $(C_3\text{-}C_6)$cycloalkyl group, a halo $(C_1\text{-}C_6)$ alkyl group, a $(C_1\text{-}C_6)$alkylcarbonyl group, or a halo $(C_1\text{-}C_6)$ alkylcarbonyl group, (ae) an $R^{10}$-($R^{11}$-N=) S group wherein $R^{10}$ and $R^{11}$ are the same as above, (af) a pyrazolyl group, (ag) a phenoxy group, (ah) a phenyl group, and (ai) a substituted phenyl group having, on a ring, 1 to 5 substituents each independently selected from the group consisting of a halogen atom, a cyano group, a $(C_1\text{-}C_6)$ alkyl group, a $(C_1\text{-}C_6)$ alkoxy group, a $(C_3\text{-}C_6)$ cycloalkyl group, a $(C_1\text{-}C_6)$alkylthio group, a $(C_1\text{-}C_6)$alkylsulfinyl group, a $(C_1\text{-}C_6)$ alkylsulfonyl group, a halo$(C_1\text{-}C_6)$alkyl group and a halo $(C_1\text{-}C_6)$ alkoxy group, or (aj) a methylenedioxy group formed by two adjacent substituents together and optionally substituted by 1 or 2 substituents selected from a halogen atom, a phenyl group and a $(C_1\text{-}C_6)$alkyl group, provided that an atom adjacent to a bonding atom in the pyrazolyl group is not substituted by the $(C_1\text{-}C_6)$alkylsulfonyl group, the halo$(C_1\text{-}C_6)$alkylsulfonyl group, the N-$(C_1\text{-}C_6)$alkylaminosulfonyl group, or the $R^{10}$-($R^{11}$-N=)O=S group wherein $R^{10}$ and $R^{11}$ are the same as above; (c19) a pyridyl group; (c20) a substituted pyridyl group having, on a ring, 1 to 4 substituents each independently selected from the group consisting of a halogen atom, a cyano group, a nitro group, a $(C_1\text{-}C_6)$alkyl group, a $(C_1\text{-}C_6)$alkoxy group, a $(C_3\text{-}C_6)$cycloalkyl group, a $(C_1\text{-}C_6)$alkylthio group, a $(C_1\text{-}C_6)$alkylsulfinyl group, a $(C_1\text{-}C_6)$ alkylsulfonyl group, a halo$(C_1\text{-}C_6)$alkyl group, a halo $(C_1\text{-}C_6)$ alkoxy group, a halo $(C_1\text{-}C_6)$alkylthio group, a halo$(C_1\text{-}C_6)$alkylsulfinyl group and a halo$(C_1\text{-}C_6)$alkylsulfonyl group; (c23) a pyridazinyl group; (c24) a substituted pyridazinyl group having, on a ring, 1 to 3 substituents each independently selected from the group consisting of a halogen atom, a cyano group, a $(C_1\text{-}C_6)$alkyl group, a $(C_1\text{-}C_6)$alkoxy group, a $(C_3\text{-}C_6)$cycloalkyl group, a $(C_1\text{-}C_6)$alkylthio group, a $(C_1\text{-}C_6)$alkylsulfinyl group, a $(C_1\text{-}C_6)$ alkylsulfonyl group, a halo$(C_1\text{-}C_6)$alkyl group and a halo$(C_1\text{-}C_6)$alkoxy group; (c25) a pyrimidinyl group; (c26) a substituted pyrimidinyl group having, on a ring, 1 to 3 substituents each independently selected from the group consisting of a halogen atom, a cyano group, a $(C_1\text{-}C_6)$ alkyl group, a $(C_1\text{-}C_6)$ alkoxy group, a $(C_3\text{-}C_6)$ cycloalkyl group, a $(C_1\text{-}C_6)$alkylthio group, a $(C_1\text{-}C_6)$alkylsulfinyl group, a $(C_1\text{-}C_6)$alkylsulfonyl group, a halo$(C_1\text{-}C_6)$alkyl group and a halo$(C_1\text{-}C_6)$alkoxy group; (c27) a pyrazinyl group; (c28) a substituted pyrazinyl group having, on a ring, 1 to 3 substituents each independently selected from the group consisting of a halogen atom, a cyano group, a $(C_1\text{-}C_6)$ alkyl group, a $(C_1\text{-}C_6)$ alkoxy group, a $(C_3\text{-}C_6)$ cycloalkyl group, a $(C_1\text{-}C_6)$ alkylthio group, a $(C_1\text{-}C_6)$alkylsulfinyl group, a $(C_1\text{-}C_6)$alkylsulfonyl group, a halo$(C_1\text{-}C_6)$alkyl group and a halo$(C_1\text{-}C_6)$alkoxy group; (c29) a triazinyl group; (c30) a substituted triazinyl group having, on a ring, 1 or 2 substituents each independently selected from the group consisting of a halogen atom, a cyano group, a $(C_1\text{-}C_6)$alkyl group, a $(C_1\text{-}C_6)$alkoxy group, a $(C_3\text{-}C_6)$cycloalkyl group, a $(C_1\text{-}C_6)$alkylthio group, a $(C_1\text{-}C_6)$alkylsulfinyl group, a $(C_1\text{-}C_6)$alkylsulfonyl group, a halo$(C_1\text{-}C_6)$alkyl group and a halo $(C_1\text{-}C_6)$ alkoxy group; (c37) an oxazolyl group; (c38) a substituted oxazolyl group having, on a ring, 1 or 2 substituents each independently selected from the group consisting of a halogen atom, a cyano group, a $(C_1\text{-}C_6)$alkyl group, a $(C_1\text{-}C_6)$alkoxy group, a $(C_3\text{-}C_6)$cycloalkyl group, a $(C_1\text{-}C_6)$alkylthio group, a $(C_1\text{-}C_6)$alkylsulfinyl group, a $(C_1\text{-}C_6)$alkylsulfonyl group, a halo$(C_1\text{-}C_6)$alkyl group and a halo$(C_1\text{-}C_6)$alkoxy group; (c39) a thiazolyl group; (c40) a substituted thiazolyl group having, on a ring, 1 or 2 substituents each independently selected from the group consisting of a halogen atom, a cyano group, a $(C_1\text{-}C_6)$alkyl group, a $(C_1\text{-}C_6)$alkoxy group, a $(C_3\text{-}C_6)$cycloalkyl group, a $(C_1\text{-}C_6)$alkylthio group, a $(C_1\text{-}C_6)$alkylsulfinyl group, a $(C_1\text{-}C_6)$alkylsulfonyl group, a halo$(C_1\text{-}C_6)$alkyl group and a halo $(C_1\text{-}C_6)$alkoxy group; (c41) an isoxazolyl group; (c42) a substituted isoxazolyl group having, on a ring, 1 or 2 substituents each independently selected from the group consisting of a halogen atom, a cyano group, a $(C_1\text{-}C_6)$alkyl group, a $(C_1\text{-}C_6)$alkoxy group, a $(C_3\text{-}C_6)$cycloalkyl group, a $(C_1\text{-}C_6)$alkylthio group, a $(C_1\text{-}C_6)$alkylsulfinyl group, a $(C_1\text{-}C_6)$ alkylsulfonyl group, a halo$(C_1\text{-}C_6)$alkyl group and a halo $(C_1\text{-}C_6)$alkoxy group; (c43) an isothiazolyl group; (c44) a substituted isothiazolyl group having, on a ring, 1 or 2 substituents each independently selected from the group consisting of a halogen atom, a cyano group, a $(C_1\text{-}C_6)$alkyl group, a $(C_1\text{-}C_6)$alkoxy group, a $(C_3\text{-}C_6)$cycloalkyl group, a $(C_1\text{-}C_6)$alkylthio group, a $(C_1\text{-}C_6)$alkylsulfinyl group, a $(C_1\text{-}C_6)$ alkylsulfonyl group, a halo$(C_1\text{-}C_6)$alkyl group and a halo $(C_1\text{-}C_6)$alkoxy group; (c45) an imidazolyl group; (c46) a substituted imidazolyl group having, on a ring, 1 to 3 substituents each independently selected from the group consisting of a halogen atom, a cyano group, a $(C_1\text{-}C_6)$ alkyl group, a $(C_1\text{-}C_6)$ alkoxy group, a $(C_3\text{-}C_6)$ cycloalkyl group, a $(C_1\text{-}C_6)$ alkylthio group, a $(C_1\text{-}C_6)$alkylsulfinyl group, a $(C_1\text{-}C_6)$alkylsulfonyl group, a halo$(C_1\text{-}C_6)$alkyl group and a halo $(C_1\text{-}C_6)$ alkoxy group; (c47) a pyrazolyl group; (c48) a substituted pyrazolyl group having, on a ring, 1 to 3 substituents each independently selected from the group consisting of a halogen atom, a cyano group, a $(C_1\text{-}C_6)$alkyl group, a $(C_1\text{-}C_6)$alkoxy group, a $(C_3\text{-}C_6)$cycloalkyl group, a $(C_1\text{-}C_6)$alkylthio group, a $(C_1\text{-}C_6)$alkylsulfinyl group, a $(C_1\text{-}C_6)$alkylsulfonyl group, a halo$(C_1\text{-}C_6)$alkyl group and a halo$(C_1\text{-}C_6)$alkoxy group; (c49) a triazolyl group; (c50) a substituted triazolyl group having, on a ring, 1 or 2 substituents each independently selected from the group consisting of a halogen atom, a cyano group, a $(C_1\text{-}C_6)$alkyl group, a $(C_1\text{-}C_6)$alkoxy group, a $(C_3\text{-}C_6)$cycloalkyl group, a $(C_1\text{-}C_6)$alkylthio group, a $(C_1\text{-}C_6)$alkylsulfinyl group, a $(C_1\text{-}C_6)$alkylsulfonyl group, a halo$(C_1\text{-}C_6)$alkyl group and a halo$(C_1\text{-}C_6)$alkoxy group; (c51) a tetrazolyl group; (c52) a substituted tetrazolyl group having, on a ring, one substituent selected from the group

consisting of a halogen atom, a cyano group, a $(C_1-C_6)$alkyl group, a $(C_1-C_6)$alkoxy group, a $(C_3-C_6)$cycloalkyl group, a $(C_1-C_6)$ alkylthio group, a $(C_1-C_6)$ alkylsulfinyl group, a $(C_1-C_6)$alkylsulfonyl group, a halo $(C_1-C_6)$ alkyl group and a halo$(C_1-C_6)$alkoxy group; (c53) a thiadiazolyl group; (c54) a substituted thiadiazolyl group having, on a ring, one substituent selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$ alkylsulfonyl group, a halo $(C_1-C_6)$ alkyl group and a halo$(C_1-C_6)$alkoxy group; (c55) a quinolinyl group; (c56) a substituted quinolinyl group having, on a ring, 1 to 5 substituents each independently selected from the group consisting of a halogen atom, a cyano group, a $(C_1-C_6)$ alkyl group, a $(C_1-C_6)$ alkoxy group, a $(C_3-C_6)$cycloalkyl group, a $(C_1-C_6)$ alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c57) a benzoxazolyl group; (c58) a substituted benzoxazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$ alkylsulfinyl group, a $(C_1-C_6)$ alkylsulfonyl group, a halo $(C_1-C_6)$ alkyl group and a halo $(C_1-C_6)$alkoxy group; (c59) a benzothiazolyl group; (c60) a substituted benzothiazolyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo $(C_1-C_6)$alkoxy group; (c67) an indanyl group; (c68) a substituted indanyl group having, on a ring, 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$alkyl group and a halo $(C_1-C_6)$ alkoxy group; (c69) a tetrahydronaphthyl group; (c70) a substituted tetrahydronaphthyl group having, on a ring, 1 to 4 substituents each independently selected from the group consisting of a halogen atom, a cyano group, a $(C_1-C_6)$ alkyl group, a $(C_1-C_6)$alkoxy group, a $(C_3-C_6)$cycloalkyl group, a $(C_1-C_5)$alkylthio group, a $(C_1-C_6)$ alkylsulfinyl group, a $(C_1-C_6)$ alkylsulfonyl group, a halo$(C_1-C_6)$alkyl group and a halo$(C_1-C_6)$alkoxy group; (c73) a phenyl$(C_1-C_6)$alkyl group; (c74) a substituted phenyl$(C_1-C_6)$alkyl group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo$(C_1-C_6)$alkoxy group; (c75) a $(C_1-C_6)$alkoxycarbonyl group; (c76) a phenylcarbonyl group; (c77) a thienyl group; (c78) a substituted thienyl group having, on a ring, 1 to 3 substituents each independently selected from the group consisting of a halogen atom, a cyano group, a nitro group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group, a halo $(C_1-C_6)$alkoxy group, a halo $(C_1-C_6)$ alkylthio group, a halo $(C_1-C_6)$ alkylsulfinyl group and a halo$(C_1-C_6)$alkylsulfonyl group; (c79) a naphthyl group; (c80) a substituted naphthyl group having, on a ring, 1 to 7 substituents each independently selected from the group consisting of a halogen atom, a cyano group, a nitro group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group, a halo $(C_1-C_6)$ alkoxy group, a halo $(C_1-C_6)$alkylthio group, a halo$(C_1-C_6)$alkylsulfinyl group and a halo$(C_1-C_6)$alkylsulfony group; (c81) a 1,1-dioxidetetrahydro-2H-thiopyranyl group; (c82) an imidazopyridyl group; or (c83) a substituted imidazopyridyl group having, on a ring, 1 to 5 substituents each independently selected from the group consisting of a halogen atom, a cyano group, a nitro group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$ alkylsulfinyl group, a $(C_1-C_6)$alkylsulfonyl group, a halo $(C_1-C_6)$alkyl group, a halo $(C_1-C_6)$alkoxy group, a halo $(C_1-C_6)$alkylthio group, a halo$(C_1-C_6)$alkylsulfinyl group, a halo$(C_1-C_6)$alkylsulfonyl group and a phenyl group,
Q is

(d1) a substituted phenyl group having, on a ring, 1 to 5 substituents each independently selected from substituent group A;
(d2) a naphthyl group; (d3) a substituted naphthyl group having, on a ring, 1 to 7 substituents each independently selected from substituent group B; (d4) a pyridyl group; (d5) a substituted pyridyl group having, on a ring, 1 to 4 substituents each independently selected from substituent group B; (d6) a pyridazinyl group; (d7) a substituted pyridazinyl group having, on a ring, 1 to 3 substituents each independently selected from substituent group B; (d8) a pyrimidinyl group; (d9) a substituted pyrimidinyl group having, on a ring, 1 to 3 substituents each independently selected from substituent group B; (d10) a pyrazinyl group; (d11) a substituted pyrazinyl group having, on a ring, 1 to 3 substituents each independently selected from substituent group B; (d12) a triazinyl group; (d13) a substituted triazinyl group having, on a ring, 1 or 2 substituents each independently selected from substituent, group B; (d14) a 2-oxopyridyl group; (d15) a substituted 2-oxopyridyl group having, on a ring, 1 to 4 substituents each independently selected from substituent group B;

(d16) an isoxazolyl group; (d17) a substituted isoxazolyl group having, on a ring, 1 or 2 substituents each independently selected from substituent group B; (d18) an oxazolyl group; (d19) a substituted oxazolyl group having, on a ring, 1 or 2 substituents each independently selected from substituent group B; (d20) a thienyl group; (d21) a substituted thienyl group having, on a ring, 1 to 3 substituents each independently selected from substituent group B; (d22) an isothiazolyl group; (d23) a substituted isothiazolyl group having, on a ring, 1 or 2 substituents each independently selected from substituent group B; (d24) a thiazolyl group; (d25) a substituted thiazolyl group having, on a ring, 1 or 2 substituents each independently selected from substituent group B; (d44) a benzothienyl group; (d45) a substituted benzothienyl group having, on a ring, 1 to 5 substituents each independently selected from substituent group B; (d46) a benzothiazolyl group; (d47) a substituted benzothiazolyl group having, on a ring, 1 to 4 substituents each independently selected from substituent group B; (d48) a benzisothiazolyl group; (d49) a substituted benzisothiazolyl group having, on a ring, 1 to 4 substituents each independently selected from substituent group B; (d90) a quinoxalinyl group; (d91) a substituted quinoxalinyl group having, on a ring, 1 to 5 substituents each independently selected from substituent group B; (d92) a quinolinyl group; (d93) a substituted quinolinyl group having, on a ring, 1 to 6 substituents each independently selected from substituent group B; (d94) an isoquinolinyl group; (d95) a substituted isoquinolinyl group having, on a ring, 1 to 6 substituents each independently selected from substituent group B; (d100) a quinazolinyl group; (d101) a substituted quinazolinyl group having, on a ring, 1 to 5 substituents each independently selected from substituent group B; or (d104) a ferrocenyl group,

X and Y are each independently an oxygen atom or a sulfur atom,
Z is an $OR^4$ group wherein $R^4$ is a hydrogen atom, a $(C_1-C_6)$ alkyl group, a $(C_1-C_6)$alkylcarbonyl group or a $(C_1-C_6)$ alkoxycarbonyl group, an $S(O)nR^5$ group wherein n is 0, 1 or 2, and $R^5$ is a hydrogen atom, a $(C_1-C_6)$alkyl group or a phenyl $(C_1-C_6)$ alkyl group, or an $N(R^6)R^7$ group wherein $R^6$ and $R^7$ are hydrogen atoms, hydroxyl groups, $(C_1-C_6)$alkyl groups, $(C_1-C_6)$alkoxy groups, $(C_1-C_6)$alkylcarbonyl groups or $(C_1-C_6)$alkoxycarbonyl groups, and $R^6$ and $R^7$ are the same or different,
substituent group A consists of

(e1) a cyano group; (e2) a nitro group; (e4) a carboxyl group;
(e5) a $(C_1-C_6)$alkyl group; (e6) a $(C_2-C_6)$alkenyl group; (e7) a $(C_2-C_6)$ alkynyl group; (e8) a $(C_3-C_6)$ cycloalkyl group; (e9) a substituted $(C_3-C_6)$cycloalkyl group having, on a ring, 1 to 3 substituents each independently selected from the group consisting of a halogen atom, a cyano group, a $(C_1-C_6)$alkyl group, a $(C_1-C_6)$alkoxy group and a $(C_1-C_6)$ alkylcarbonyl group; (e10) a $(C_1-C_6)$alkoxy $(C_1-C_6)$ alkyl group; (e11) a $(C_1-C_6)$ alkylthio group; (e12) a $(C_1-C_6)$alkylsulfinyl group; (e13) a $(C_1-C_6)$alkylsulfonyl group;
(e14) a halo$(C_1-C_6)$alkyl group; (e17) a halo $(C_1-C_6)$alkoxy group;
(e18) a halo $(C_3-C_6)$ cycloalkyl group; (e19) a halo $(C_1-C_6)$ alkylthio group; (e20) a halo$(C_1-C_6)$alkylsulfinyl group; (e21) a halo$(C_1-C_6)$alkylsulfonyl group; (e22) an $SF_5$ group; (e23) a $(C_1-C_6)$alkylcarbonylamino group; (e24) a halo$(C_1-C_6)$alkylcarbonylamino group; (e25) a $(C_1-C_6)$alkylsulfonylamino group; (e26) a halo$(C_1-C_6)$alkylsulfonylamino group; (e27) an N,N-di$(C_1-C_6)$alkylcarboxamido group; (e28) an N-halo$(C_1-C_6)$alkylcarboxamido group; (e29) a $(C_1-C_6)$alkoxycarbonyl group; (e30) an N-$(C_1-C_6)$alkylaminosulfonyl group; (e32) a pyrazolyl group; (e33) a substituted pyrazolyl group having, on a ring, 1 to 3 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$ alkoxy group; (e36) a phenyl group;
(e37) a substituted phenyl group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo$(C_1-C_6)$alkyl group and a halo $(C_1-C_6)$ alkoxy group; (e38) a phenoxy group; (e39) a substituted phenoxy group having, on a ring, 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a $(C_1-C_6)$alkylthio group, a $(C_1-C_6)$alkylsulfinyl group, a $(C_1-C_6)$alkylsulfonyl group, a halo $(C_1-C_6)$ alkyl group and a halo $(C_1-C_6)$ alkoxy group; (e40) a methylenedioxy group formed by two adjacent substituents together and optionally substituted by 1 or 2 substituents selected from the group consisting of a halogen atom, a phenyl group and a $(C_1-C_6)$ alkyl group; (e41) an $R^8$-$(R^9$-N=)O=S group wherein $R^8$ is a $(C_1-C_6)$alkyl group, a $(C_3-C_6)$cycloalkyl group, a halo $(C_1-C_6)$ alkyl group, or a $(C_1-C_6)$ alkoxy $(C_1-C_6)$ alkyl group, and $R^9$ is a hydrogen atom, a cyano group, a $(C_1-C_6)$ alkyl group, a $(C_3-C_6)$cycloalkyl group, a halo $(C_1-C_6)$ alkyl group, a $(C_1-C_6)$alkylcarbonyl group, or a halo$(C_1-C_6)$alkylcarbonyl group; (e42) a carboxamido group;

(e43) a morpholinyl group; (e44) an N,N-di($C_1$-$C_6$)alkylamino group; (e45) an $R^8$-($R^9$-N=)S group wherein $R^8$ and $R^9$ are the same as above; (e46) an N-($C_1$-$C_6$)alkylcarboxamido group; (e47) a phenyl ($C_1$-$C_6$)alkylthio group; and (e48) a substituted phenyl($C_1$-$C_6$)alkylthio group having, on a ring, 1 to 5 substituents each independently selected from the group consisting of a halogen atom, a cyano group, a ($C_1$-$C_6$) alkyl group; a. ($C_1$-$C_6$) alkoxy group, a ($C_3$-$C_6$) cycloalkyl group, a ($C_1$-$C_6$)alkylthio group, a ($C_1$-$C_6$)alkylsulfinyl group, a ($C_1$-$C_6$) alkylsulfonyl group, a halo($C_1$-$C_6$)alkyl group and a halo ($C_1$-$C_6$)alkoxy group, and

substituent group B consists of
(f1) a halogen atom; (f2) a cyano group; (f3) a nitro group; (f6) a ($C_1$-$C_6$)alkyl group; (f7) a ($C_2$-$C_6$)alkenyl group; (f8) a ($C_2$-$C_6$)alkynyl group; (f9) a ($C_1$-$C_6$)alkoxy group; (f10) a ($C_3$-$C_6$)cycloalkyl group; (f11) a substituted ($C_3$-$C_6$)cycloalkyl group having, on a ring, 1 to 3 substituents each independently selected from the group consisting of a halogen atom, a cyano group, a ($C_1$-$C_6$)alkyl group, a ($C_1$-$C_6$) alkoxy group and a ($C_1$-$C_6$)alkylcarbonyl group; (f12) a ($C_1$-$C_6$) alkoxy ($C_1$-$C_6$) alkyl group; (f13) a ($C_1$-$C_6$) alkylthio group; (f14) a ($C_1$-$C_6$)alkylsulfinyl group; (f15) a ($C_1$-$C_6$)alkylsulfonyl group; (f16) a halo ($C_1$-$C_6$) alkyl group; (f19) a halo ($C_1$-$C_6$) alkoxy group; (f20) a halo ($C_3$-$C_6$) cycloalkyl group; (f21) a halo ($C_1$-$C_6$) alkylthio group; (f22) a halo ($C_1$-$C_6$) alkylsulfinyl group; (f23) a halo ($C_1$-$C_6$)alkylsulfonyl group; (f25) a ($C_1$-$C_6$)alkylcarbonylamino group; (f26) a halo($C_1$-$C_6$)alkylcarbonylamino group; (f27) a ($C_1$-$C_6$) alkylsulfonylamino group; (f28) a halo($C_1$-$C_6$)alkylsulfonylamino group; (f29) an N,N-di($C_1$-$C_6$)alkylcarboxamido group; (f30) an N-halo ($C_1$-$C_6$) alkylcarboxamido group; (f31) a ($C_1$-$C_6$) alkoxycarbonyl group; (f34) a pyrazolyl group; (f35) a substituted pyrazolyl group having, on a ring, 1 to 3 substituents each independently selected from the group consisting of a halogen atom, a cyano group, a ($C_1$-$C_6$)alkyl group, a ($C_1$-$C_6$) alkoxy group, a ($C_3$-$C_6$)cycloalkyl group, a ($C_1$-$C_6$)alkylthio group, a ($C_1$-$C_6$) alkylsulfinyl group, a ($C_1$-$C_6$)alkylsulfonyl group, a halo ($C_1$-$C_6$) alkyl group and a halo ($C_1$-$C_6$) alkoxy group; (f38) a phenyl group; (f39) a substituted phenyl group having, on a ring, 1 to 5 substituents each independently selected from the group consisting of a halogen atom, a cyano group, a ($C_1$=$C_6$)alkyl group, a ($C_1$-$C_6$) alkoxy group, a ($C_3$-$C_6$) cycloalkyl group, a ($C_1$-$C_6$) alkylthio group, a. ($C_1$-$C_6$) alkylsulfinyl group, a ($C_1$-$C_6$) alkylsulfonyl group, a halo($C_1$-$C_6$)alkyl group and a halo ($C_1$-$C_6$) alkoxy group; (f40) a phenoxy group; (f41) a substituted phenoxy group having, on a ring, 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a ($C_1$-$C_6$)alkylthio group, a ($C_1$-$C_6$)alkylsulfinyl group, a ($C_1$-$C_6$)alkylsulfonyl group, a halo($C_1$-$C_6$)alkyl group and a halo($C_1$-$C_6$)alkoxy group; (f42) a methylenedioxy group formed by two adjacent substituents together and optionally substituted by 1 or 2 substituents selected from the group consisting of a halogen atom, a phenyl group and a ($C_1$-$C_6$)alkyl group; (f44) a carboxamido group; (f45) a morpholinyl group; and (f46) an N,N-di($C_1$-$C_6$)alkylamino group.

**3.** The compound and salts thereof according to claim 1 or 2,
wherein

$R^1$ is
(a1) a hydrogen atom; (a2) a ($C_1$-$C_6$)alkyl group; or (a4) a phenyl group,
$R^2$ is (b1) a hydrogen atom,
$R^3$ is

(c1) a ($C_1$-$C_6$)alkyl group; (c2) a ($C_2$-$C_6$)alkenyl group; (c4) a ($C_3$-$C_6$)cycloalkyl group; (c8') a substituted ($C_1$-$C_6$)alkyl group having, on a ring, 1 to 3 substituents each independently selected from the group consisting of a ($C_1$-$C_6$)alkoxy group, a ($C_1$-$C_6$)alkoxycarbonyl group and a ($C_2$-$C_6$)alkenylcarbonyloxy group; (c18') a substituted phenyl group having, on a ring, 1 to 5 substituents each independently selected from the group consisting of (a) a halogen atom, (b) a cyano group, (c) a nitro group, (e) a carboxyl group, (f) a ($C_1$-$C_6$)alkyl group, (g) a ($C_1$-$C_6$)alkoxy group, (h) a ($C_3$-$C_6$)cycloalkyl group, (i) a ($C_1$-$C_6$)alkylthio group, (j) a ($C_1$-$C_6$)alkylsulfinyl group, (k) a ($C_1$-$C_6$)alkylsulfonyl group, (1) a halo ($C_1$-$C_6$) alkyl group, (m) a halo ($C_1$-$C_6$) alkoxy group, (o) a halo ($C_1$-$C_6$) alkylthio group, (p) a halo ($C_1$-$C_6$) alkylsulfinyl group, (q) a halo($C_1$-$C_6$)alkylsulfonyl group, (r) a ($C_1$-$C_6$)alkylcarbonyl group, (s) a ($C_1$-$C_6$)alkoxycarbonyl group, (t) a ($C_1$-$C_6$)alkylcarbonylamino group, (v) a ($C_1$-$C_6$)alkylsulfonylamino group, (z) an N-halo($C_1$-$C_6$)alkylcarboxamido group, (aa) an N-($C_1$-$C_6$)alkylaminosulfonyl group, (ab) a ($C_1$-$C_6$)alkoxy($C_1$-$C_6$)alkyl group, (ac) an $SF_5$ group, (ae') an $R^{10}$-($R^{11}$-N=)S group wherein $R^{10}$ is ($C_1$-$C_6$)alkyl, and $R^{11}$ is a cyano group, (ag) a phenoxy group and (ah) (aj') a phenyl group, or a methylenedioxy group formed by two adjacent substituents together; (c19) a pyridyl group; (c20') a substituted pyridyl group having, on a ring, 1 to 4 substituents each independently selected from the group consisting of a halogen atom and a halo($C_1$-$C_6$)alkyl group; (c24') a substituted pyridazinyl group having, on a ring, 1 to 3 substituents each independently selected from

halogen atoms; (c26') a substituted pyrimidinyl group having, on a ring, 1 to 3 substituents each independently selected from halogen atoms; (c27) a pyrazinyl group; (c37) an oxazolyl group; (c39) a thiazolyl group; (c42') a substituted isoxazolyl group having, on a ring, 1 or 2 substituents each independently selected from ($C_1$-$C_6$)alkyl groups; (c44') a substituted isothiazolyl group having, on a ring, 1 or 2 substituents each independently selected from ($C_1$-$C_6$)alkyl groups; (c45) an imidazolyl group; (c48') a substituted pyrazolyl group having, on a ring, 1 to 3 substituents each independently selected from ($C_1$-$C_6$)alkyl groups; (c49) a triazolyl group; (c52') a substituted tetrazolyl group having, on a ring, one substituent selected from a ($C_1$-$C_6$)alkyl group; (c54') a substituted thiadiazolyl group having, on a ring, one substituent selected from a halo($C_1$-$C_6$)alkyl group; (c55) a quinolinyl group; (c58') a substituted benzoxazolyl group having, on a ring, 1 to 4 substituents each independently selected from halogen atoms; (c59) a benzothiazolyl group; (c73) a phenyl($C_1$-$C_6$)alkyl group; (c74') a substituted phenyl ($C_1$-$C_6$)alkyl group having, on a ring, 1 to 5 substituents each independently selected from halogen atoms; (c75) a ($C_1$-$C_6$)alkoxycarbonyl group; (c76) a phenylcarbonyl group; (c77) a thienyl group; (c79) a naphthyl group; (c81) a 1,1-dioxidetetrahydro-2H-thiopyranyl group; or (c83') a substituted imidazopyridyl group having, on a ring, 1 to 5 phenyl groups,

Q is

(d1) a substituted phenyl group having, on a ring, 1 to 5 substituents each independently selected from substituent group A;
(d2) a naphthyl group; (d5) a substituted pyridyl group having, on a ring, 1 to 4 substituents each independently selected from substituent group B; (d8) a pyrimidinyl group; (d17) a substituted isoxazolyl group having, on a ring, 1 or 2 substituents each independently selected from substituent group B; (d18) an oxazolyl group; (d20) a thienyl group; (d21) a substituted thienyl group having, on a ring, 1 to 3 substituents each independently selected from.substituent group B; (d22) an isothiazolyl group; (d24) a thiazolyl group; (d92) a quinolinyl group; or (d104) a ferrocenyl group,

X and Y are each independently an oxygen atom or a sulfur atom,
Z is an $OR^4$ group wherein $R^4$ is a hydrogen atom, an $S(O)nR^5$ group wherein n is 0, and $R^5$ is a phenyl ($C_1$-$C_6$) alkyl group, or an $N(R^6)R^7$ group wherein $R^6$ and $R^7$ are hydrogen atoms, hydroxyl groups, or ($C_1$-$C_6$) alkoxy groups, and $R^6$ and $R^7$ are the same or different, substituent group A consists of

(e1) a cyano group; (e2) a nitro group; (e4) a carboxyl group;
(e5) a ($C_1$-$C_6$)alkyl group; (e11) a ($C_1$-$C_6$)alkylthio group; (e12) a ($C_1$-$C_6$)alkylsulfinyl group; (e13) a ($C_1$-$C_6$)alkylsulfonyl group;
(e14) a halo ($C_1$-$C_6$) alkyl group; (e17) a halo ($C_1$-$C_6$) alkoxy group;
(e19) a halo($C_1$-$C_6$)alkylthio group; (e20) a halo ($C_1$-$C_6$)alkylsulfinyl group; (e21) a halo($C_1$-$C_6$)alkylsulfonyl group; (e29) a ($C_1$-$C_6$)alkoxycarbonyl group; (e38) a phenoxy group; (e40') a methylenedioxy group formed by two adjacent substituents together;
(e42) a carboxamido group; (e43) a morpholinyl group; (e44) an N,N-di($C_1$-$C_6$)alkylamino group; (e45') an $R^8$-($R^9$-N=)S group wherein $R^8$ is a ($C_1$-$C_6$)alkyl group, and $R^9$ is a cyano group; (e46) an N-($C_1$-$C_6$)alkylcarboxamido group; and (e48') a substituted phenyl($C_1$-$C_6$)alkylthio group having, on a ring, 1 to 5 substituents each independently selected from ($C_1$-$C_6$)alkyl groups, and substituent group B consists of
(f1) a halogen atom; (f6) a ($C_1$-$C_6$) alkyl group; (f16) a halo($C_1$-$C_6$)alkyl group; and (f38) a phenyl group.

**4.** A pest control agent comprising the compound or salts thereof according to any one of claims 1 to 3 as an active ingredient.

**5.** An agrohorticultural insect pest control agent comprising the compound or salts thereof according to any one of claims 1 to 3 as an active ingredient.

**6.** An ectoparasite control agent for animals, comprising the compound or salts thereof according to any one of claims 1 to 3 as an active ingredient.

**7.** An endoparasite control agent for animals, comprising the compound or salts thereof according to any one of claims 1 to 3 as an active ingredient.

**8.** A method for controlling an agrohorticultural insect pest, comprising treating a plant or soil with an effective amount of the agrohorticultural insect pest control agent according to claim 5.

**9.** A method for controlling an ectoparasite in an animal, comprising orally or parenterally administering an effective amount of the ectoparasite control.agent for animals according to claim 6 to a target animal.

**10.** A method for controlling an endoparasite in an animal, comprising orally or parenterally administering an effective amount of the endoparasite control agent for animals according to claim 7 to a target animal.

**11.** Use of the compound or salts thereof according to any one of claims 1 to 3 as a pest control agent.

**INTERNATIONAL SEARCH REPORT**

International application No.
**PCT/JP2022/047949**

**A. CLASSIFICATION OF SUBJECT MATTER**

***C07C 235/82***(2006.01)i; ***A01N 37/22***(2006.01)i; ***A01N 37/24***(2006.01)i; ***A01N 43/10***(2006.01)i; ***A01N 43/30***(2006.01)i; ***A01N 43/40***(2006.01)i; ***A01N 43/50***(2006.01)i; ***A01N 43/78***(2006.01)i; ***A01N 47/02***(2006.01)i; ***A01P 5/00***(2006.01)i; ***A01P 7/04***(2006.01)i; ***A61K 31/167***(2006.01)i; ***A61K 31/36***(2006.01)i; ***A61K 31/381***(2006.01)i; ***A61K 31/426***(2006.01)i; ***A61K 31/44***(2006.01)i; ***A61K 31/4402***(2006.01)i; ***A61K 31/505***(2006.01)i; ***A61P 33/14***(2006.01)i; ***C07C 317/40***(2006.01)i; ***C07C 323/40***(2006.01)i; ***C07D 213/81***(2006.01)i; ***C07D 239/26***(2006.01)i; ***C07D 277/44***(2006.01)i; ***C07D 317/66***(2006.01)i; ***C07D 333/36***(2006.01)i

FI: C07C235/82; A61K31/167; C07D213/81; C07D333/36; C07D317/66; C07D239/26; A61P33/14; A61K31/426; A61K31/381; A61K31/4402; A61K31/36; A61K31/505; A61K31/44; A01P7/04; A01P5/00; A01N37/22 101; A01N37/24 101; A01N47/02; A01N43/10 A; A01N43/40 101J; A01N43/78 B; A01N43/30; A01N43/40 101A; A01N43/50 A; A01N43/10 B; C07C317/40; C07C323/40; C07D277/44 CSP

According to International Patent Classification (IPC) or to both national classification and IPC

**B. FIELDS SEARCHED**

Minimum documentation searched (classification system followed by classification symbols)

C07C235/82; A01N37/22; A01N37/24; A01N43/10; A01N43/30; A01N43/40; A01N43/50; A01N43/78; A01N47/02; A01P5/00; A01P7/04; A61K31/167; A61K31/36; A61K31/381; A61K31/426; A61K31/44; A61K31/4402; A61K31/505; A61P33/14; C07C317/40; C07C323/40; C07D213/81; C07D239/26; C07D277/44; C07D317/66; C07D333/36

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WINKELMANN, E., et al., Antimalarial and anticoccidial activity of 3-aryl-7-chloro-3,4-dihydroacridine-1,9-(2H,10H)-diones. 1-Imines, 1-amines, 1-oximes, 1-hydrazones and related compounds, Arzneimittel-Forschung, 1987, vol. 37(6), pp. 647-661<br>table 1 | 1-11 |
| X | JP 49-100054 A (BAYER AKTIENGESELLSCHAFT) 20 September 1974 (1974-09-20)<br>tables 1, 2 (in particular, production examples no. 34, 35) | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 February 2023** | **14 March 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/047949**

**C. DOCUMENTS CONSIDERED TO BE RELEVANT**

| Category* | Citation of document, with indication, where appropriate, of the relevant passages | Relevant to claim No. |
|---|---|---|
| A | JP 04-005276 A (NIPPON NOHYAKU CO., LTD.) 19 January 1992 (1992-01-19) entire text | 1-11 |
| A | US 3976785 A (AMERICAN CYANAMID CO.) 24 August 1976 (1976-08-24) entire text | 1-11 |
| A | JP 51-125738 A (NIPPON SODA CO., LTD.) 02 November 1976 (1976-11-02) entire text | 1-11 |
| A | UKITA, Tyunoshin, et al., In vitro screening of tricarbonylmethane and related compounds for their antitumor effect by cylinder agar plate method, Chemical & Pharmaceutical Bulletin, 1960, vol. 8, pp. 1016-1020 entire text | 1-11 |
| A | LIU, Jinglin, et al., Efficient synthesis of highly functionalized vinylogous thiol esters, Tetrahedron Letters, 2010, vol.51 (52), pp. 6918-6920 entire text | 1-11 |

**INTERNATIONAL SEARCH REPORT**
**Information on patent family members**

International application No.
**PCT/JP2022/047949**

| Patent document cited in search report | Publication date (day/month/year) | Patent family member(s) | Publication date (day/month/year) |
|---|---|---|---|
| JP 49-100054 A | 20 September 1974 | GB 1377319 A<br>tables 1, 2<br>DE 2232730 A<br>FR 2190806 A<br>JP 49-100225 A | |
| JP 04-005276 A | 19 January 1992 | (Family: none) | |
| US 3976785 A | 24 August 1976 | (Family: none) | |
| JP 51-125738 A | 02 November 1976 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3976785 A
- EP 0017195 A
- EP 0374753 A
- WO 9307278 A
- WO 9534656 A
- EP 0427529 A
- EP 451878 A
- WO 03052073 A


### Non-patent literature cited in the description


- *Arzneimittel-Forsching,* 1987, vol. 37, 647-661
- *Proc. Natl. Acad. Sci. USA,* 1990, vol. 87, 7175-7179
- *Weed Science,* 2005, vol. 53, 728-746
- **GURA T.** Repairing the Genome's Spelling Mistakes. *Science,* 1999, vol. 285, 316-318